# EUROPEAN PATENT SPECIFICATION

(11) **EP 1 943 265 B1**
(45) Date of publication and mention of the grant of the patent: **12.09.2012**
(21) Application number: 06815854.2
(22) Date of filing: 29.09.2006
(51) Int. Cl.: C07H 21/04, C07H 21/00, C12N 15/00

(54) **REGULATABLE FUSION PROMOTERS**
REGULIERBARE FUSIONSPROMOTOREN
PROMOTEURS DE FUSION REGULABLES

(30) Priority: 01.10.2005 US 722568 P
(43) Date of publication of application: 16.07.2008
(60) Divisional application: 11160708.1
(73) Proprietor: Stout, Charles, Linda, California 92354 (US)
(72) Inventor: Stout, Charles, Linda, California 92354 (US)
(74) Representative: Williams, Gareth Owen
(86) International application number: PCT/US2006/038154
(87) International publication number: WO 2007/041350

(56) References cited:
- US-A1- 2005 130 184
- STAMMINGER T ET AL: "CELL TYPE-SPECIFIC INDUCTION OF THE MAJOR IMMEDIATE EARLY ENHANCER OF HUMAN CYTOMEGALOVIRUS BY CYCLIC AMP" JOURNAL OF GENERAL VIROLOGY, vol. 71, no. 1, 1990, pages 105-114, XP002503010 ISSN: 0022-1317
- LOBO S M ET AL: "A 7 bp mutation converts a human RNA polymerase II snRNA promoter into an RNA polymerase III promoter", 14 July 1989 (1989-07-14), CELL, CELL PRESS, US, PAGE(S) 55 - 67, XP023872220, ISSN: 0092-8674 [retrieved on 1989-07-14]
- YUAN JING ET AL: "ShRNA transcribed by RNA pol II promoter induce RNA interference in mammalian cell", March 2006 (2006-03), MOLECULAR BIOLOGY REPORTS, VOL. 33, NR. 1, PAGE(S) 43-49 ISSN: 0301-4851
- LUPIEN CAROLINE ET AL: "Expression of glial fibrillary acidic protein in primary cultures of human Muller cells", September 2004 (2004-09), EXPERIMENTAL EYE RESEARCH, VOL. 79, NR. 3, PAGE(S) 423-429 ISSN: 0014-4835

## Description

### FIELD OF THE INVENTION

The present invention relates to RNA polymerase promoters for targeted and/or regulated transcription of coding sequences, and in particular for expressing RNA sequences for RNA interference (RNAi), micro RNA (miRNA), aptamers, short interferring RNA (siRNA), and/or short hairpin RNA (shRNA).

### BACKGROUND OF THE INVENTION

The following discussion is provided solely to assist the understanding of the reader, and does not constitute an admission that any of the information discussed or references cited constitute prior art to the present invention.

Short RNA duplexes of approximately 18 to 30 base pairs have been shown to initiate several types of sequence-specific regulation of gene expression. In one type of regulation, i.e. RNA interference (RNAi), these short RNA duplexes cause sequence-selective degradation of mRNA in a wide range of eukaryotic cells, including mammalian cells. In one embodiment of RNAi, small interfering RNAs (siRNAs) are about 21 nucleotides (nt) long and paired such that they have a 19 base pair stem and 2-nt 3'-overhanging ends that, when introduced to eukaryotic cells, cause sequence-selective degradation of targeted mRNA and gene suppression (Caplen, et al. (2001) Proc Natl Acad Sci USA, 98, 9742-9747; Elbashir, et al. (2001) Nature, 411, 494-498). In another embodiment of RNAi, in vivo transcription of DNA constructs delivered into eukaryotic cells is utilized to introduce: 1) long dsRNAs which are enzymatically processed resulting in short dsRNAs, 2) small hairpin RNAs (shRNAs) or, 3) separate short complementary strands that can hybridize in vivo to form siRNA. The short dsRNA duplexes delivered by any of the mentioned methods trigger degradation of target RNAs mediated by incorporation of one of the strands in a RNA-induced silencing complex (RISC). It has been observed that double-stranded RNA longer than 30 base pairs can activate the interferon response causing nonspecific tranlational arrest and apoptosis.

Another type of regulation of gene expression by short RNA duplexes involves a class of genes that encode short dsRNA hairpin loops of about 24 to 30 basepairs in length that are processed to about 21 to 23 nt small RNAs. These short RNA duplexes, termed micro RNAs (miRNAs), function in the same pathway as siRNAs by associating with Argonaute proteins that are required for guiding target mRNA recognition. mRNAs cleave complementary target mRNAs in plants but appear to repress mRNA translation rather than mRNA cleavage in animals.

Another category of functional short RNAs, termed aptamers or intramers, are RNAs that are 23 to 400 nucleotides in length that display high affinity and selectivity towards a diverse array of targets, including both proteins and small molecules. Binding of aptamers to the target protein or molecule can block or otherwise modulate molecular function. Riboswitches are natural RNA aptamers involved in genetic regulation.

siRNA, shRNA, RNAi, RNA aptamers, e.g., riboswitches, and miRNA (termed short RNAs throughout this document) can be introduced into cells via classic gene transfer methods such as liposome-mediated transfection, electroporation, calcium shock, hydrodynamic shock or microinjection which requires chemical or enzymatic synthesis of siRNAs prior its application. They can also be generated intracellularly by transcription from plasmid DNA, integrated transgene loci, or retroviral, lentiviral or adenoviral constructs. Intracellular transcription of small RNA molecules is possible by cloning the siRNA templates into RNA polymerase III (pol III) transcription units, which normally encode the small nuclear RNA U6 or the human Rnase P RNA H1.

Typically, shRNAs are synthesized from vectors (e.g., plasmids or viral vectors). Generally, such synthesis is driven by type III RNA polymerase (Pol III) promoters. Pol III promoters are generally ubiquitous. A commonly used Pol III promoter is the U6 promoter, a strong constitutive promoter. In general, Pol III produces small, non-coding transcripts such as U6 small nuclear RNA (snRNA), which are not capped at the 5' and not polyadenylated at the 3' end. Pol III promoter elements include a distal sequence element (DSE), proximal sequence element (PSE), and TATA box, located 5' to the initiation site. Additionally, transcription driven by Pol III promoters initiates at defined nucleotides, terminates when the transcription encounters four or more Ts in succession, and the resulting transcripts carry 3'-overhangs of one to four Us (the termination sequence). Such 3'-overhangs are similar to the 3'-overhangs described as advantageous for siRNAs.

A growing body of evidence demonstrates that delivery of small RNAs to nontargeted cells can be deleterious and/or lead to nonspecific effects. Thus, for practical applications, selective delivery of small RNAs to the targeted cell population would be advantageous. Since Pol III promoters are essentially ubiquitous, DNA based small RNA delivery methods are not transcription targeted and are thus can be subject to these nonspecific and toxic side effects. Several methods of targeting small RNAs have been explored including LoxCre, Tet, ligand-affinity mediated liposome encapulated delivery etc. LoxCre and Tet are DNA based methods that rely of DNA regulatory regions placed 5' and 3' to the Pol III promoter. In some strategies, the DNA regulatory regions are placed within the Pol III promoter or replaces part of it. These methods can involve relatively large amounts of DNA (i.e. dicistronic as in the case of the LoxCre method where the Cre enzyme is under the expression control of a Pol II pomoter and the Lox sites when excised bring the shRNA into register with the full U6 promoter), can rely on the exogenous addition of Tet or some other antibiotic or ligand, and can be "leaky" since they have the full active U6 promoter in place. Thus a clear simple method for creating targeted monocistronic small RNA promoters would be advantageous.

Pol II promoters display a wide range of endogenous targeting patterns. The expression profiles of Pol II promoters include (non inclusive) tissue specificity, tumor specificity, organ specificity, radiation specificity, ligand specificity (i.e. including estrogen, tamoxifen etc), ultrasound specificity, inflammation specificity, viral specificity, and various disease specificities. It is common practice to identify genes activated by specific conditions using gene-chip micro arrays and clone their promoters for use as pol II promoters activated by the specific condition. Thus, there is a very large number of promoters with known expression profiles and a systematic way to identify addition promoters with clinically or scientifically interesting expression profiles. No similar collection of pol III promoters with interesting expression profiles exists.

### SUMMARY OF THE INVENTION

The present invention is based, at least in part, on the discovery that constructs generated to include promoters with a basal region(s) of a Pol III promoter and a regulatory region(s) of a Pol II promoter can be used to target and/or regulate expression of short RNA molecules in cells. Such constructs take advantage of some of both Pol III and Pol II promoter functions, namely obtaining RNA with a specific length and obtaining RNA with a specific expression profile. Some currently available constructs include full Pol III promoters, such as U6 promoters, which do not allow for specific targeting and/or regulation. The present application features constructs with a specific expression profile, similar to a Pol II expression profile.

According to a first aspect of the present invention, there is provided a nucleic acid construct in accordance with claim 1. Other aspects of the invention are defined in the dependent claims. The invention further provides *in vitro* methods for expressing RNAi agents, for inhibiting expression of a target genre in a cell, for analysing gene function in a cell, for validating a target as a therapeutic target, or for positive control of a biological effect of a small molecule test compounds, as described in the independent claims.

The present invention concerns genetic constructs that can be used to target and/or regulate expression of short RNA molecules in cells, particularly dsRNA molecules that can participate in RNA inhibition (RNAi), microRNA (miRNA) mediated expression regulation, such as mRNAs, short hairpin RNAs (shRNA), and miRNAs or RNA aptamers, e.g., riboswitches. Such regulation can be of many different types, such as spatial (e.g., in particular cells or tissues, including tumor-specific expression), temporal (occurring at particular times, such as particular development stages, and environmental (in response to particular environmental conditions), such as in response to radiation.

Generally, such genetic constructs include a sequence that will bind a RNA polymerase III complex, along with regulatory elements from a RNA polymerase n promoter region or regions. For example, such genetic constructs can be constructed as a fusion between a Pol III basal promoter region operatively linked with cis-acting regulatory region or regions (e.g., specific regulation enhancer and/or repressor elements) from a Pol II promoter region(s). Likewise, such genetic constructs can be constructed by mutagenizing a Pol II basal promoter region or regions such that it binds a Pol III complex. Such a mutant or modified sequence can be constructed by various methods, such as by mutation of a parent sequence or by chemical synthesis. However produced, the present genetic constructs that provide Pol III binding along with Pol II regulatory elements are referred to herein as "fusion promoters", or alternatively as "chimeric promoters".

Such fusion promoters can be used to provide regulated expression of inhibitory RNA molecules for the various applications of such inhibitory RNA molecules, generally involving gene knock-down or knock-out. For example, such uses include gene function analyses, drug development, gene pathway studies, development of RNA-based therapeutics, therapeutic and prophylactic applications, and as controls or indicators in small molecule drug screening and development

In one aspect, the disclosure features a nucleic acid construct that includes a Pol III/Pol II fusion promoter. The fusion promoter includes an RNA Polymerase III-binding basal promoter region, one or more cis-acting regulatory regions from a Pol II promoter operably linked with that basal promoter region. The cis-acting regulatory region or regions provide specific regulation of expression from the construct. In particular embodiments, a nucleic acid construct includes two linked Pol III/Pol II fusion promoters having different specific regulation characteristics.

In certain embodiments, the cis-acting regulatory region or regions provide cell-specific regulation; tissue-specific regulation; cell-cycle specific regulation; tumor-specific regulation in vivo; radiation-induced expression in vivo; estrogen-induced expression in vivo; ligand-induced expression in vivo; pattern specific expression in vivo such as expression in the same distribution as a virus or the same distribution as the expression of a viral gene or expression in the distribution similar to an RNA polymerase type II promoter like developmental program or immune specific or regional specific in vivo; ultrasound induced expression in vivo; heat induced expression in vivo; cold induced expression in vivo (e.g., metallothionein-1); glucose induced expression in vivo; hyperglycemic induced expression in vivo; disease induced expression in vivo; inflammation induced expression in vivo (e.g., acid-sensing ion channel (ASIC) polypeptides such as ASIC3 and mucosal addressin cell adhesion molecule-1 (MAdCAM-1) such as in inflammatory bowel disease (IBD), cyclooxygenase-2 (COX-2) such as in human pulmonary epithelial cells); tissue response induced expression in vivo; light induced expression in vivo (e.g., fos, NGFI-A, and NGFI-B); medication induced expression in vivo; apoptosis induced expression in vivo; spreading depression induced expression in vivo (e.g., atrial natriuretic peptide, COX-2, TNF-alpha, IL-1beta, galanin, and metalloproteinases such as MMP-9); infarction induced expression in vivo (e.g., P-selectin); pulmonary embolism induced expression in vivo; hypoxia induced expression in vivo (e.g., hypoxia inducible factor 1 alpha, vascular endothelial growth factor (VEGF), endothelial growth response 1 (Egr-1), erythropoietin); stroke induced expression in vivo; and combinations thereof.

In certain embodiments, the construct also includes a sequence encoding an RNAi agent operably linked with the fusion promoter, e.g., a shRNA or siRNA (i.e., encoding the two strands of an siRNA). In particular embodiments, the RNAi agent is targeted to mRNA of a gene associated with a disease or condition. A variety of such genes have been identified; some of which have been targeted and inhibited using RNAi.

In particular embodiments, the basal promoter region is from a Pol III promoter (e.g., a U6 basal promoter, an H1 basal promoter, a tRNA basal promoter); has the sequence of a Pol III basal promoter; is a mutated Pol II basal promoter that preferentially binds Pol III instead of Pol II.

In particular embodiments, the cis-acting regulatory region or regions include the entire regulatory region from a Pol II-transcribed gene, except for the basal promoter elements. In particular embodiments, the cis-acting regulatory region or regions include CMV early intermediate regulatory region or regions.

In another aspect, the invention also provides a vector that includes a Pol III/Pol II fusion promoter of the present invention, e.g., as described above or otherwise described herein.

In particular embodiments, the vector is a plasmid, a viral-based vector; a cosmid; a YAC, or a BAC. In particular embodiments, the vector is replication defective; the vector is replication competent.

Similarly, in another related aspect, the invention concerns a cell that includes a Pol III/Pol II fusion promoter of the invention operably linked with a coding sequence, such as an RNAi agent such as an shRNA or siRNA. The fusion promoter and linked RNAi agent-encoding sequence can be in a vector as described herein or incorporated in a chromosome(s).

In certain embodiments, the cell is in cell culture; is in an animal, e.g., a human, a feline, a canine, a bovine, a porcine, an ovine, an equine animal, a bird; a fungus; a plant. In particular cases, the cell is an animal cell, e.g., a human cell, a feline cell, a canine cell, a bovine cell, a porcine cell, an ovine cell, an equine cell; a bird cell; an insect cell; a plant cell.

In yet another related aspect, the invention provides a non-human transgenic organism that includes a plurality of cells that include a genetic constmct of the present invention.

In particular embodiments, such cells are as described above or otherwise herein; the organism is as described herein.

Also disclosed herein is a kit that includes a packaged amount of one or more genetic constructs of the present invention. Typically such a kit also includes additional component(s), such as instructions for use; the genetic construct is packaged in single use form; the genetic construct is in a vector, the genetic construct also includes a coding sequence operably linked with the Pol III/Pol II fusion promoter; the genetic construct is formulated in a pharmaceutical composition; the kit also includes a second active compound; the genetic construct is packaged in unit dose form

Another aspect of the invention concerns a pharmaceutical composition that includes a genetic construct of the invention, where the genetic construct also includes an RNAi agents such as an shRNA or siRNA sequence operatively linked with the fusion promoter, and a pharmaceutically acceptable carrier or excipient.

In certain embodiments, such pharmaceutical composition is formulated as an injectable composition; formulated for topical administration; formulated as a liposomal composition; includes a vector containing the construct; includes a viral vector that includes the construct; includes a plurality of vectors containing different constructs.

A further aspect concerns a method for making a genetic construct, of the present invention by operably linking a nucleic acid sequence encoding an RNAi agent with a Pol III/ Pol II fusion promoter of the invention.

In certain embodiments the construct, operably linked coding sequence, specific regulation properties, and/or other characteristics of the construct or its use are as described herein, e.g., RNAi agent is an shRNA, or siRNA.

In connection with the use of the present constructs and related materials, another aspect of the invention concerns an in vitro method for expressing an RNAi agent in a cell by maintaining a cell under expression conditions, where the cell includes a genetic construct of the present invention operably linked with a RNAi agent encoding sequence. In some embodiments RNAi agent is shRNA; siRNA.

Likewise, another aspect concerns an in vitro method for inhibiting expression of a target gene in a cell. The method involves transfecting the cell with a vector that includes a genetic construct of the present invention operably linked with a nucleic acid sequence encoding an RNAi agent targeted to the target gene, and maintaining the cell under expression conditions.

In particular embodiments the construct includes a tissue-specific regulatory element and the target gene is preferentially inhibited in cells of tissue corresponding to that tissue-specific regulatory element; the construct includes a tumor-specific regulatory element and the target gene is preferentially inhibited in cells of tumors corresponding to that tumor-specific regulatory element; the inhibition is induced in response to radiation; the inhibition is induced in response to the presence of an effective amount of a non-peptide and non-nucleotidic chemical species, e.g., an estrogen.

Further, another aspect concerns an in vitro method for analyzing gene function, which involves inhibiting expression of a gene in a cell, where the inhibiting is due to expression of an RNAi agent from a genetic construct of the present invention operably linked with a nucleic acid sequence encoding the RNAi agent; determining a biological change in the cell following the inhibiting, where such biological change is indicative of the function of the gene.

In particular embodiments, the determining involves comparing at least one biological characteristic with a control cell in which expression of the gene is not inhibited; the method also involves transfecting the cell with a vector that includes the genetic construct, e.g., a viral vector of a plasmid.

Another aspect provides an in vitro method for validating a target as a therapeutic target, and includes inhibiting expression of a putative therapeutic target gene in the cell, where the inhibiting is due to expression of an RNAi agent from a genetic construct of the invention operably linked with a nucleic acid sequence encoding the RNAi agent, and determining whether a biological change in the cell following that inhibiting corresponds with a therapeutic effect. Correspondence of the biological change with the therapeutic effect is indicative that the gene is a therapeutic target gene.

In another aspect, the invention provides a useful test control, thus providing an in vitro method for positive control of a biological effect of a small molecule test compound. The method involves contacting a first cell with a test compound; inhibiting a target gene in a comparison cell using expression of an RNAi agent from a genetic construct of the present invention operably linked with a sequence encoding the RNAi agent, and comparing the effect of the test compound in the first cell with the effect of inhibition of the target gene is the comparison cell.

In particular embodiments, the test compound is pre-selected to be active on the target gene; comparing includes determining whether the test compound has effects additional to the effects of the inhibiting by the RNAi agent.

In still another aspect, described herein is a method for treating a disease or condition in which inhibition of a target gene provides a beneficial effect. The method includes administering a pharmacologically effective amount of a nucleic acid construct, vector, cell, kit, or a pharmaceutical composition that includes a genetic construct of the invention operably linked with a sequence encoding an RNAi agent targeted to the target gene, to a subject suffering from or at risk of such disease or condition. The disease or condition can be, e.g., a cancer, an infectious disease, or a neurodegenerative disease, e.g., caused by mutations in SOD1 gene.

In particular embodiments, the vector is a plasmid ; the vector is a viral vector; the subject is a human; the subject is a non-human animal; the subject is a plant; RNAi agent is shRNA; RNAi agent is siRNA.

Also disclosed is the use of disclosed nucleic acid constructs, vectors, cells, kits, or pharmaceutical compositions in the treatment or prevention of a disease or condition wherein inhibition of a target gene provides a beneficial effect. The disease can be, e.g., a cancer, an infectious disease, or a neurodegenerative disease, e.g., one caused by mutations in SOD1 gene. In one aspect, the disclosure features use of a vector including a genetic construct comprising a Pol III/Pol II fusion promoter providing specific regulation of expression, operably linked with a sequence encoding an RNAi agent, wherein said fusion promoter comprises a RNA Polymerase III-binding basal promoter region and cis-regulatory region or regions from a Pol II promoter operably linked with said basal promoter region, wherein said cis-acting regulatory region or regions provide specific regulation of expression from said fusion promoter for treatment of a disease or condition wherein inhibition of a target gene provides beneficial effect

In particular embodiments, the vector is a plasmid; the vector is a viral vector; the RNAi agent is shRNA; the RNAi agent is siRNA.

Also within the invention is the use of disclosed nucleic acid constructs in the manufacture of a medicament for treatment or prevention of a disease or condition wherein inhibition of a target gene provides a beneficial effect. The medicament can be in any form described herein. The disease can be, e.g., a cancer, an infections disease, or a neurodegenerative disease, e.g., one caused by a mutation or mutations in SOD1 gene. In one aspect, the disclosure features use of a vector including a genetic construct comprising a Pol III/Pol II fusion promoter providing specific regulation of expression, operably linked with a sequence encoding an RNAi agent, wherein said fusion promoter comprises a RNA Polymerase III-binding basal promoter region and cis-regulatory region or regions from a Pol II promoter operably linked with said basal promoter region, wherein said cis-acting regulatory region or regions provide specific regulation of expression from said fusion promoter in preparation of a medicament for treatment of a disease or condition wherein inhibition of a target gene provides a beneficial effect.

In particular embodiments the vector is a plasmid; the vector is a viral vector, the RNAi agent is shRNA; the RNAi agent is siRNA.

As used in connection with the present constructs, the term "cis-acting regulatory region" or "regions" refers to nucleic acid sequences in the vicinity of a structural gene portion that affects the transcription of the structural gene.

As used in connection with nucleotide sequences, the term "encodes" indicates that the nucleotide sequence or molecule (generally DNA) contains a sequence that is complementary to a reference RNA sequence. Thus, a DNA sequence that encodes a particular RNA molecule can produce such RNA molecule when operatively linked with suitable control sequences and in the presence of necessary reaction components. In reference to amino acid sequences, the term "encodes" means that the indicated nucleotide sequence has a sequence that can be translated to the indicated amino acid sequence (in the case of an RNA sequence) or transcribed to a complementary RNA which can be translated to the indicated amino acid sequence (in the case of a DNA sequence) when such nucleotide sequences are operatively linked with suitable control sequences and in the presence of necessary reaction components.

As used herein, the terms "genetic construct" and "construct" refer to genetically engineered DNA molecules that include a basal promoter operatively linked with one or more enhancer and/or repressor regulatory regions. The construct can also include additional sequences, such as a shRNA coding region operatively linked with the basal promoter and enhancer and/or repressor regulatory regions.

The term "enhancer" refers to a DNA sequence which, when bound by a specific protein factor(s), enhances the level of expression of a gene, but is not sufficient alone to cause expression. In many cases, an "enhancer" is capable of enhancing expression of a gene even if located a substantial distance from the gene and in either sequence orientation relative to the gene.

In connection with the present invention, the term "kit" refers to a packaged manufacture (e.g. in a box, bottle, vial, or other container or combination of containers) that includes at least one reagent, e.g. a construct, for activating RNAi in a cell or organism. In particular embodiments, the kit is prepared containing one or more unit dose preparations of the present constructs.

In connection with the present genetic constructs, the term "unit dose" refers to a quantity of the construct designed and suitable for single use, e.g., for a single therapeutic administration or a single knock-down test for a gene.

The term "intron" refers to a sequence within the coding sequences of a gene that is not translated into protein. Such intron is transcribed into RNA but is removed (by RNA splicing) before the RNA is translated into protein.

The term "gene" includes genomic DNAs, cDNAs, RNA, or other polynucleotides that encode gene products, and includes introns and control sequences that affect transcription, translation, or other regulation and/or processing function.

The terms "exogenous gene" and "foreign gene" refer to a gene that has been obtained from an organism or cell type other than the organism or cell type in which it is expressed. Unless expressly indicated to the contrary; these terms also include a gene from the same organism that has been translocated from its normal situs in the genome. Similarly, the terms "exogenous sequence", "foreign sequence" and the like refer to nucleotide sequences from such other source or location.

As used herein the term "target gene" refers to a gene intended for downregulation (i.e., inhibition), such as by using RNA interference ("RNAi"). Similarly, the term "target RNA" refers to an RNA molecule, e.g., a mRNA molecule intended for downregulation (e.g., via RNAi-induced degradation).

As used herein in connection with the present nucleic acid constructs, the term "promoter" refers to a DNA sequence to which RNA polymerase can bind and initiate transcription of an operably linked coding sequence, along with associated regulatory elements that provide additional transcriptional control (e.g., binding elements for other transcription factors).

The term "Pol III promoter" refers to an RNA polymerase III promoter. Examples of Pol III promoters include, but are not limited to, the U6 promoter, the H1 promoter, and the tRNA promoters.

By "Pol II promoter" is meant an RNA polymerase II promoter. Examples of Pol II promoters include, but are not limited to, the Ubiquitin C promoter and the CMV early intermediate promoter.

In the context of the production a product from a gene or coding region, the term "expression" refers to the enzymatic synthesis of the product via transcription and/or translation processes, and includes expression in a cell(s) as well as transcription and/or translation of nucleic acid(s) in cell-free expression systems, cloning systems, and the like.

As used herein, the terms "RNA interference" and "RNAi" refer to a sequence-specific process by which a target molecule (e.g., a target gene, protein or RNA) is downregulated via downregulation of expression. Without being bound to a specific mechanism, as currently understood by those of skill in the art, RNAi involves degradation of RNA molecules, e.g., mRNA molecules within a cell, catalyzed by an enzymatic, RNA-induced silencing complex (RISC). RNAi occurs in cells naturally to remove foreign RNAs (e.g., viral RNAs) triggered by dsRNA fragments cleaved from longer dsRNA which direct the degradative mechanism to other RNA sequences having closely homologous sequences. As practiced as a technology, RNAi can be initiated by human intervention to reduce or even silence the expression of target genes using either exogenously synthesized dsRNA or dsRNA transcribed in the cell (e.g., synthesized as a sequence that forms a short hairpin structure).

As used herein, the term "RNAi agent" refers to an RNA (or RNA analog) that includes a sequence having sufficient sequence complementarity to a target RNA to direct RNAi to the target RNA. Such sequence complementarity may be complete complementarity, but may include a low level of mismatches, e.g., 3' or 5' terminal mismatches.

The term "RNA", "RNA molecule", and "ribonucleic acid molecule" refer to a polymer of ribonucleotides. Unless expressly indicated to the contrary, such ribonucleotides includes ribonucleotide analogs. Similarly, the terms "DNA", "DNA molecule", and "deoxyribonucleic acid molecule" refer to a polymer of deoxyribonucleotides. Unless expressly indicated to the contrary, such deoxyribonucleotides include deoxyribonucleotide analogs. DNA and RNA can be synthesized using enzymatic replication or transcription mechanisms (e.g., in a cell or in a cell-free enzymatic synthetic system), or can be chemically synthesized. RNA, in particular, can be post-transcriptionally modified on one or more ribonucleotides. DNA and RNA can be single-stranded (i.e., ssDNA and ssRNA) or multi-stranded, which is most commonly double stranded (i.e., dsRNA and dsDNA).

In the context of RNAi, the term "sequence-specific" means sufficient sequence complementarity to a target RNA molecule sequence to preferentially direct RNAi-induced degradation of such molecule. It does not mean that the RNAi agent is perfectly complementary to the target sequence or that there is no off target degradation directed by the agent.

The terms "mRNA" and "messenger RNA" are used conventionally to refer to a single-stranded RNA that has a sequence that encodes the amino acid sequence(s) of one or more polypeptide chains. Such coding sequence is translated during protein synthesis, producing the corresponding amino acid sequence.

The term "transcript" refers to a RNA molecule transcribed from a DNA or RNA template by a RNA polymerase. The term "transcript" includes RNAs that encode polypeptides (i.e., mRNAs) as well as noncoding RNAs ("ncRNAs").

As used herein, the terms "small interfering RNA" and "short interfering RNA" ("siRNA") refer to a short RNA molecule, generally a double-stranded RNA molecule about 10-50 nucleotides in length (the term "nucleotides" including nucleotide analogs), preferably between about 15-25 nucleotides in length. In most cases, the siRNA is 17, 18, 19, 20, 21, 22, 23, 24, or 25 nucleotides in length. Such siRNA can have overhanging ends (e.g., 3'-overhangs of 1, 2, or 3 nucleotides (or nucleotide analogs). Such siRNA can mediate RNA interference.

As used in connection with the present invention, the term "shRNA" refers to an RNA molecule having a stem-loop structure. The stem-loop structure includes two mutually complementary sequences, where the respective orientations and the degree of complementarity allow base pairing between the two sequences. The mutually complementary sequences are linked by a loop region, the loop resulting from a lack of base pairing between nucleotides (or nucleotide analogs) within the loop region.

The term "subject" refers to a living higher organism, such as an animal (e.g., a mammal or a bird) or a plant. Examples of animal subjects include humans, monkeys, cows, horses, sheep, goats, dogs, cats, mice, rats, and transgenic non-human derivatives or non-human variants thereof.

The term "treatment", as used herein, means the application or administration of a therapeutic agent to a subject (or application or administration of a therapeutic agent to an isolated tissue or cell line from a subject) who has a disease or condition, a symptom of a disease or condition, a predisposition toward a disease or condition, or is otherwise at risk of contracting the disease or condition. Such treatment is intended to relieve at least in part at least one symptom of the disease or condition, to alter the course of the disease or condition, and/or to reduce the likelihood that the subject will develop the disease or condition, e.g., to cure, heal, alleviate, relieve, alter, remedy, ameliorate, improve, or affect the disease or condition, the symptoms of the disease or condition, the predisposition toward a disease or condition, or the likelihood of developing the disease or condition.

As used herein, the term "therapeutic agent" means a composition, e.g., a molecule that produces a therapeutic effect when administered or applied to a subject suffering from or at risk of a disease or condition. Such therapeutic agents can, for example, be small molecules, peptides, antibodies, ribozymes, antisense oligonucleotides, chemotherapeutic agents, and radiation.

The term "effective amount", as used here in, is defined as that amount sufficient to produce a particular pharmacological effect.

The tem therapeutic amount" refers to an amount sufficient to treat or prevent a particular disease or condition. Such amount can vary depending on such factors as the size, weight, and condition of the subject, the type of the disease or condition, the particular agent being administered, and the method and route of administration of the agent. One of ordinary skill in the art determine such therapeutic amount of the agent without undue experimentation.

The term "mutation" refers to a substitution, addition, or deletion of a nucleotide or small number of nucleotides within a gene sequence. Such mutations can result in aberrant production (e.g., misregulated production) of the protein encoded by the gene sequence, production of an aberrant or variant product, or can be silent.

The term "nucleoside" refers to a molecule having a purine or pyrimidine base covalently linked to a ribose or deoxyribose sugar. Exemplary nucleosides include adenosine, guanosine, cytidine, uridine and thymidine. The term "nucleotide" refers to a nucleotide having one or more phosphate groups joined in ester linkages to the sugar moiety. Exemplary nucleotides include nucleoside monophosphates, diphosphates and triphosphates. The terms "polynucleotide" and "nucleic acid molecule" are used interchangeably herein and refer to a polymer of nucleotides joined together by a phosphodiester linkage between 5' and 3' carbon atoms.

The term "pharmaceutical composition" as used herein, refers to an active agent formulated with one or more compatible fillers, diluents, carriers, excipient, or encapsulating substances which are suitable for administration to a human or other animal subject.

Certain methods described herein include comparing a value, level, feature, characteristic, property, etc. to a "suitable control" (also referred to as an "appropriate control"). Such a control is any control or standard acceptable to one of ordinary skill in the art useful for comparison purposes. In one embodiment, a "suitable control" or "appropriate control" is a value, level, feature, characteristic, property, etc. determined prior to performing an RNAi methodology, as described herein. For example, a transcription rate, mRNA level, translation rate, protein level, biological activity" cellular characteristic or property, genotype, phenotype, etc. can be determined prior to introducing an RNAi agent of the invention into a cell or organism or in a reference cell or organism.

The term "upstream" refers to nucleotide sequences that precede, e.g., are on the 5' side of, a reference sequence.

The term "downstream" refers to nucleotide sequences that follow, e.g., are on the 3' side of, a reference sequence.

As used in connection with the present invention, the term "vector" refers to a nucleic acid molecule capable of transporting another nucleic acid to which it has been linked into a cell. Such vector include plasmids, viral vectors, cosmids, YACs, BACs, and the like. "Plasmids" are small circular double stranded DNA molecules which replicate independently of the cellular genome. Typically such plasmids include one or more sites into which additional DNA segments can be inserted and ligated. "Viral vectors", which are vectors based on viral genomes, which may be engineered and/or recombinant viral vectors. Often, such viral vectors have non-essential genes removed, may be engineered to add cloning sites, and/or may be selected or modified to be an attenuated virus and/or to be replication defective or to have other selected properties. Examples of viral vectors include vectors derived lentiviral (e.g., HIV, SIV, EAIV, FIV), adenovirus, adeno-associated virus, oncoretrovirus, pox virus (e.g., vaccinia virus and caarypox virus), herpesvirus, foamyvirus, MMLV virus (Moloney murine leukemia virus), baculovirus, alphavirus (e.g., Semliki Forest virus (SFV), Sindbis virus (SIN), and Venezuelan Equine Encephalitis virus (VEE). Three different types of alphavirus vectors have been constructed. *I Replication-deficient vectors:* RNA molecules containing the viral nonstructural genes (nsP1-4) and the foreign gene of interest are packaged into alphavirus particles with the aid of a helper vector containing the viral structural genes. The generated recombinant alphavirus particles are capable of infection of host cells, but because no viral structural genes are accommodated, no further virus replication occurs. The obtained transgene expression is therefore of a transient nature. *II Replication-competent vectors:* In contrast to the suicide vectors described above, these vectors contain a second subgenomic promoter and the foreign gene of interest added to the full-length alphavirus genome. Infection of host cells with replication-competent particles will obviously lead to virus replication. *III Layered DNA-vectors:* An RNA polymerase II expression cassette is introduced to drive the transcription of a self-amplifying RNA (replicon) vector, which allows direct use of plasmid DNA for transfection and expression studies (Berglund *et al.,* 1996, Dubensky *et al.,* 1996).), and parvovirus vectors.

Additional embodiments will be apparent from the Detailed Description and from the claims.

### BRIEF DESCRIPTION OF THE DRAWINGS

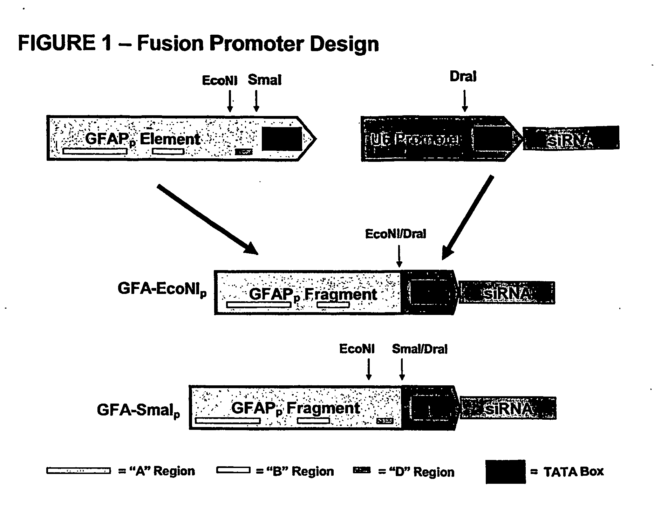

**Fig. 1** schematically shows the design of Pol III/Pol II fusion promoters utilizing U6 basal promoter with GFAP regulatory elements. Two chimeric promoters where created: 1) A chimeric promoter that included the A and B cis-acting elements of the human GFAPp element linked to the core promoter of U6 termed GFAP-EcoNI which was cloned into a plasmid termed pGFAP-EcoNI (or pGFAP-EcoNI-Control) and 2) A chimeric promoter that included the A, B, and D regions of the human GFAPp element linked to the core promoter of U6 termed GFAP-SmaI and was cloned into a plasmid termed pGFAP-SmaI (or pGFAP-SmaI-Control). Two additional plasmids were created that contained the respective chimeric promoters driving the expression of shRNAs directed against eGFAP termed pGFAP-EcoNI-eGFP and pGFAP-SmaI-eGFP. These four plasmids were used to demonstrate shRNA expression from the chimeric promoters and examine the transcript expression program of the chimeric promoters.

### DETAILED DESCRIPTION OF THE PREFERRED EMBODIMENTS

### I. General

Double-stranded RNA (dsRNA)-induced sequence-specific gene silencing is known as RNA interference (RNAi). There is a growing appreciation of the vast therapeutic potential of RNAi for treating a wide range of diseases including cancers and infectious diseases (Bi et al. Curr Gene Ther 2003, 3(5):411-417; Brisibe et al. Trends Biotechnol 2003, 21(7):306-311; Caplen Expert Opin Biol Ther 2003, 3(4):575-586; Lieberman et al. Trends Mol Med 2003, 9(9):397-403; Wang et al. World J Gastroenterol 2003, 9(8):1657-1661; Wolff & Herweijer Ernst Schering Res Found Workshop 2003(43):41-59). In addition, RNAi is a powerful tool for basic scientists to explore the functions of genes through reverse genetic manipulations (Scherr et al. Curr Med Chem 2003, 10(3):245-256; Szweykowska-Kulinska et al. Acta Biochim Pol 2003, 50(1):217-229; Wimmer Nat Rev Genet 2003, 4(3):225-232). As the role of RNAi in both science and medicine continues to grow, targeted delivery of short dsRNAs (siRNA) to the desired tissue will become increasingly important. Some reports have indicated that chemically synthesized siRNA can be targeted using physical targeting technologies like nanogels and PEGylated immunoliposomes. DNA-based RNAi approaches offer the advantage of being both less labile than unmodified dsRNA and displaying amplification, i.e. many dsRNAs can be expressed from a single delivered DNA promoter construct. Described approaches for targeting DNA-based RNAi constructs include Lox/Cre based approaches and physical targeting of the DNA constructs using nanogels or PEGylated immunoliposomes. However, the present simple transcriptional targeting method for delivery of siRNA represents an important advance in RNAi therapy.

In the DNA-based approach to RNAi, DNA based constructs are used to express short dsRNAs in cells in the form of either short hairpin RNA (shRNA) or expression of both strands of the double stranded complementary sequences. Each of these approaches has typically relied on the use of an RNA polymerase type III promoter so that transcription is terminated at the appropriate length. While some uses of RNA polymerase type II promoters have been described, generally an RNA polymerase promoter type II yields much longer RNA molecules which has been shown to activate cellular inflammatory cascades and other non-specific effects.

Both RNA polymerase type II promoters and RNA polymerase type III promoters have a structure that includes a core (or basal) promoter and a collection of enhancers, silencers and other elements. Typically, the respective holoenzyme attaches to the core promoter region along with a collection of transcription factors to create the preinitiation complex. The transcription rate is then largely controlled by the action of enhancers, silencers and other elements located both 5' and 3' to the core promoter. This basic mechanism is responsible for the complex trascriptional targeting displayed by RNA polymerase type II promoters including (non-exhaustive) tissue specific, tumor specific, radiation inducible, estrogen inducible, cell-cycle dependent and organism specific promoters.

While RNA polymerase III promoters have the advantage of appropriate termination, currently characterized RNA polymerase type III promoters display nearly ubiquitous expression. In contrast to RNA Pol III promoters, RNA polymerase type II promoters display a rich array of transcriptional control, but are still not generally appropriate for use for expression of siRNAs or shRNAs due to the production of long RNA molecules.

Thus, the present invention concerns the use enhancers, silencers and other regulatory elements from an RNA polymerase type II promoter to regulate the transcription rate of an RNA polymerase type III preinitiation complex to create a chimeric RNAi expression promoter. Changing an RNA polymerase type II promoter such that an RNA polymerase type III would form a transcriptional complex instead of a polymerase type II yields an RNAi promoter with expression characteristics similar to the parent RNA polymerase type II promoter while retaining the polymerization and termination properties characteristics of RNA Pol III.

There are several potential ways to accomplish this, including exchanging the core promoter of an RNA polymerase type II promoter with one from an RNA polymerase type III promoter, and creating mutations to the core promoter region for an RNA polymerase II promoter such that it preferentially binds a RNA Polymerase III. Further, different combinations of enhancers, silencers and other elements from both promoters may be combined to achieve an RNAi promoter with desired expression characteristics.

Thus, the present invention concerns specifically regulatable genetic constructs for the expression of RNAs, in particular RNA agents for activating RNAi such as shRNAs and siRNAs. Such regulation can be spatial, temporal, or environmental. The present regulatable genetic constructs include fusion promoters that include a basal promoter that binds an RNA polymerase III, operably linked with at least one additional regulatory element from a RNA polymerase II promoter region.

Surprisingly, such fusion promoters offer the advantageous synthetic properties associated with Type III polymerases, while also offering the regulatory range and flexibility of Type II polymerase regulation.

Thus, the present invention provides compositions for RNA interference and methods for preparing and using such compositions. The compositions are useful for the range of applications of RNAi, including determining and analyzing gene functions for both normal and mutant genes, determining and analyzing gene pathways, analyzing and validating putative drug targets, and targeting genes for therapeutic and prophylactic applications. Advantageously, such applications can be carried out in a regulated manner, with a variety of different regulatory characteristics available for use.

### II. Fusion Promoters

### A. Design and Construction of Pol III/ Pol II Fusion (Chimeric) promoters

A variety of methods can be used to produce the present fusion promoters using standard techniques. On such approach is to create a fusion promoters is to replace a RNA Pol II basal promoter with a RNA Pol III basal promoter (e.g., using conventional cloning techniques). The resulting construct has the regulatory elements from a Pol II regulatory region associated with a Pol III basal promoter. The converse can also be performed, with one or more regulatory elements from a Pol II regulatory region linked with a Pol III basal promoter. In either case, the result is a chimeric nucleic acid, with a Pol III basal promoter and at least one additional regulatory element from a Pol II regulatory region.

For example, the U6 promoter, an RNA polymerase type III promoter, has a basal or core promoter and two regulatory elements termed the PSE and the DSE. The glial acid fibrillary protein (GFAP) protein promoter, a glial cell selective promoter, has a core promoter and three regulatory regions 5' to the core promoter and one 3' to the promoter. It is possible to create a chimeric promoter consisting of the basal promoter of the U6 and the regulatory regions of the GFAP promoter that displays tissue selective expression ofRNAi. This evidence supports the broader concept that this methodology could be used to systematically create a broad range ofRNAi promoters with interesting expression targeting characteristics. These include, but are not limited to, creation of other tissue specific promoters, radiation inducible promoters, ligand inducible promoters, estrogen inducible promoters, organism specific promoters (i.e. viral specific promoters that express in the same general distribution of the replication of the virus or parasite or yeast specific promoters), tumor specific promoters, cell-cycle dependent promoters and developmental stage specific promoters.

### B. Mutated promoters

It has been demonstrated that simple point mutations to the TATA box region can convert a promoter from an RNA polymerase type II promoter into an RNA polymerase type III promoter. This approach can be used to convert RNA polymerase type II promoters into RNAi promoters while retaining the targeting characteristics of the RNA polymerase type II promoter. For example, mutation of the TATA box of glial fibrillary acid protein (GFAP) from ATAA to AATAT converts it into an RNAi promoter with tissue selective expression.

Similarly, a wide range of RNA polymerase type III promoters can be converted into RNA polymerase type III promoters using this simple methodology. This results in the creation of targeting RNAi promoters with a variety of characteristics including, but not be limited to, creation of other tissue specific promoters, radiation inducible promoters, ligand inducible promoters, estrogen inducible promoters, organism specific promoters (i.e. viral specific promoters that express in the same general distribution of the replication of the virus or parasite or yeast specific promoters), tumor specific promoters, cell-cycle dependent promoters and developmental stage specific promoters.

### C. More general mixing of regulatory elements and combination of directed mutagenesis and mixing of regulatory elements to create RNAi promoters.

Creation of RNAi promoters can include the mixing of multiple types of regulatory elements (in addition to the mixing of core promoters or in lieu of mixing of core promoters) or a combination of directed mutagenesis with any degree of combination of mixing of regulatory elements. In addition, compound RNAi promoters with regulatory elements from multiple types ofRNA polymerase type II promoters may be used to produce more specific control of RNAi expression. This would include, but is not limited to, using regulatory elements from two promoters with similar expression characteristics like two promoters that are glial specific to improve the targeting of RNAi expression to glial cells or using regulatory elements from a tissue specific RNAi promoter with elements from a tumor specific RNAi promoter to create a chimeric promoter capable of targeting tumors in particular tissues. Including or leaving out specific regulatory elements can be used to control the degree of targeting or even expand or completely change the targeting of the RNAi promoter.

### D. Pol III Promoters

Many different Pol III promoters can be used in construction of the present Pol III/Pol II fusion promoters. Well-known examples of promoters include the U6 promoter, the H1 promoter, and tRNA promoters (e.g., selenocysteine tRNA gene (TRSP)). The sequences of those promoters are known and can be manipulated by conventional molecular biology methods to create recombinant nucleic acid constructs.

Other Pol III promoters include the 7SL RNA promoter (e.g., Arabidopsis, human, or mouse), and the RNase P RNA (RPPH1) gene promoter (e.g., from the domestic dog (Canis familiaris)), and the adenoviral VA1 polymerase III (pol III) promoter. Additional Pol III promoters that are known or identified can also be used. Such promoters can be identified by methods similar to the methods by which prior Pol III promoters have been identified.

### E. Pol II Regulatory Region Elements

Similarly to Pol III promoters, many different Pol II regulatory regions and elements are known and more are being identified. Such regions and elements can be used to construct the present fusion promoters. Pol II promoters and their associated regulatory elements are notable for the variety of specific regulation demonstrated for the corresponding genes. Such regulatory elements can be incorporated in the present Pol III/Pol II fusion promoters, thereby providing specific regulation of expression from the fusion promoter of an operably linked coding sequence.

Examples of the specific regulation provided by such Pol II regulatory elements include cell type specific, tissue specific, cell cycle specific, development stage specific, radiation induced, and hormone induced regulation. Elements providing different types of regulation can even be used in combination to provide multiple types of regulation with a single construct and/or additive or synergistic specific regulatory effects.

### III. Nucleic Acids Encoding RNAi Agents and Other RNA Molecules

The present nucleic acid constructs include those with a fusion promoter with an operably linked nucleic acid sequence encoding an RNAi agent or other short RNA such as micro RNA (miRNA). Such RNAi agents include siRNAs and shRNAs, as well as longer sequences that are processed by RNAi machinery to shorter sequences intracellularly.

A shRNA-encoding nucleic acid sequence or molecule includes a first sequence (or portion) and a second sequence (or portion) that have nucleotide sequences such that the RNA sequences encoded by those portions are sufficiently complementary to hybridize with each other to form a duplex or double-stranded stem portion. Such sufficient complementarity does not require that the portions are fully or perfectly complementary. The stem-forming portions are connected by a portion (referred to as a loop-portion or loop-encoding portion) having a sequence such that the encoded RNA from that portion does not anneal or hybridize to other portions of the shRNA (i.e., forms a single strand loop). Such shRNA-encoding nucleic acid sequences or molecules are transcribed, thereby forming shRNAs. shRNAs can also include one or more bulges, i.e., extra nucleotides that create a small nucleotide "loop" in a portion of the stem, for example a one-, two- or three-nucleotide loop. The encoded stem portions can be the same length, or one portion can include an overhang of, for example, 1-5 nucleotides (e.g., a 3-overhang).

For shRNA, one strand of the stem portion of the encoded shRNA is sufficiently complementary (e.g., antisense) to a target RNA (e.g., mRNA) sequence to mediate degradation or cleavage of that target RNA via RNA interference (RNAi). The antisense portion can be on the 5' or 3' end of the stem. The stem-encoding portions of a shRNA-encoding nucleic acid (or stem portion of a shRNA) are typically about 15 to about 50 nucleotides in length. When used in mammalian cells, the length of the stem portions can be selected to be less than about 30 nucleotides to avoid provoking non-specific responses like the interferon pathway. In non-mammalian cells, the stem can be longer than 30 nucleotides. In fact, a stem portion can include much larger sections complementary to the target mRNA (up to, and including the entire mRNA). The loop portion in the shRNA (or loop-encoding portion in the encoding DNA) can be of various lengths, e.g., about 2 to about 20 nucleotides in length, i.e., about 2, 3, 4, 5, 6, 7, 8, 9, 10, 11, 12, 13, 14, 15, 16, 17, 18, 19, 20 or more nucleotides in length. Certain loop portions are or include a 4 nucleotide sequence, referred to as a "tetraloop" sequence. Without limitation, such tetraloop sequences include the sequences GNRA, where N is any nucleotide and R is a purine nucleotide, GGGG, and UUUU.

For siRNAs, the construct can be designed such that expression is from a bicistronic sequence, with inverted regions for the sense and antisense strands of the double stranded siRNA. The two complementary strands can then hybridize in the cell. Alternatively, expression of each strand can be driven from separate fusion promoters, which may be the same or different. In the case of different promoters, the promoters may be selected such that together they increase the specificity of regulation of dsRNA, e.g., a cell type specific promoter combined with a tumor specific promoter.

The sequence of the antisense portion of a siRNA or shRNA can be designed by selecting an 18, 19, 20, 21, 22, 23, 24, 25 nucleotide, or longer, sequence from within the target RNA (e.g., mRNA), for example, from a region 100 to 200 or 300 nucleotides upstream or downstream of the start of translation. In general, the sequence can be selected from any portion of the target RNA (e.g., mRNA) including the 5' UTR (untranslated region), coding sequence, or 3' UTR. This sequence can optionally follow immediately after a region of the target gene containing two adjacent AA nucleotides. The last two nucleotides of the nucleotide sequence can be selected to be UU. shRNAs and longer dsRNAs so generated are processed under appropriate conditions (e.g., in an appropriate in vitro reaction or in a cell) by RNAi machinery (i.e., Dicer and/or RISC complexes) to generate siRNAs. Single stranded RNAs (including shRNAs and miRNAs) can be synthesized exogenously or can be transcribed in vivo from an RNA polymerase (e.g., a Pol II or Pol III polymerase).

### IV. Vectors and Host Cells

The invention also concerns vectors that include the present constructs. Of particular benefit are expression vectors, especially those for expression in eukaryotic cells. Such vectors can, for example, be viral, plasmid, cosmid, or artificial chromosome (e.g., yeast artificial chromosome) vectors.

Typically, plasmids are circular, dsDNA elements that include one or more cloning sites for insertion of selected DNA sequences, e.g., coding sequences. Such plasmids may include a functional origin of replication and thus are replication competent, or may be replication defective.

In addition to plasmids, viral vectors (e.g., replication defective retroviruses, lentiviruses, adenoviruses and adeno-associated viruses) can also be advantageously used. A large number of such viral vectors have been developed having a broad variety of different properties. For example, such viral vectors may be replication defective retroviruses, adenoviruses and adeno-associated viruses. Techniques and procedures for producing recombinant retroviruses and for infecting cells in vitro or in vivo with such viruses are provided in Current Protocols in Molecular Biology, Ausubel, F. M. et al. (eds.) Greene Publishing Associates, (1989), Sections 9.10-9.14 and other standard laboratory manuals. Examples of suitable retroviruses include pLJ, pZIP, pWE and pEM which are well known to those skilled in the art. Examples of suitable packaging virus lines include .psi.Crip, .psi.Cre, .psi.2 and .psi.Am.

The genome of adenovirus can be manipulated such that it encodes and expresses a regulatable shRNA constructs, as described herein, but is inactivated in terms of its ability to replicate in a normal lytic viral life cycle. See for example Berkner et al. (1988) BioTechniques 6:616; Rosenfeld et al. (1991) Science 252:431-434; and Rosenfeld et al. (1992) Cell 68:143-155. Suitable adenoviral vectors derived from the adenovirus strain Ad type 5 d1324 or other strains of adenovirus (e.g., Ad2, Ad3, Ad7 etc.) are well known to those skilled in the art. Alternatively, an adeno-associated virus vector such as that described in Tratschin et al. (1985) Mol. CelL Biol. 5:3251-3260 can be used to express a trausactivator fusion protein.

Other viral vector alternatives include lentiviral vectors. Such vectors and their preparation and use are described, for example, in U.S. Patent documents 6,924,123; 6,863,884; 6,830,892; 6,818,209; 6,808,923; 6,799,657, all of which are incorporated herein in their entireties.

The vectors of the invention can advantageously include a RNAi agent-encoding (e.g., shRNA-encoding) nucleic acid operatively linked with Pol III/Pol II fusion promoters. Other elements included in the design of a particular expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The vectors described herein can be introduced into cells or tissues by any one of a variety of known methods within the art. Such methods are described for example in Sambrook et al., Molecular Cloning; A Laboratory Manual, Cold Spring Harbor Laboratory, New York (1992). See, also, Ausubel et al., Current Protocols in Molecular Biology, John Wiley and Sons, Baltimore, Md.(1989); Hitt et al., "Construction and propagation of human adenovirus vectors," in Cell Biology: A Laboratory Handbook, Ed. J. E. Celis., Academic Press. 2-sup.nd Edition, Volume 1, pp: 500-512, 1998; Hitt et al., "Techniques for human adenovirus vector construction and characterization," in Methods in Molecular: Genetics, Ed. K. W. Adolph, Academic Press, Orlando, Fla., Volume 7B, pp:12-30,1995; Hitt, et al., "Construction and propagation of human adenovirus vectors," in Cell Biology: A Laboratory Handbook," Ed. J. E. Celis. Academic Press. pp:179-490, 1994 . The methods include, for example, stable or transient transfection, lipofection, electroporation and infection with recombinant viral vectors. The term "transfecting" or "transfection" is intended to encompass all conventional techniques for introducing nucleic acid into host cells, including calcium phosphate coprecipitation, DEAE-dextran-mediated transaction, lipofection, electroporation and microinjection. Suitable methods for transfecting host cells canbe found in Sambrook et al. (Molecular Cloning: A Laboratory Manual, 2nd Edition, Cold Spring Harbor Laboratory press (1989)), and other laboratory textbooks.

For plant cells, a Ti plasmid or viral vector is often used. For example, such plasmids and viral vectors can be used to transfect host plant cells via *Agrobacterium tumefaciens-*mediated transfection (for plant cells susceptible to *A. tumefaciens* infection), or can be directly inserted in cells, e.g., using microinjection, particle bombardment, or electroporation. In other methods, protoplasts can be made from plant cells and then transfected.

The number of host cells transformed with a nucleic acid constructs of the invention will depend, at least in part, upon the type of recombinant expression vector and the type of transfection technique used. Nucleic acid can be introduced into a host cell transiently, or for long-term expression. For long-term expression, the nucleic acid is stably integrated into the genome of the host cell or remains as a stable episomal element.

For integration of nucleic acid into host cell DNA, typically a gene is used that encodes a selectable marker (e.g., drug resistance) is introduced into the host cells along with the nucleic acid of interest. A variety of such selectable markers are commonly used, such as the drugs hygromycin and neomycin. Selectable markers can be introduced on a separate plasmid or other vector from the nucleic acid of interest or, are introduced on the same vector. Host cells transfected with a nucleic acid construct of the invention (e.g., a recombinant expression vector) and a gene for a selectable marker can be identified by selecting for cells using the selectable marker.

The present nucleic acid constructs can be introduced into eukaryotic cells growing in culture in vitro by conventional transfection techniques (e.g.", calcium phosphate precipitation, DEAE-dextran transfection, electroporation, and other methods). Cells can also be transfected in vivo, for example by application of a delivery mechanism suitable for introduction of nucleic acid into cells in vivo, such as viral vectors (see e.g., Ferry, N et al. (1991) Proc. Natl. Acad. Sci. USA 88:8377-8381; and Kay, M. A. et al. (1992) Human Gene Therapy 3:641-647), adenoviral vectors (see e.g., Rosenfeld, M. A. (1992) Cell 68:143-155; and Herz, J. and Gerard, R D. (1993) Proc. Natl. Acad. Sci. USA 90:2812-2816), receptor-mediated DNA uptake (see e.g., Wu, G. and Wu, C. H. (1988) J. Biol. Chem. 263:14621; Wilson et al. (1992) J. Biol. Chem. 267:963-967; and U.S. Pat. No. 5,166,320), direct infection of DNA (see e.g., Acsadi et al. (1991) Nature 332: 815-818; and Wolff et al. (1990) Science 247:1465-1468) or particle bombardment (see e.g., Cheng, L. et al. (1993) Proc. Natl. Acad. Sci. USA 90:4455-4459; and Zelenin, A. V. et al. (1993) FEBS Letters 315:29-32). Thus, in the present invention, cells can be transfected in vitro or ex vivo, and administered to a subject or, alternatively, cells can be directly modified in vivo.

Another aspect of the invention pertains to host cells into which a host construct of the invention has been introduced, i.e., a "recombinant host cell." It is understood that the term "recombinant host cell" refers not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell, although eukaryotic cells are preferred. Exemplary eukaryotic cells include mammalian cells (such as Chinese hamster ovary cells (CHO) or COS cells). Other suitable host cells are known to those skilled in the art.

### V. Transgenic Animals

The present invention also concerns non-human transgenic organisms, such as non-human animals, which are animals that have at least some cell that express a transgene. Such nonhuman transgenic animals can be used, for example, in screening assays designed to identify active agents or compounds, e.g., drugs, pharmaceuticals, etc., which are capable of ameliorating detrimental symptoms of selected disorders, such as disease and disorders associated with mutant or aberrant gene expression, gain-of-function mutants and neurological diseases and disorders.

A transgene is a construct that has been or is designed to be incorporated into a cell, e.g., a mammalian cell, that is incorporated in a living animal such that the construct containing the nucleotide sequence is expressed. The transgene may include a sequence (e.g., a RNAi agent-encoding sequence) that is endogenous or exogenous to the non-human transgenic animal. A transgene may be present as an extrachromosomal element in some or all of the cells of a non-human transgenic animal or integrated into some or all of the cells, more preferably into the germline DNA of the non-human animal (i.e., such that the transgene is transmitted to all or some of the animal's progeny), thereby directing expression of the product of the transgene in one or more cell types or tissues of the transgenic animal. Unless clearly indicated to the contrary, reference to a non-human transgenic animal herein will mean that the transgene is present long term as opposed to transiently, e.g., stably incorporated in the chromosomes of germline cells. In many cases, it is desirable for the transgene to be incorporated in the genome at a site such that it does not interfere with endogenous gene expression.

A present transgenic non-human animal can be, e.g., a mammal, a bird, a reptile or an amphibian. Suitable mammals for uses described herein include: rodents; ruminants; ungulates; domesticated mammals; and dairy animals. Preferred non-human animals include: rodents, goats, sheep, camels, cows, pigs, horses, oxen, llamas, chickens, geese, and turkeys. In a preferred embodiment, the non-human animal is a mouse or a rat.

Various methods for producing transgenic animals have been described (see, e.g., Watson, J. D., et al., "The Introduction of Foreign Genes Into Mice," in Recombinant DNA, 2d Ed., W. H. Freeman & Co., New York (1992), pp. 255-272; Gordon, J. W., Intl. Rev. Cytol. 115:171-229 (1989); Jaenisch, R., Science 240: 1468-1474 (1989); Rossant, J., Neuron 2: 323-334 (1990)). An exemplary protocol for the production of a transgenic pig can be found in White and Yannoutsos, Current Topics in Complement Research: 64th Forum in Immunology, pp. 88-94; U.S. Pat. No. 5,523,226; U.S. Pat. No. 5,573,933; PCT Application WO93/25071; and PCT Application WO95/04744. An exemplary protocol for the production of a transgenic rat can be found in Bader and Ganten, Clinical and Experimental Pharmacology and Physiology, Supp. 3:S81-S87, 1996. An exemplary protocol for the production of a transgenic cow can be found in Transgenic Animal Technology, A Handbook, 1994, ed., Carl A. Pinkert, Academic Press, Inc. An exemplary protocol for the production of a transgenic sheep can be found in Transgenic Animal Technology, A Handbook, 1994, ed., Carl A. Pinkert, Academic Press, Inc. Certain exemplary methods are set forth in more detail bellow.

### A. Pronucleus Injection

Transgenic non-human animals can be produced by injecting a nucleic acid construct according to the present invention into egg cells. Embryonic target cells at various developmental stages are used to introduce the transgenes. Different method are used depending on the stage of development of the embryonal target cell(s). Exemplary methods for introducing transgenes include, but are not limited to, microinjection of fertilized ovum or zygotes (Brinster, et al., Proc. Natl. Acad. Sci. USA (1985) 82: 4438-4442), and viral integration (Jaenisch R., Proc. Natl. Acad. Sci. USA (1976) 73:1260-1264; Jahner, et al., Proc. Natl. Acad. Sci.USA (1985) 82: 6927-6931; Van der Putten, et al., (1985) Proc. Natl. Acad. Sci (USA) 82: 6148-6152). Procedures for embryo manipulation and microinjection are described in, for example, Manipulating the Mouse Embryo (Cold Spring Harbor Laboratory Press, Cold Spring Harbor, N.Y.,1986 ). Similar methods are used for production of other transgenic animals.

### B. Transgenic Animals from Embryonic Stem Cells

Another method of making non-human transgenic animals, e.g., transgenic mice, recombinant DNA molecules (e.g., constructs or transgenes) are introduced into non-human embryonic stem (ES) cells, e.g., mouse cells. Resulting recombinant ES cells are then microinjected into mouse blastocysts using standard techniques.

In general, ES cells are obtained from pre-implantation non-human embryos and cultured in vitro (Evans, M J., et al., Nature 292: 154156 (1981); Bradley, M. O. et aL, Nature 309: 255-258 (1984); Gossler, et al., Proc. Natl. Acad. Sci. (USA) 83:9065-9069 (1986), Robertson et al., Nature 322: 445448 (1986)). Any ES cell line that is capable of integrating into and becoming part of the germ line of a developing non-human embryo is suitable for creating germ line transmission of the construct. The non-human ES cells can be cultured and prepared for DNA insertion using methods known in the art, e.g., as described in Robertson, Teratocarcinomas and Embryonic Stem Cells. A Practical Approach, E. J. Robertson, ed. IRL Press, Washington, D.C., 1987; in Bradley et al., Current Topics in Devel. Biol., 20:357-371, 1986; and in Hogan et al., Manipulating the Mouse Embryo: A Laboratory Manual, Cold Spring Harbor Laboratory Press, Cold Spring Harbor, N.Y., 1986.

Expression constructs can be introduced into the non-human ES cells by methods known in the art, e.g., those described in Sambrook et al., Molecular Cloning. A Laboratory Manual, 2.sup.nd Ed., ed., Cold Spring Harbor laboratory Press: 1989 . Exemplary methods include, but are not limited to, electroporation, microinjection, and calcium phosphate treatment methods.

Transformed non-human ES cells are typically identified by screening for the presence of the construct. For example, ES cell genomic DNA can be examined directly. This can be accomplished, for example, by extracting the DNA from the non-human ES cells using standard methods and probing on a Southern blot with a probe or probes designed to specifically hybridize to the transgene sequence. Genomic DNA can also be amplified by PCR with use of primers specifically designed to amplify DNA fragments of a particular size and sequence of the construct or transgene such that, only those cells containing the construct or transgene will generate DNA fragments of the proper size. In another approach, a marker gene is incorporated in the construct, and the cells tested for the presence of the marker gene. For example, for an antibiotic resistance markers gene, the cells can be cultured in the presence of an otherwise lethal concentration of antibiotic. The presence of the antibiotic selects for those cells that contain the transgene construct. If the marker gene encodes an enzyme with detectable activity (e.g., beta.-galactosidase or luciferase), the enzyme substrate can be added to the cells under suitable conditions, and the enzymatic activity can be determined as an indicator of the presence of the transgene construct.

Transgenic non-human animals can be identified after birth by standard protocols. For example, DNA from tissue can be screened for the presence of the transgene construct, e.g., using Southern blots and/or PCR. Offspring that appear to be mosaics can be crossed to each other in order to generate homozygous non-human animals. If it is unclear whether the offspring will have germ line transmission, they can be crossed with a parental or other strain and the offspring screened for heterozygosity. The heterozygotes are identified by Southern blots and/or PCR amplification of the DNA. The heterozygotes can then be crossed with each other to generate homozygous non-human transgenic offspring. Homozygotes can be identified by Southern blotting of equivalent amounts of genomic DNA from offspring that are the product of this cross, as well as animals that are known heterozygotes and wild type animals. Probes to screen the Southern blots can be designed based on the sequence of the construct or transgene, or a marker gene, or both.

Other techniques for identifying and characterizing transgenic non-human animals are known in the art. For example, western blots can be used to assess the level of expression of a gene targeted for inhibition by probing with an antibody against the targeted protein. Alternatively, an antibody against a marker gene product can be used.

The invention also concerns cells containing a present transgene derived from non-human transgenic animals. Because certain genetic changes may occur in succeeding generations, e.g., due to mutation or recombination, such progeny cells may not be identical to the parent cell.

### VI. Construction and Testing of Pol III/Pol II Fusion Promoters

Fusion promoters can be constructed using a basal promoter from a Pol III transcribed gene along with one or more additional regulatory elements from at least one Pol II regulatory region. In addition, additional regulatory elements from the same or different Pol III gene may be incorporated in the fusion promoter. For simplicity of construction, it is advantageous to select and use regulatory elements that are 5' to the start site.

Several Pol III promoters have been described that could be used in the present fusion promoters, such promoters include U6, H1, tRNA promoters, adenovirus VA1, and the like. For promoters that have been studied to identify basal promoter elements, such basal promoters can be used in the present fusion promoters.

For tRNA promoters, the promoter sequences necessary and sufficient for RNA polymerase III transcription are encoded in the tRNA gene and thus transcribed into the tRNA and are still deductable from sequences located in the D and T.PSLC loop. In some cases, there are additional regulatory sequences upstream (5') of the main body of the gene. However, most mammalian genomes encode more than 100 tRNA genes, that are redundant and many of them lack any 5' regulatory sequences (e.g., Thomann et al. 1989 J Mol Biol 209:505-523). A database of tRNA gene compilations can be found at http://rna.wustl.edu/tRNAdb/ showing that the human genome encodes 648 tRNA genes, some of them known to be pseudogenes but a majority (496) encoding functional tRNAs (many redundantly) that are needed to encode the 20 amino acids.

Additional Pol III basal promoters can also be identified and used using conventional promoter analysis. Thus, identification of a gene as a RNA Pol III transcribed gene provides the material for identifying the corresponding basal promoter, as well as additional regulatory elements.

Similarly, a number of regulatory regions for Pol II-transcribed genes have been analyzed, and constituent regulatory elements identified. Additional Pol II regulatory regions and elements can be identified by conventional means and used in the present invention. In most cases, it is advantageous to include the element of set of elements that are demonstrated or found to be responsible for the specific regulation properties of the regulatory region.

Examples of specifically regulated Pol II-transcribed genes that can be used to provide cis-acting regulatory elements include any of the variety of such genes identified in the art. Many such genes have been described, including examples for which promoter and regulatory elements have been described.

### VII. Target Genes and Target Sites

In general, any gene can be down-regulated using RNAi in cells that contain functional RNAi machinery, and in particular such genes can be down-regulated using the present nucleic acid constructs. A large number of such inhibitions of gene expression have been described using either siRNAs or shRNAs. Targeting of such genes is also useful in connection with the present invention.

One such target gene is mutant Cu, Zn superoxide dismutase (SOD1). (See, e.g., U.S. Patent Appl. Publ. 2005013018 ). Mutations in Cu, Zn superoxide dismutase (SOD1) gene cause a subset of amyotrophic lateral sclerosis, a neurodegenerative disease that leads to motor neuron degeneration, paralysis and death (Brown and Robberecht, 2001; Siddique and Lalani, 2002). It has been well established that mutant SOD1 causes motor neuron degeneration by acquisition of a toxic property (Cleveland and Rothstein, 2001). However, neither the molecular basis of this toxic property nor mechanism that leads to motor neuron death is understood. Because of this incomplete understanding of the disease mechanism, rational design of therapy has not produced robust efficacious outcomes. On the other hand, because the toxicity that kills motor neurons originates from the mutated protein (Cleveland and Rothstein, 2001), decrease of the mutant protein should alleviate or even prevent the disease.

Suitable target sites for RNAi can be identified by any of a variety of methods, e.g., known to those of ordinary skill in the art. It has been found that most sites will provide at least some level of inhibition by RNAi, but some sites are found to provide substantially higher levels of inhbition. One way of identifying such "good" sites is by simple testing of potential target sites. In addition, a number of different algorithms have been designed to identify good target sites. Generally, several sites are identified using such algorithm, and then are tested for relative effectiveness.

Thus, exemplary methods for selecting suitable regions in a mRNA target are described in available publications (see, for example, Vickers et al., J. BioL Chem. 278:7108-7118, 2003; Elbashir et al., Nature 411:494-498, 2001; Elbashir et aL, Genes Dev. 15:188-200, 2001). Good target sequences are generally those sensitive to down regulation by low concentrations of siRNA. Guidelines for the design of siNA include those provided in Ambion's Technical Bulletin #506 (available from Ambion Inc., Austin, Tex.). The use of low concentrations of siRNA and avoidance of sequences that occur in alternative spliced gene products is useful for avoiding or limiting off-target, non-sequence specific inhibition. Assessing whether a gene has been downregulated, and the extent of downregulation, can be performed using, for example, real-time PCR, PCR, western blotting, flow cytometry or ELISA methods.

As an example, potential target sites in the mRNA are identified based on rational design principles, which include target accessibility and secondary structure prediction. Each of these may affect the reproducibility and degree of knockdown of expression of the mRNA target, and the concentration of siRNA required for therapeutic effect. In addition, the thermodynamic stability of the siRNA duplex (e.g., antisense siRNA binding energy, internal stability profiles, and differential stability of siRNA duplex ends) may be correlated with its ability to produce RNA interference. (Schwarz et al., Cell 115:199-208, 2003; Khvorova et al., Cell 115:209-216, 2003). Empirical rules, such as those provided by the Tuschl laboratory (Elbashir et al., Nature 411:494-498,2001; Elbashir et al., Genes Dev. 15:188-200,2001) are also used.

Software and internet interactive services for siRNA design are available at the Ambion and Invitrogen websites. Additional software system for design and prioritization of siRNA oligos have also been described (see, e.g., Levenkova et al., Bioinformatics 20:430-432, 2004). The Levenkova system is available on the internet and is downloadable freely for both academic and commercial purposes.

The selection of siRNA oligos can also involve uniqueness vs human sequences (i.e., a single good hit vs human Unigene, and a large difference in hybridization temperature (Tm) against the second best hit) and on GC content (i.e., sequences with % GC in the range of 40-60%).

A more detailed picture on the potential hybridization of the oligos, RNA target accessibility and secondary structure prediction can be carried out using available RNA structure prediction software, for example, Sfold software (Ding Y and Lawrence, C. E. (2004) Rational design of siRNAs with Sfold software. In: RNA Interference: from Basic Science to Drug Development. K. Appasani (Ed.), Cambridge University Press; Ding and Lawrence, Nucleic Acids Res. 29:1034-1046,2001; Nucleic Acids Res. 31:7280-7301, 2003). Sfold is available on the internet. RNA secondary structure determination is also described in Current Protocols in Nucleic Acid Chemistry, Beaucage et al., ed, 2000, at 11.2.1-11.2.10.

In addition, certain mutations (e.g., A or U inserted or substituted at the first, second, or third positions on the 5' end of the antisense strand of a siRNA or shRNA) insertions, have been described as providing enhanced RNAi efficiency and can be incorporated in the present constructs. Such mutations are described, for example, in U.S. Appl. Publ. 20050166272.

### VIII. Methods of Use

The present invention is suitable for a variety of different applications,, e.g., in biotechnology, gene analysis, drug identification and development identification and development of drugs, and in medical treatment methods. For example, there is currently performed a great deal of analysis of gene function in humans as well as in other animals and other organisms. Thus, the ability to conveniently provide transgenic non-human organisms or recombinant cells with modulation of specific genes enables the analysis of gene function, as well as the identification and evaluation of drug compound.

In particular, by determining the effect of down-regulating specific genes in transgenic non-human animals or cells, the biological function of those genes can be determined. Having an identified gene function allows drug targets to be validated, and for disease models to be established. Thus, specific cells may be transfected in vivo or ex vivo with recombinant vectors (e.g., viral vectors such as retrovirus vectors) encoding an RNAi agent that down-regulates the activity of a gene, for example, a gene whose activity is associated or correlated with a particular disease or condition.

In some applications it can be advantageous to determine the presence and/or level of a particular nucleic acid or polypeptides, such as the RNAi agents (e.g., siRNA or shRNA) and/or target mRNAs and/or the gene products encoded by such target mRNAs. A variety of applicable qualitative and quantitative detection methods and related techniques are known and can be used, including, for example, nucleic acid cloning and sequencing, oligonucleotide ligation, use of the polymerase chain reaction (PCR) and variations thereof, single nucleotide primer-guided extension assays, hybridization techiques using target-specific oligonucleotides, and sandwich hybridization methods.

Sequencing maybe carried out with commercially available automated sequencers utilizing labeled primers or terminators, or using sequencing gel-based methods. Sequence analysis is also carried out by methods based on ligation of oligonucleotide sequences which anneal immediately adjacent to each other on a target DNA or RNA molecule (Wu and Wallace, Genomics 4: 560-569 (1989); Landren et al., Proc. Natl. Acad. Sci. 87: 8923-8927 (1990); Barany, F., Proc. Natl. Acad. Sci. 88:189-193 (1991)). The Ligase Chain Reaction (LCR), which utilizes the thermostable Taq ligase for target amplification, is particularly useful such that the ligation reaction can be carried out at elevated reaction temperatures providing high stringency (Barany, F., PCR Methods and Applications 1: 5-16 (1991)).

Hybridization reactions may be carried out in a variety of formats, including filter-based, Southern blots, slot blots, "reverse" dot blots, solution hybridization, solid support based sandwich hybridization, bead-based, silicon chip-based and microtiter well-based hybridization formats. Specific oligonucleotide probes typically range in size between 10-1,000 bases, more commonly between 15 and 50 bases. In order to achieve a needed target discrimination using the oligonucleotide probes, hybridization reactions are generally run in the range of 20-60 degrees C, and more commonly in the range of 30-50 degrees C, with the temperature and/or salt concentrations and/or inclusion of other chaotropic agents such as formamide in the washes selected to provide optimal discrimination.

Detection of specific proteins or polypeptides is commonly performed using directly- or indirectly labeled specific antibodies, e.g., monoclonal or polyclonal antibodies, or fragments thereof. Examples of such labels include fluorescent moieties, colorometric moieties, light scattering moieties, and radioisotopes. Those of ordinary skill in the art are familiar with carrying out such detection.

The general detection methods mentioned above, as well as other methods, can be used in testing and/or using the present nucleic acid constructs.

### A. Screening, Assays, and Therapeutic Agent Testing

The present invention is applicable to use in screening assays, e.g., to identify and/or analyze potential pharmacological agents, e.g. identifying new pharmacological agents from a library of test compounds and/or characterizing mechanisms of action and/or side effects of compounds that have known pharmacological activities.

Thus, the present invention concerns materials and methods for carrying out a variety of biological assays and/or drug screening assays using cells or organisms that express agents, especially RNAi agents, from the present Pol III/Pol II fusion promoters. Generally such cells are eukaryotic cells (e.g., animal or plant cells) and/or such organisms are eukaryotic organisms (e.g., non-human transgenic animals).

Such assays and tests generally involve expressing a nucleic acid sequence (e.g., an RNAi agent-encoding sequence) in a cell or organism and determining at least one effect of that expression. The assay may be conducted to test or assay a single or small number of RNAi agents, or can be carried out in large scale assaying, e.g., for compounds in a compound library, such as assaying or testing at least 10, 100, 1000, 10⁴, 10⁵, 10⁶ compounds.

Assays or tests involving determination of the effect(s) of RNAi agent expression can advantageously also involve determining or comparing the effect(s) of the absence of the RNAi agent expression, the presence of a positive and/or negative control compound, and/or the presence of one or more test compounds. Typically, such assays or test involve determining pharmacological properties of the RNAi agent and/or other test compounds.

Test compounds can be obtained in many different ways, e.g., using any of the numerous approaches in compound library methods known in the art. For example, libraries can be commercially available compound libraries, libraries constructed from commercially available compounds, custom compound libraries, synthetic compound libraries, and natural product libraries, e.g., produced by bacteria, yeast, and/or fungi.

One broad category of libraries and library methods are combinatorial library methods including without limitation: biological libraries; spatially addressable parallel solid phase or solution phase libraries; synthetic library methods requiring deconvolution; the 'one-bead one-compound' library method; and synthetic library methods using affinity chromatography selection. (Lam, K. S. (1997) Anticancer Drug Des. 12:145). Such libraries can be peptide and/or peptide analog, oligonucleotide and/or oligonucleotide analog, and/or small molecule libraries.

Examples of methods for the synthesis of molecular libraries can be found in the art, for example in: DeWitt et al. (1993) Proc. Natl. Acad. Sci. U.S.A. 90:6909; Erb et al. (1994) Proc. Natl. Acad. Sci. USA 91:11422; Zuckermann et al. (1994). J. Med. Chem. 37:2678; Cho et al. (1993) Science 261:1303; Carrell et al. (1994) Angew. Chem. Int. Ed. Engl. 33:2059; Carell et al. (1994) Angew. Chem. Int. Ed. Engl. 33:2061; and in Gallop et al. (1994) J. Med. Chem. 37:1233.

Libraries of compounds may be presented, for example, in solution (e.g., Houghten (1992) Biotechniques 13:412-421), on beads (Lam (1991) Nature 354:82-84), chips (Fodor (1993) Nature 364:555-556), bacteria (Ladner U.S. Pat. No. 5,223,409), spores (Ladner USP'409), plasmids (Cull et al. (1992) Proc Natl Acad Sci USA 89:1865-1869) or on phage (Scott and Smith (1990) Science 249:386-390); (Devlin (1990) Science 249:404-406); (Cwirla et al. (1990) Proc. Natl. Acad. Sci. 87:6378-6382); (Felici (1991) J. Mol. Biol. 222:301-310); (Ladner supra.)).

Additional compounds or agents identified according to screening assays can be further tested and/or developed and/or used therapeutically or prophylactically either alone or in combination, for example, with an RNAi agent of the invention.

### B. Functional Genomics and/or Proteomics

Certain applications for the cells and organism of the invention include the analysis of gene expression profiles and/or proteomes. In may cases, such analysis involves knock-out or knock-down of a target gene, and determining a phenotypic change as in indicator of gene function or effect of inhibition. Alternatively, such analysis can be directed to a variant or mutant form of one or several target proteins, where the variant or mutant forms are reintroduced into the cell or organism by an exogenous target nucleic acid as described above. The combination of knockout of an endogeneous gene and rescue by using mutated, e.g., partially deleted exogenous target has certain advantages such as assisting in identifying functional domains of the targeted protein. Such analysis can be carried out for multiple cell types and/or tissues and/or organisms. These cells and/or organisms are generally selected from: (i) a control cell or control organism without target gene inhibition, (ii) a cell or organism with target gene inhibition and (iii) a cell or organism with target gene inhibition plus target gene complementation by an exogenous target nucleic acid.

Such RNA knockout complementation method may be used for its preparative purposes, e.g., for the affinity purification of proteins or protein complexes from eukaryotic cells, particularly mammalian cells and more particularly human cells. In this embodiment of the invention, the exogenous target nucleic acid preferably codes for a target protein which is fused to an affinity tag. This method is suitable for functional proteome analysis in mammalian cells, particularly human cells. Another utility of the present invention is a method of identifying gene function in an organism by using an RNA molecule to inhibit the activity of a target gene of previously unknown function. Instead of the time consuming and laborious isolation of mutants by traditional genetic screening, functional genomics would envision determining the function ofuncharacterized genes by employing the invention to reduce the amount and/or alter the timing of target gene activity. The invention can be used in determining potential targets for pharmaceutics, understanding normal and pathological events associated with development, determining signaling pathways responsible for postnatal development/aging, and the like.

Creation of cells/organisms containing the target gene allows the present invention to be used in high throughput screening (HTS). For example, solutions containing such cells containing RNAi agent capable of inhibiting the different expressed genes can be placed into individual wells positioned on a microtiter plate as an ordered array, and intact cells/organisms in each well can be assayed for any changes or modifications in phenotype, behavior, and/or development corresponding to inhibition of target gene activity. Such screening is amenable to cells as well as to small subjects that can be processed in large number, for example: arabidopsis, drosophila, fungi, nematodes, viruses, zebrafish, plants, and tissue culture cells derived from various organisms, such as mammals. A nematode or other organism that produces a colorimetric, fluorogenic, or luminescent signal in response to a regulated promoter (e.g., transfected with a reporter gene construct) can be assayed in an HTS format.

HTS can be used to identify and/or characterize new drug targets. The potential drug targets may also be validated using the present invention. For example, a particular disease phenotype may be induced by a gene mutation or a chemical. RNAi may be used to down-regulate genes and some of these down-regulations can lead to the reversal of the disease phenotype or other phenotypic change indicting a therapeutic or prophylactic effect. These genes are potential drug targets.

### C. Treatment Methods

The present invention provides RNAi agent-expressing constructs that are therapeutically useful (e.g., in certain prophylactic and/or therapeutic applications). For example, such agents can be used as prophylactic and/or therapeutic agents in the treatment of diseases or disorders associated with unwanted or aberrant expression of the corresponding target gene.

Thus, described herein are prophylactic methods of treating a subject at risk of (or susceptible to) a disease or disorder, for example, a disease or disorder associated with aberrant or unwanted target gene expression or activity or susceptible to an infection. Administration of a prophylactic agent can occur prior to the manifestation of symptoms characteristic of the disease or condition, such that a disease or disorder is prevented or delayed in its progression or reduced in severity.

Likewise, described herein are therapeutic methods of treating a subject having a disease or disorder, for example, a disease or disorder associated with aberrant or unwanted target gene expression or activity or expression from an infective agent.

Knowledge of RNAi agents and their targets thus allows specific inhibition of such target genes to treat any of a number of disorders (including cancer, inflammation, neuronal disorders, etc.) using the present constructs and methods.

For such prophylactic and therapeutic methods of treatment, the treatments maybe tailored or modified, based on knowledge obtained from the field of pharmacogenomics. "Pharmacogenomics", as used herein, refers to the application of genomics technologies such as gene sequencing, statistical genetics, and gene expression analysis to drugs in clinical development and on the market. More specifically, the term refers the study of how a patient's genes determine his or her response to a drug (e.g., a patient's "drug response phenotype", or "drug response genotype"). Thus, the invention also provides methods for tailoring an individual's prophylactic or therapeutic treatment with the present constructs and methods according to that individual's drug response genotype. Pharmacogenomics allows a clinician or physician to target prophylactic or therapeutic treatments to patients who will most benefit from the treatment and to avoid treatment of patients who will experience toxic drug-related side effects.

### IX. Pharmaceutical Compositions

For preparation of pharmaceutical compositions containing the present RNAi agents for prophylactic and/or therapeutic treatments, the agents are routinely incorporated into pharmaceutical compositions suitable for administration. Such compositions include the nucleic acid molecule and commonly include a pharmaceutically acceptable carrier, and may include additional components. The use of such carriers for pharmaceutically active substances is well known in the art. Any conventional media or agent incompatible with the active compound can be used in the present pharmaceutical compositions. The additional components can, for example, include additional or supplementary active compounds.

A present pharmaceutical composition is formulated to be compatible with its intended route of administration, for example, parenteral, e.g., intravenous, intradermal, subcutaneous, intraperitoneal, intramuscular, oral (e.g., inhalation), transdermal (topical), and transmucosal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The composition can be aliquoted or packaged in ampules, disposable syringes, single or multiple dose vials made of glass or plastic, bottles, and the like. Preferably the composition is sterile at a medically acceptable level in view of the intended route of administration. In some cases, the pharmaceutical composition is approved by a governmental drug regulatory agency (e.g., the U.S. FDA) for administration to a particular class of subject, such as human subjects.

Pharmaceutical compositions adapted for injection include, for example, sterile aqueous solutions (where water soluble) or dispersions and sterile powders for the extemporaneous preparation of sterile injectable solutions or dispersion. For intravenous administration, suitable carriers include, for example, physiological saline, bacteriostatic water, Cremophor EL™. (BASF, Parsippany, N.J.) and phosphate buffered saline (PBS). In all cases, the composition should be sterile and should be fluid or convertible to a fluid at least sufficient for easy syringability. The composition and/or nucleic acid constructs should be stable under the conditions of manufacture and storage and should be preserved against the contaminating action of microorganisms such as bacteria and fungi.

The carrier can be a solvent or dispersion medium containing, for example, water, ethanol, polyol (for example, glycerol, propylene glycol, and liquid polyetheylene glycol, and the like), and suitable mixtures thereof. Fluidity can be maintained, for example, by the use of a coating such as lecithin, by the maintenance of the required particle size in the case of dispersions and by the use of surfactants.

Preservatives against microorganisms can include various antibacterial and antifungal agents, for example, parabens, chlorobutanol, phenol, ascorbic acid, thimerosal, and the like.

In many cases, it will be desirable for the composition to be isotonic to blood. This can be accomplished using various isotonic agents, for example, sugars, polyalcohols such as manitol, sorbitol, sodium chloride in the composition.

Delayed or extended absorption of the injectable compositions can be desirable and can be achieved by including in the composition an agent which delays absorption, for example, aluminum monostearate and gelatin, or by coating micro- or nano-particles of active agent in the composition with materials that delayed or extended release of components.

Sterile injectable solutions can be prepared, for example, by solubilizing or suspending the active compound in the required amount in an appropriate solvent with one or a combination of additional ingredients. Typically creation of such solution or suspension is followed by sterile filtration. Generally, dispersions are prepared by incorporating the active compound into a sterile vehicle which contains a basic dispersion medium and the other desired ingredients. In the case of sterile powders for the preparation of sterile injectable solutions, the preparation is dried, e.g., by vacuum drying and/or freeze-drying.

Compositions for oral administration typically include an inert or edible diluent or edible carrier. Such compositions can be formulated in various ways, e.g., in liquid, capsule, or tablet form. Pharmaceutically compatible binding agents, and/or adjuvant materials can be included as part of the composition. The tablets, pills, capsules, troches and the like can contain any one or more of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For inhalation administration, the compounds are delivered in the form of a wet or dry aerosol spray, e.g., from a pressured container or dispenser which contains a suitable propellant, e.g., a gas such as carbon dioxide, or a nebulizer.

Systemic administration can also be by transmucosal or transdermal routes. For transmucosal or transdermal administration, penetrants appropriate to the barrier to be permeated are typically used in the formulation. A number of such penetrants are generally known in the art, and include, for example, for transmucosal administration, detergents, bile salts, and fusidic acid derivatives.

Transmucosal administration can be accomplished through the use of nasal sprays or suppositories (e.g., using conventional suppository bases such as cocoa butter and other glycerides). For transdermal administration, the active compounds are formulated into ointments, salves, gels, or creams as generally known in the art.

Such compositions can also be formulated with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. The materials can also be obtained commercially, e.g., from Alza Corporation and Nova Pharmaceuticals, Inc. Liposomal suspensions (including liposomes targeted to particular cells (e.g., targeted to infected cells) with monoclonal antibodies) can also be used to prepare pharmaceutical compositions. These can be prepared according to methods known to those skilled in the art, for example, as described in U.S. Pat. No. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of active compounds and pharmaceutical compositions can be determined by standard pharmaceutical procedures in cell cultures or experimental animals. For example, such procedures are routinely applied for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Compounds that exhibit large therapeutic indices are generally preferred.

The data obtained from the cell culture assays and animal studies can be used in formulating a range of dosage for use in humans or other intended subject. The dosage of such compounds is usually selected to produce a range of circulating concentrations that include the ED50 with little or no toxicity. The dosage may vary within this range depending upon the dosage form employed and the route of administration utilized. For any compound used in the method of the invention, the therapeutically effective dose can be estimated initially from cell culture assays. Thus, for example, a dose may be initially established in animal models to achieve a circulating plasma concentration range that includes the EC50 (i.e., the concentration of the test compound which achieves a half-maximal response) as determined in cell culture. Such information can be used to more accurately determine useful doses in humans. Levels in plasma may be measured, for example, by high performance liquid chromatography, or by other suitable analysis method adapted for the compound of interest.

### X. Exemplary Delivery Methods and Compositions

A number of delivery methods applicable to nucleic acid molecules, and particularly to transcribable nucleic acid molecules, have been described, and additional ones are being developed. All such methods and the associated compositions are applicable to the present invention. Such delivery typically utilizes naked linear nucleic acid, viral vectors, or plasmid vectors.

### EXAMPLES

The ability of Pol ID/Pol II fusion promoters to specifically regulate expression of functionally linked coding sequences, particularly RNAi agents, was demonstrated using cell-type specific expression based on the cell-type specific regulatory elements from the Pol II regulatory region of GFAP. The sequences of various constructs are provided as SEQ ID NOs:1-7. The following examples provide an illustrative description of the creation and expression of exemplary nucleic acid constructs, but are not intended to and do not limit the invention.

### EXAMPLE 1: CONSTRUCTION OF POL III/POL II FUSION PROMOTERS, ASSOCIATED CONSTRUCTS, AND VECTORS

To test whether tissue specific RNAi promoters could be created using a chimeric promoter, promoters that are the fusion of GFAP and U6 promoters were created. Promoter studies have revealed that the GFAP promoter is composed of several elements that are involved in tissue specific expression including an A, B and a D region. These cis-acting elements are instrumental in the transcription program of the human GFAP promoter. Two promoters were created: one that included the A and B elements of GFAP linked to the core promoter of U6 termed GFAP-EcoNIp and one that included the A, B and D elements of the GFAP promoter linked to the core promoter of U6 termed GFAP-SmaIp (see Fig. 1).

In each of the two promoters, the GFAP elements are placed upstream of the core promoter of U6. The core promoter of U6 has been shown to include the TATA box region and the PSE. Thus, both chimeric promoters contain elements of the RNA polymerase type II promoter and elements (specifically including the core promoter region) of an RNA polymerase type III promoter. These are named according to the restriction site used in their creation, i.e. pGliaSmaI and pGliaEcoNI. For initial testing purposes, RNAi directed against eGFP was included. Two additional plasmids were created that included the RNAi against eGFP termed pGliaSmaI-eGFP and pGliaEcoNI-eGFP (see Fig. 1). Finally, a dicistronic plasmid was created with expression cassettes for both eGFP and HcRed1. The design of the study was to cotransfect the dicistronic plasmid (i.e. peGFP/HcRed1) with the chimeric promoter containing plasmids and assay the expression of eGFP for knockdown and HcRed1 as a control.

Several additional vectors were created (maps not shown). Four more plasmids were created where the chimeric promoters (plus/minus RNAi against eGFP) was cloned into peGFP/HcRed1. These vectors were intended to allow testing of the idea without the use of cotransfection. Alternately, four additional vectors were created where the LacZ expression cassette was cloned into the plasmids containing the chimeric vectors. The purpose of these vectors was to allow for monitoring of the transfection efficiency of the chimeric promoter containing vectors in co-transfection experiments. Using this system, it is possible to monitor the transfection of both plasmids, i.e. HcRed1 expression to monitor the delivery of eGFP and LacZ to monitor the delivery of the chimeric promoter.

The vectors were constructed generally as follows. A dicistronic vector with both eGFP and HcRed1 expression cassettes (termed peGFP-HcRed1) was created in several steps. First, pHcRed1-RNAi was created by ligating the HcRed1-containing fragment of pHcRed1-N1 (Clontech) doubly digested with AgeI (blunted) and NotI into the backbone fragment of pHygeEGFP (Clontech) doubly digested with NheI (blunt) and NotI. Second, peGFP-RNAi was created by ligating the eGFP containing fragment of peGFP-1 (Clontech) doubly digested with SmaI and NotI into the backbone fragment of pHygeGFP (Clontech) doubly digested with NheI (blunted) and NotI. peGFP-HcRed1 was created by ligating the CMVie-eGFP-polyA cassette of peGFP-RNAi doubly digested with BglII and BamHI into pHcRed1-RNAi digested with BamHI and calf alkaline phosphatase.

Two RNAi chimeric promoters targeting glial cells were created using the U6 promoter and the glial fibrillary acidic protein (GFAP) promoter (pGfa2; Brenner et al). A ∼150 bp fragment of pU6-eGFP shRNA (Shi et al) doubly digested with DraI and BamHI was ligated into the backbone fragment of pGfa2 doubly digested with SmaI and BamHI to create pGFAP-SmaI-eGFP. Similarly, a control vector (pGFAP-SmaI-Control) was created by ligating an ∼150 bp fragment of pU6-control (Shi et al) into the backbone fragment of pGfa2 doubly digested with SmaI and BamHI. To make both pGFAP-EcoNI-eGFP and pGFAP-EcoNI-Control, the ∼150 bp fragment of pU6-eGFP and pU6-control were ligated (respectively) into the backbone fragment of pGfa2 doubly digested with EcoNI (blunted) and BamHI.

Four additional plasmids were created by ligating the BgIII/BamHI fragment of pGfaSmaI-control, pGfaSmaI-eGFP, pGfaEcoNI-control, or pGfaEcoNI-eGFP into peGFP/HcRed1 doubly digested with BamHI and alkaline phosphatase. Directionality was determined by restriction analysis and clones where the promoter direction of the chimeric promoter and the eGFP expression cassette were apposed were selected for testing.

A final set of four vectors were created by first cloning the LacZ containing BamHI fragment of pGfa2 (Brenner et al) into the BamHI/BclI backbone fragment of pIRES-eYFP (Clontech) to create pCMV-LacZ. Finally, the chimeric promoter containing fragments of pGfaSmaI-control, pGfaSmaI-eGFP, pGfaEcoNI-control, or pGfaEcoNI-eGFP doubly digested with BglII/BamHI were individually ligated into pCMV-LacZ doubly digested with BglII and alkaline phosphatase. These vectors, named pGfaSmaI-control-LacZ, pGfaSmaI-eGFP-LacZ, pGfaEcoNI-control-LacZ, or pGfaEcoNI-eGFP-LacZ, are useful in monitoring transfection efficiency of the chimeric promoters.

### EXAMPLE 2: TRANSFECTION OF CELLS WITH VECTORS CONTAINING POL III/POL II FUSION PROMOTERS LINKED WITH SHRNA CODING SEQUENCE

C6 glioma cells, Hela S3 cells, and HepG2 cells were cultured according to standard protocols using DMEM supplemented with 10% FBS. The day before transfection, cells were plated onto 6-well plates to achieve 60-70% density the following day for transfection.

Cells were transfected with Lipofectamine 2000 (Invitrogen) transfection reagent according to manufacturer's protocols. Briefly, 300 ng of peGFP-HcRed1 plus 3-6 micrograms of the shRNA expression (or control) plasmids were diluted in 100 microliters of OptiMem (Invitrogen). Separately, 7 microliters of Lipofectamine 2000 was diluted into 100 microliters of OptiMem. These two dilutions were combined and allowed to incubate for 20 minutes. During this time, the DMEM culture media was rinsed out of each well and replaced by 1 milliliter of OptiMem. After the twenty minute incubation for liplex formation, the lipoplexes were added to each well. After eight hours of incubation, another milliliter of OptiMem was added to each well to bring the total to 2.2 milliliters. After 24 hours, the culture media was exchanged for DMEM with 10% FBS and further experiments were conducted (i.e. either fluorescent microscopy or Western Blot analysis) at 24-48 hours after transfection.

### EXAMPLE 3: EXPRESSION AND ANALYSIS OF SHRNA

Preliminary analysis demonstrated that the exemplary constructs provided cell-specific expression of the linked coding sequences encoding shRNAs. Cells were grown generally as described above.

### Fluorescent Microscopy

To test the plasmids, C6 glioma cells and Hela S3 cells plated in 6-well plates were transfected with different combinations of the created plasmids: The media of cells grown in the six-well plates was exchanged with phosphate buffered saline containing calcium and magnesium and the plates were loaded onto an inverted microscope (Olympus IX70). Images were captured with ImagePro imaging software and hardware (Media Cybernetics), followed by image manipulation with Adobe Photoshop 6.0 (Adobe Systems, San Jose, CA). Representative images from several cell fields were captured.

Cotransfection of both peGFP/HcRed1 and the chimeric promoters (control and RNAi to eGFP) revealed that the chimeric promoters containing the D element of the GFAP promoter efficiently silenced the expression of eGFP in C6 glial cells. Further, there was no detectable silencing in Hela S3 cells. In contrast, there was no detectable silencing by the chimeric promoter that did not contain the D element in either cell line. In both cases, the visualization of HcRed1 was suboptimal so that the equal delivery of the peGFP/HcRed1 to both the control and RNAi wells could not be demonstrated fully. On the other hand, the experiment worked multiple times and peGFP/HcRed1 was diluted into a mastermix that was aliquoted into separate tubes before the addition of the chimeric promoter plasmids to ensure equal delivery.

The plasmids that contained the chimeric promoter and both eGFP and HcRed1 expression cassettes were also tested (data not shown). In these experiments, C6 glioma cells and Hela S3 cells were transfected with the single plasmid and eGFP and HcRed1 expression was visualized. However, no detectable difference eGFP fluorescence could be noted between any of the wells. Given the requirement of a copy excess of 20:1 of RNAi promoter containing plasmids to gene containing plasmid reported, it is not surprising that this did not reveal any difference. Future experiments to exchange the promoter of the eGFP expression cassette from the very active CMVie promoter to a less active promoter were considered and will be performed in the near future.

### Western Blot Analysis

In order to confirm and further test the expression results determined by fluorescent microscopy, protein levels were qualitatively tested using Westerns. These experiment were carried out in the same manner as for the fluorescent microscopy analysis by cotransfecting the chimeric promoters with peGFP/HcRed1. In most cases, fluorescent microscopy was also used to characterize the experiment, but then the Western analysis was performed on each well to obtain a more sensitive measure of the effectiveness of each chimeric promoter at silencing eGFP.

Cells were cultured generally as described above. The media was removed from each well of the 6-well plate and lysis buffer was added to each well and the cells were lysed on ice. Cells were scraped from each well and Western blotting was used to examine the expression of eGFP.

As with the fluorescent microscopy, it was found that the D element was required for efficient promoter activity in C6 glioma cells. The higher sensitivity allowed for the detection of some activity in the Hela S3 cells which was to be expected. Cotransfection of the peGFP/HcRed1 promoter with pGliaSmaI-eGFP displayed silencing of eGFP protein expression compared to HcRed1 in C6 glioma cells. In contrast, pGliaEcoNI-eGFP was not nearly as efficient at silencing eGFP protein expression.

All patents and other references cited in the specification are indicative of the level of skill of those skilled in the art to which the invention pertains

One skilled in the art would readily appreciate that the present invention is well adapted to obtain the ends and advantages mentioned, as well as those inherent therein. The methods, variances, and compositions described herein as presently representative of preferred embodiment are exemplary and are not intended as limitations on the scope of the invention. Changes therein and other uses will occur to those skilled in the art .

It will be readily apparent to one skilled in the art that varying substitutions and modification may be made to the invention disclosed herein . For example, variations can be made to the regulatory elements included in the constructs and in methods for delivering such constructs to cells and organisms.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible . Thus, it should be understood that although the present invention has been specifically disclosed by preferred embodiments and optional features, modification and variation of the concepts herein disclosed may be resorted to by those skilled in the art .

In addition, where features or aspects of the invention are described in terms of Markush groups or other grouping of alternatives, those skilled in the art will recognize that the invention is also thereby described in terms of any individual member or subgroup of members of the Markush group or other group.

Also, unless indicated to the contrary, where various numberical values are provided for embodiments, additional embodiments are described by taking any 2 different values as the endpoints of a range. Such ranges are also within the scope of the described invention.

Thus, additional embodiments are within the scope of the invention and within the following claims.

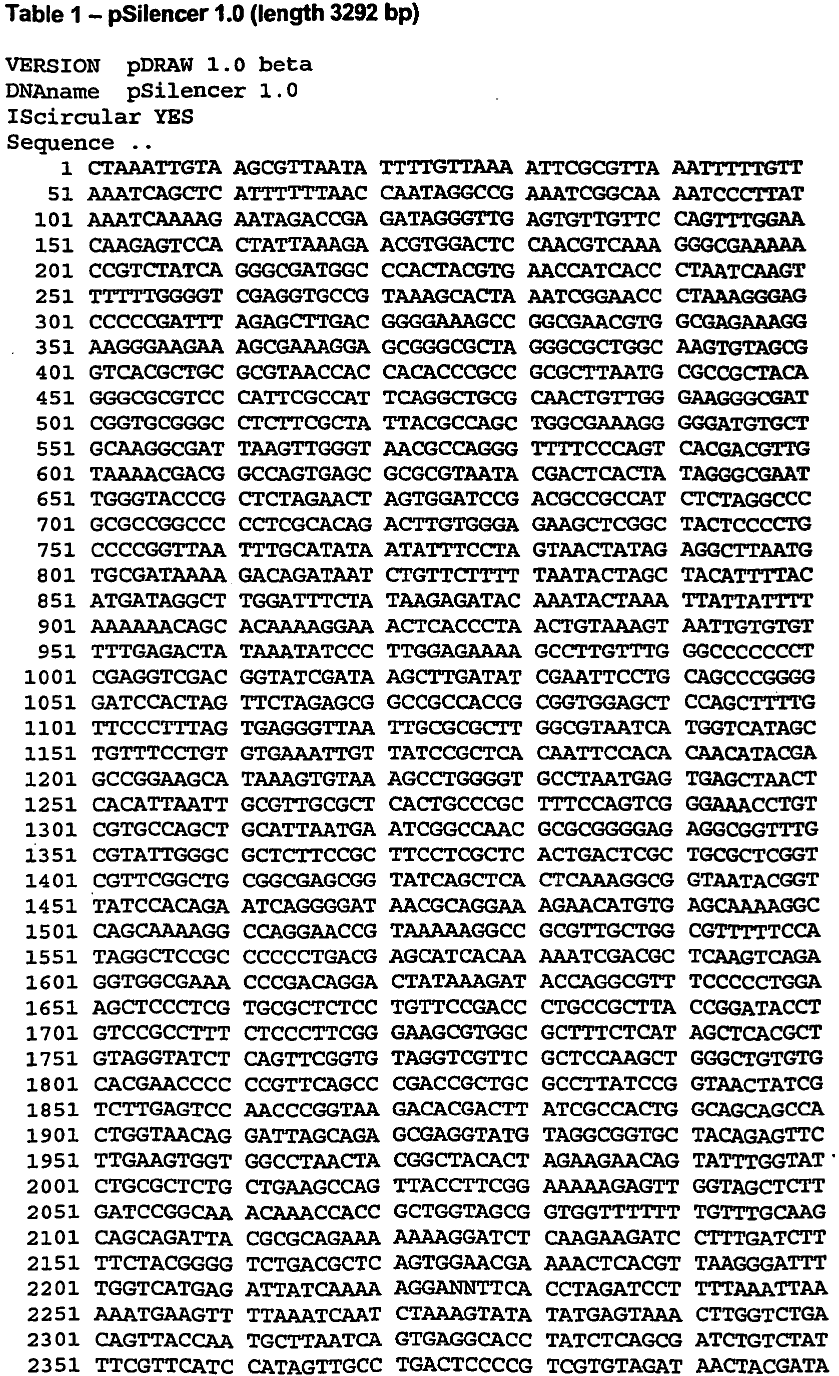

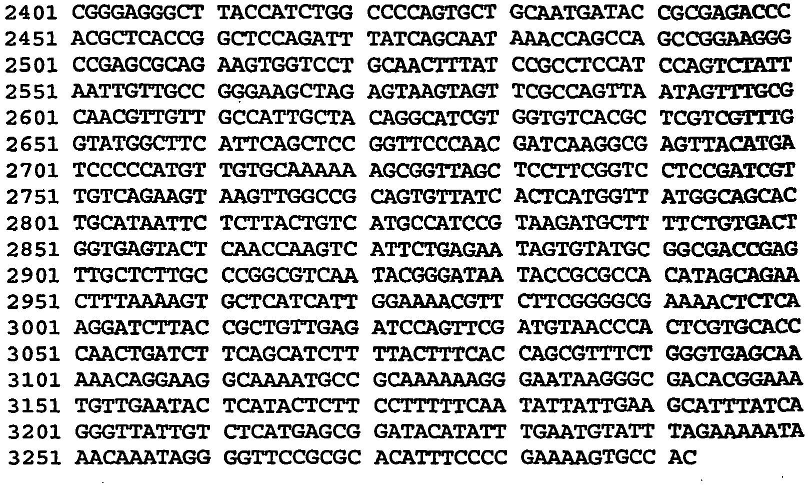

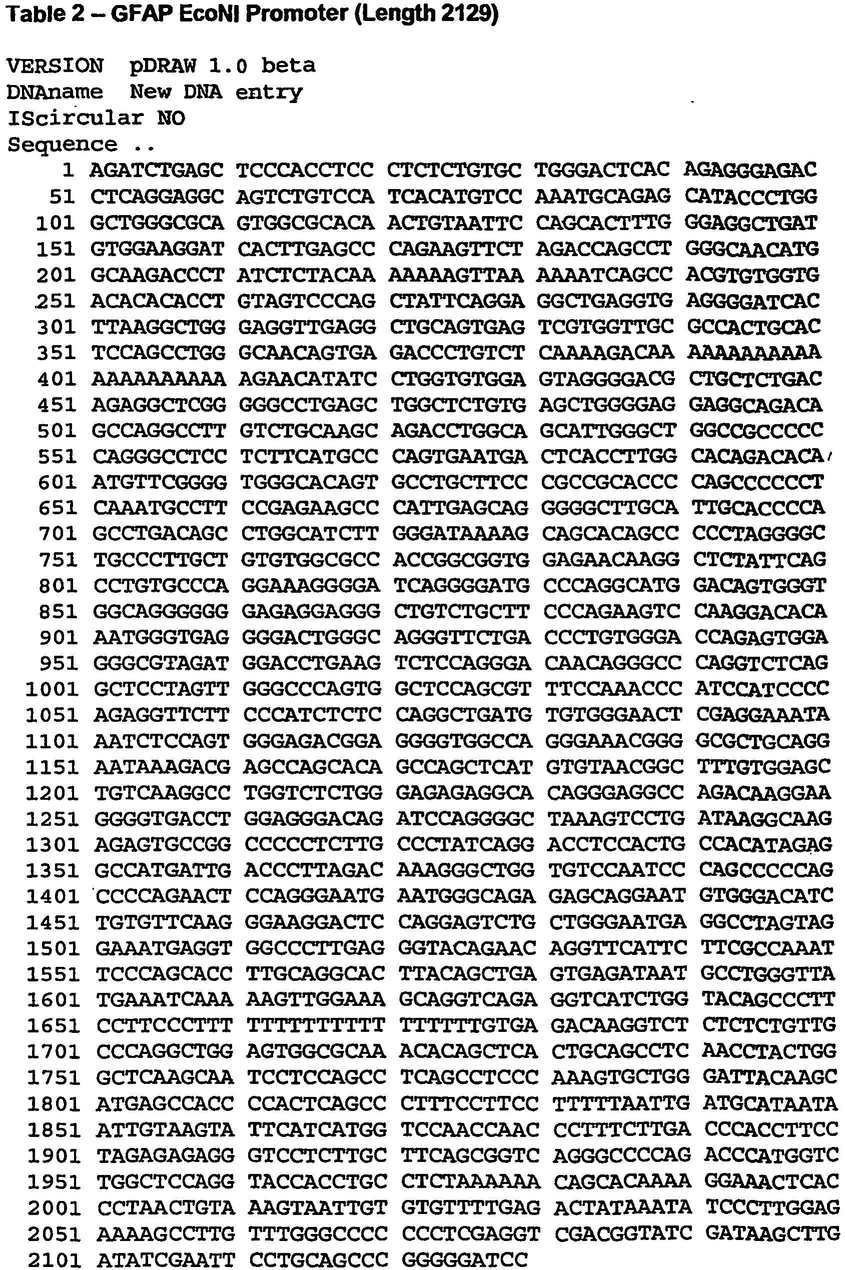

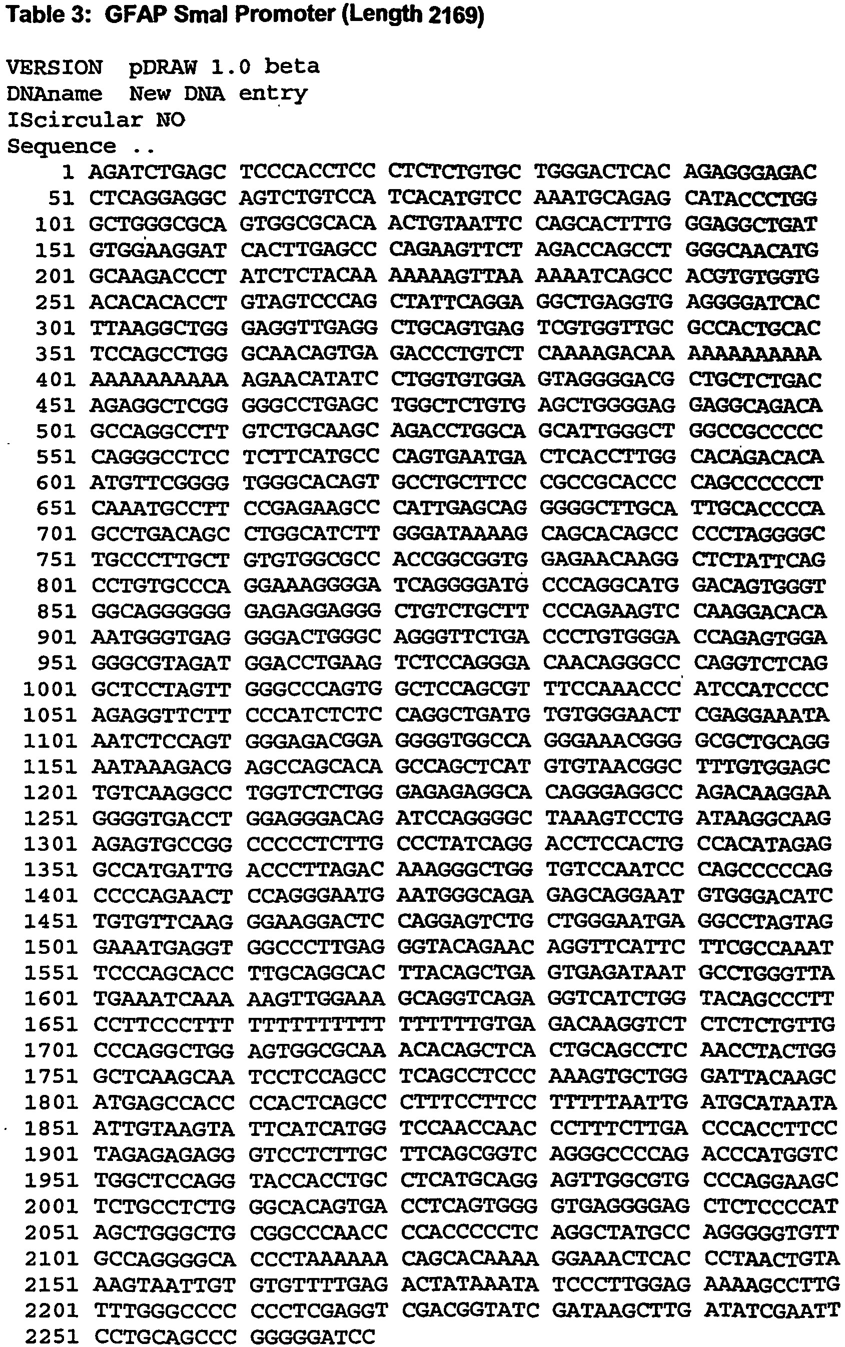

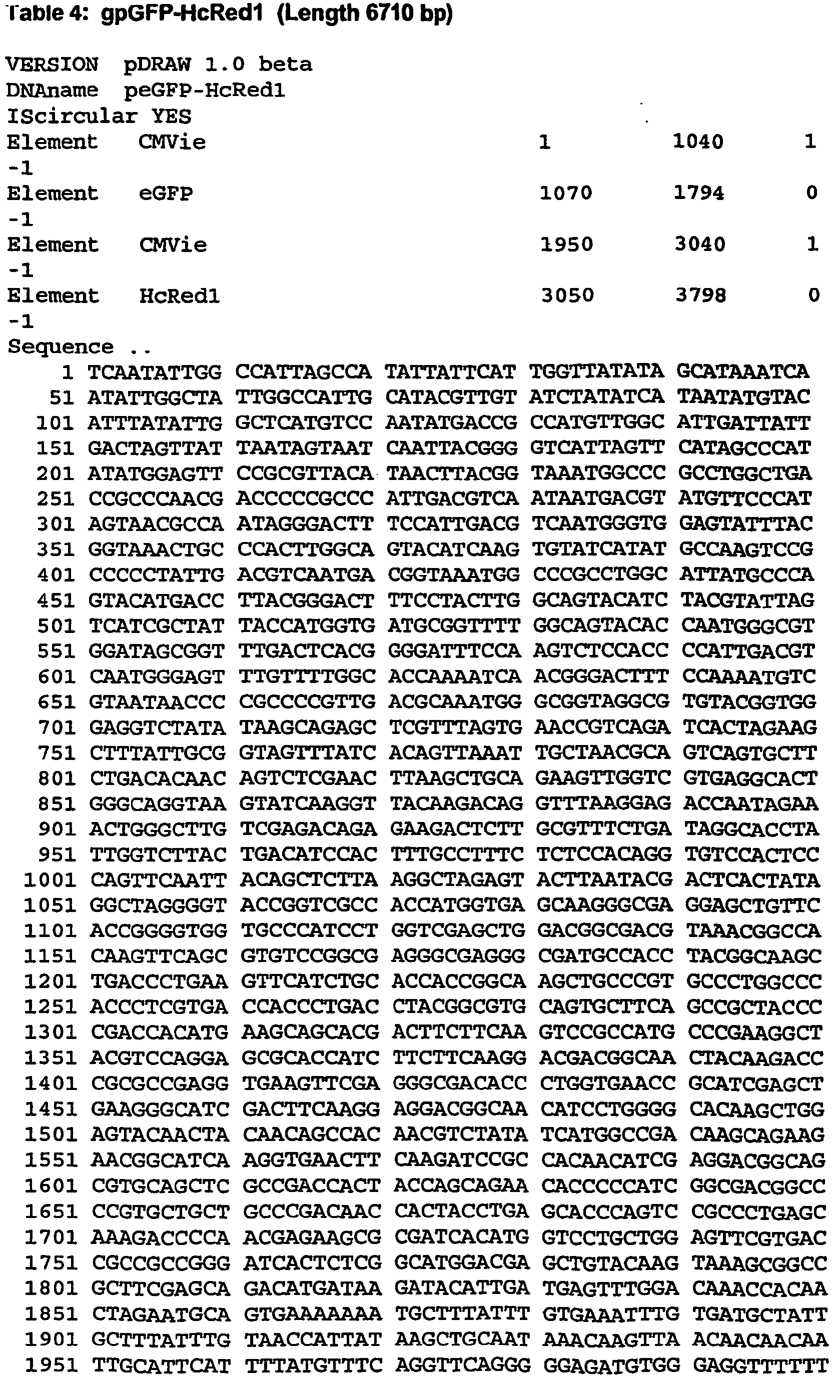

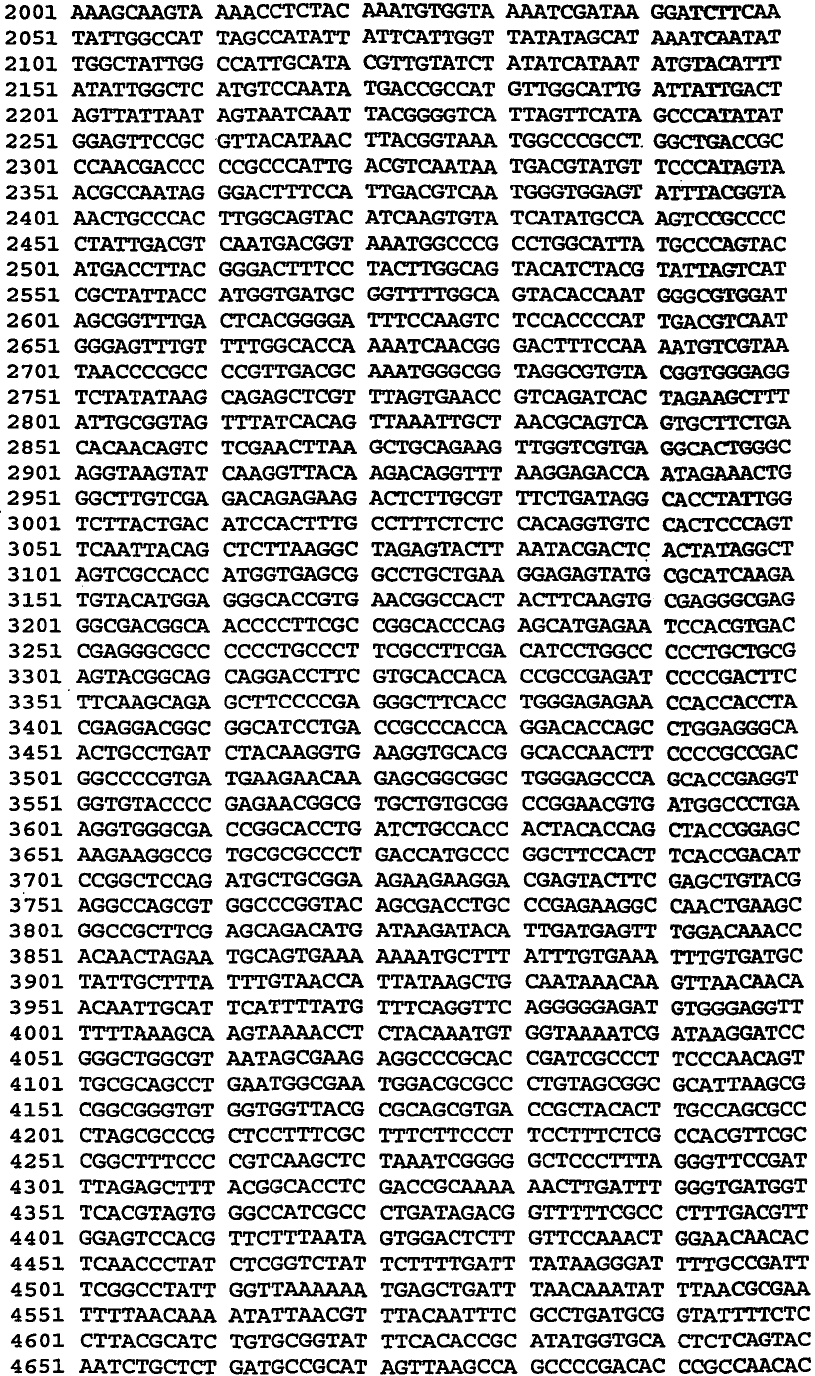

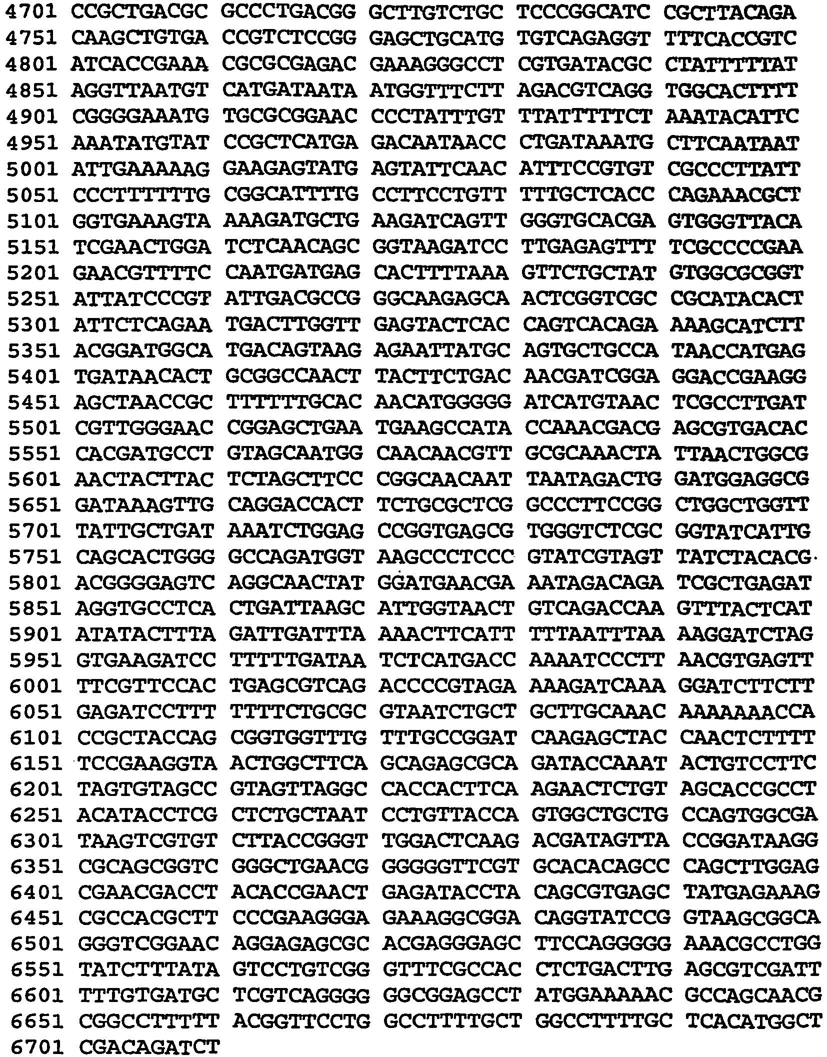

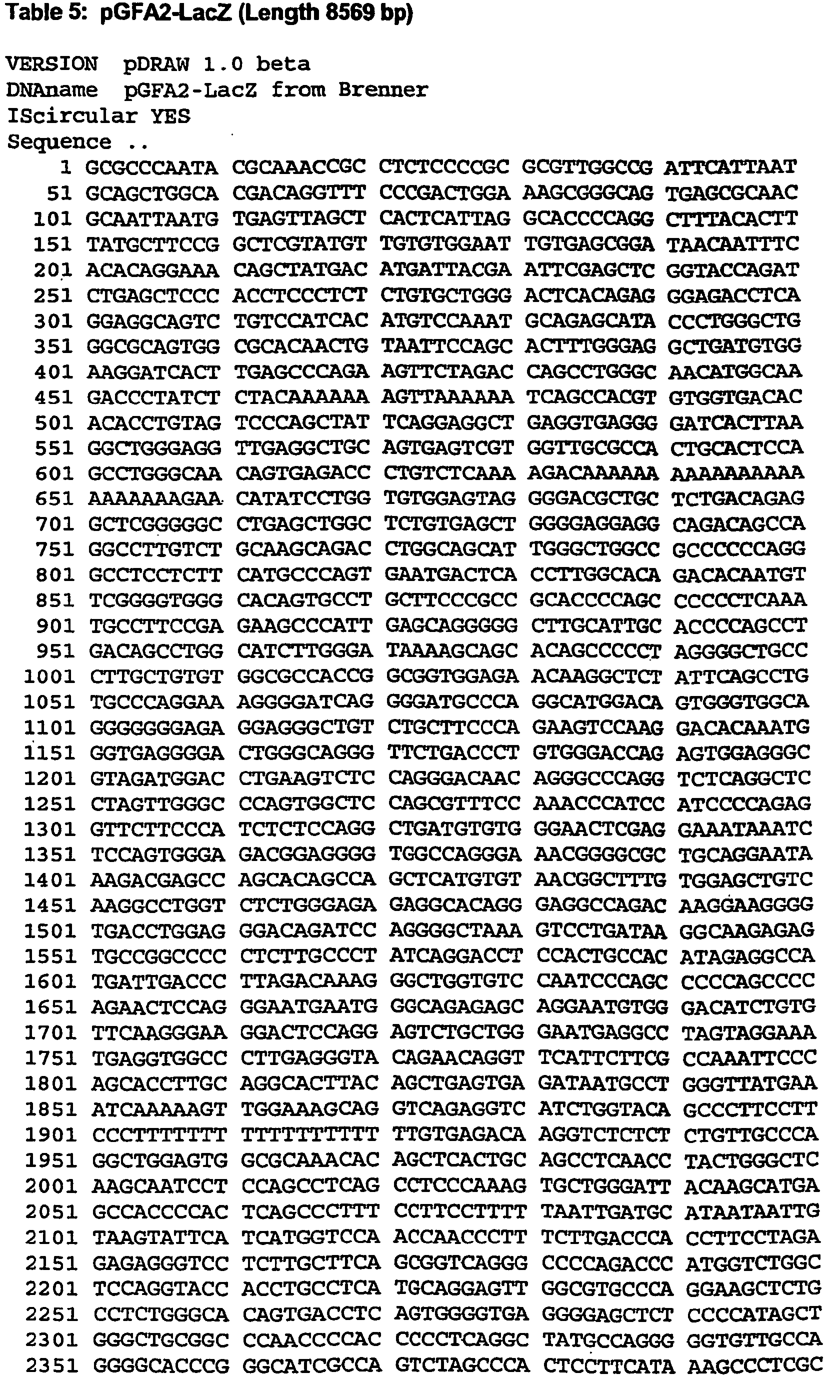

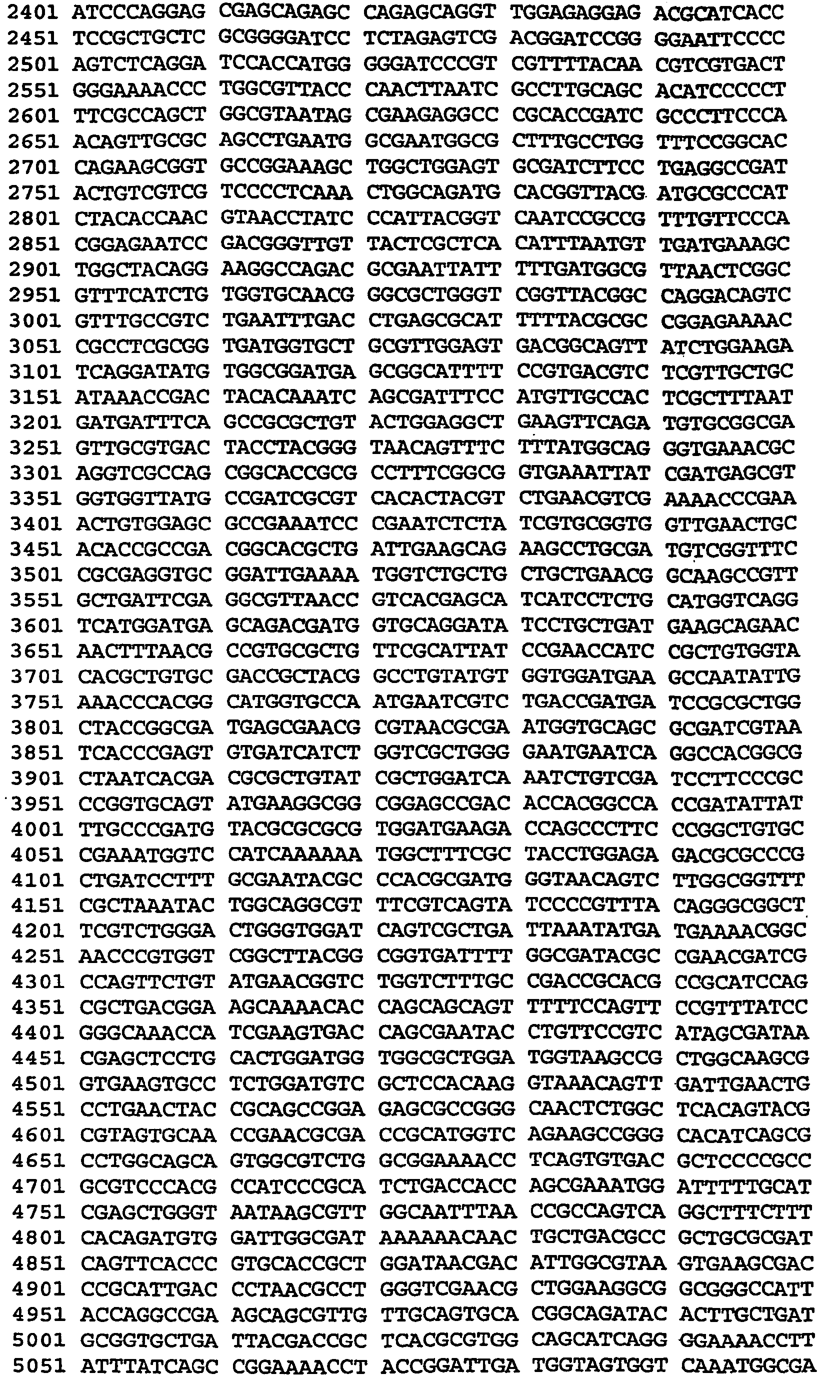

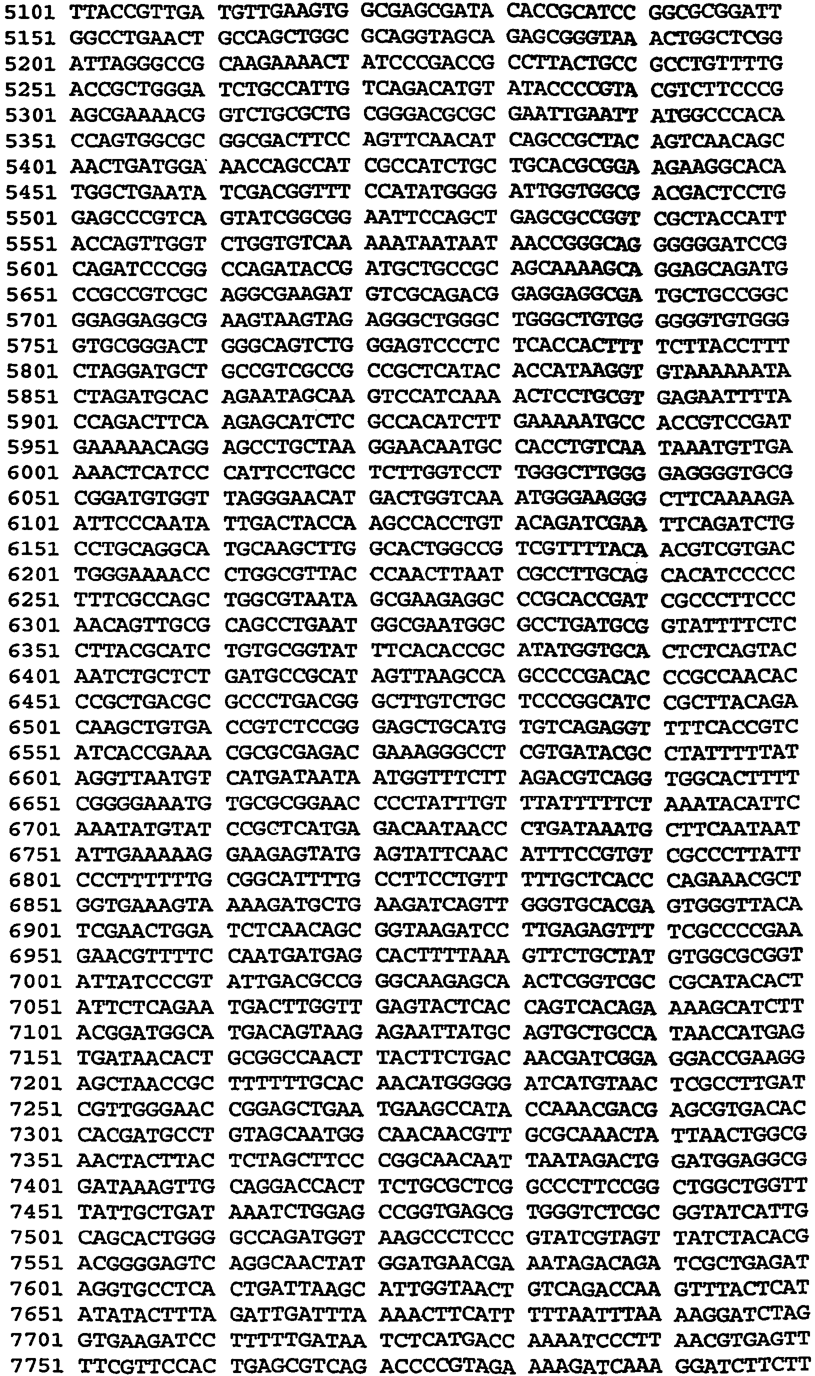

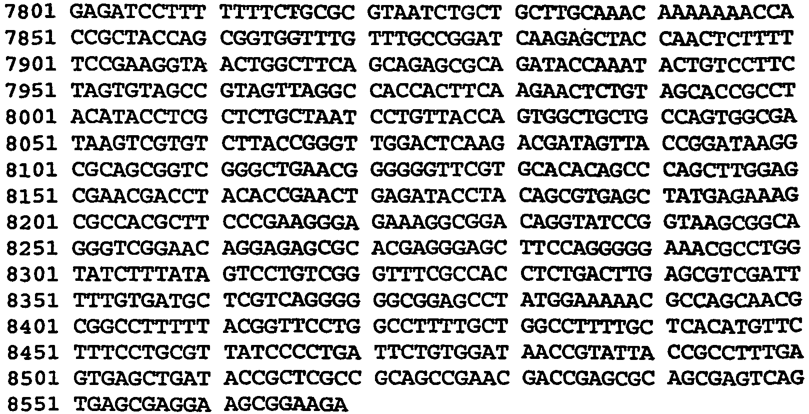

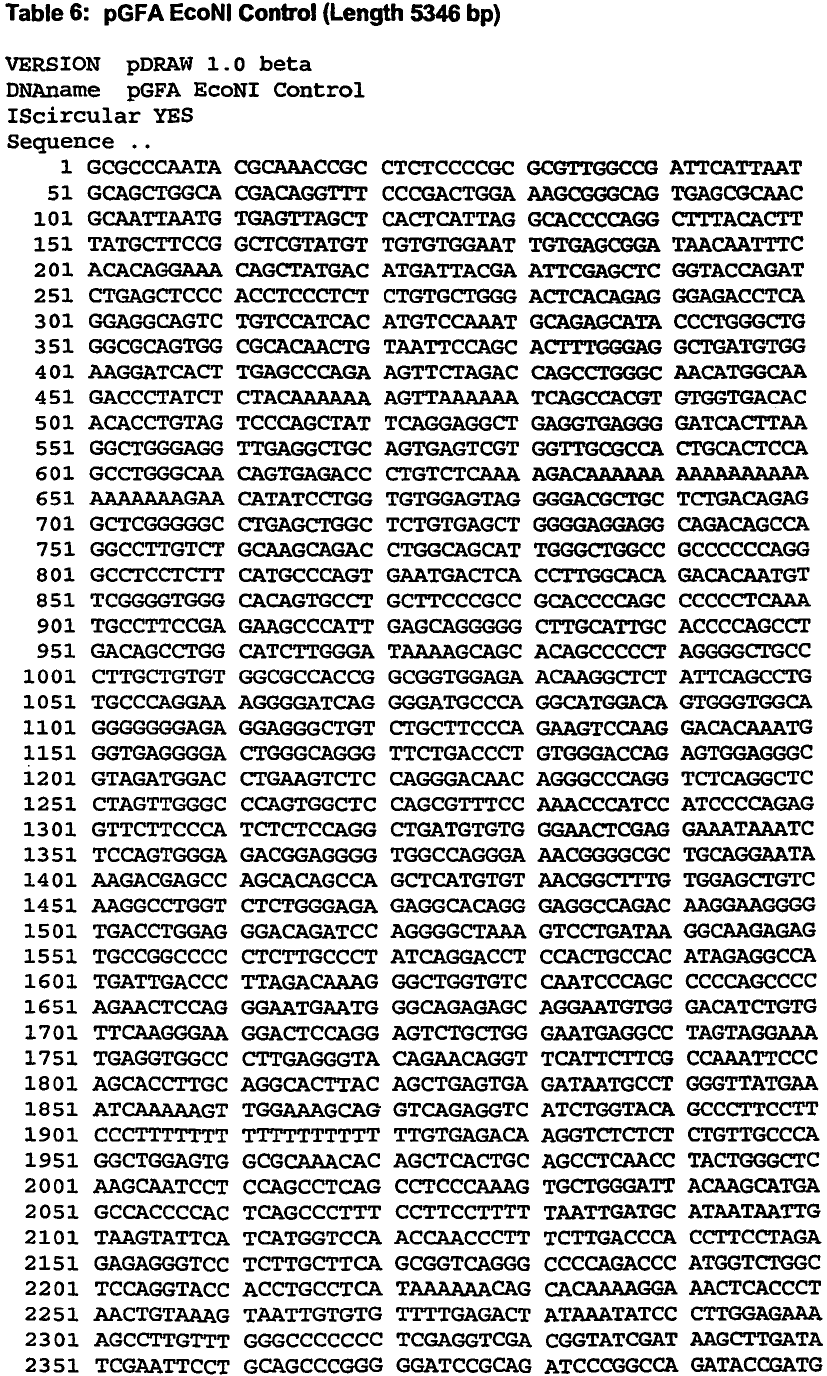

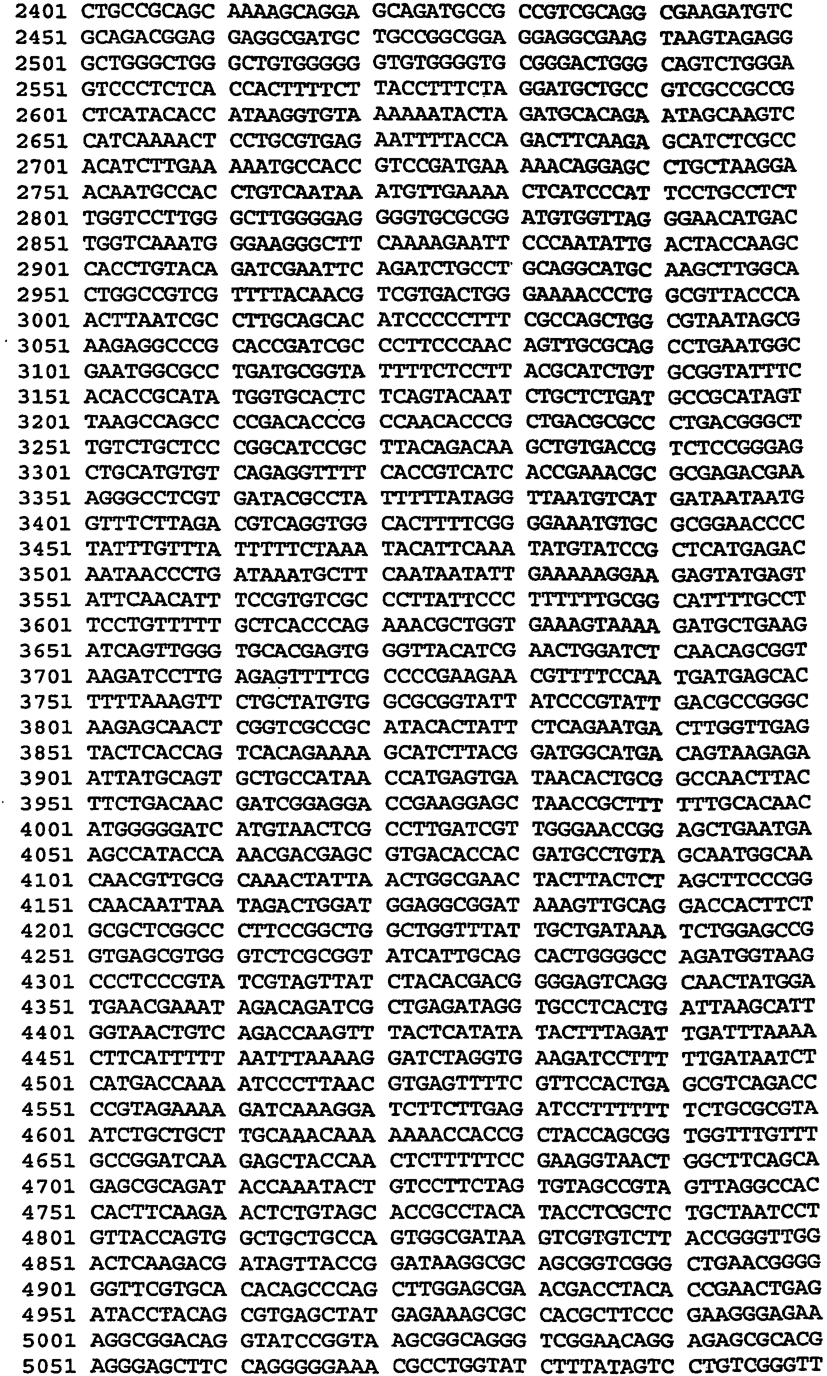

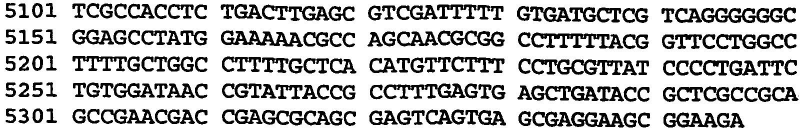

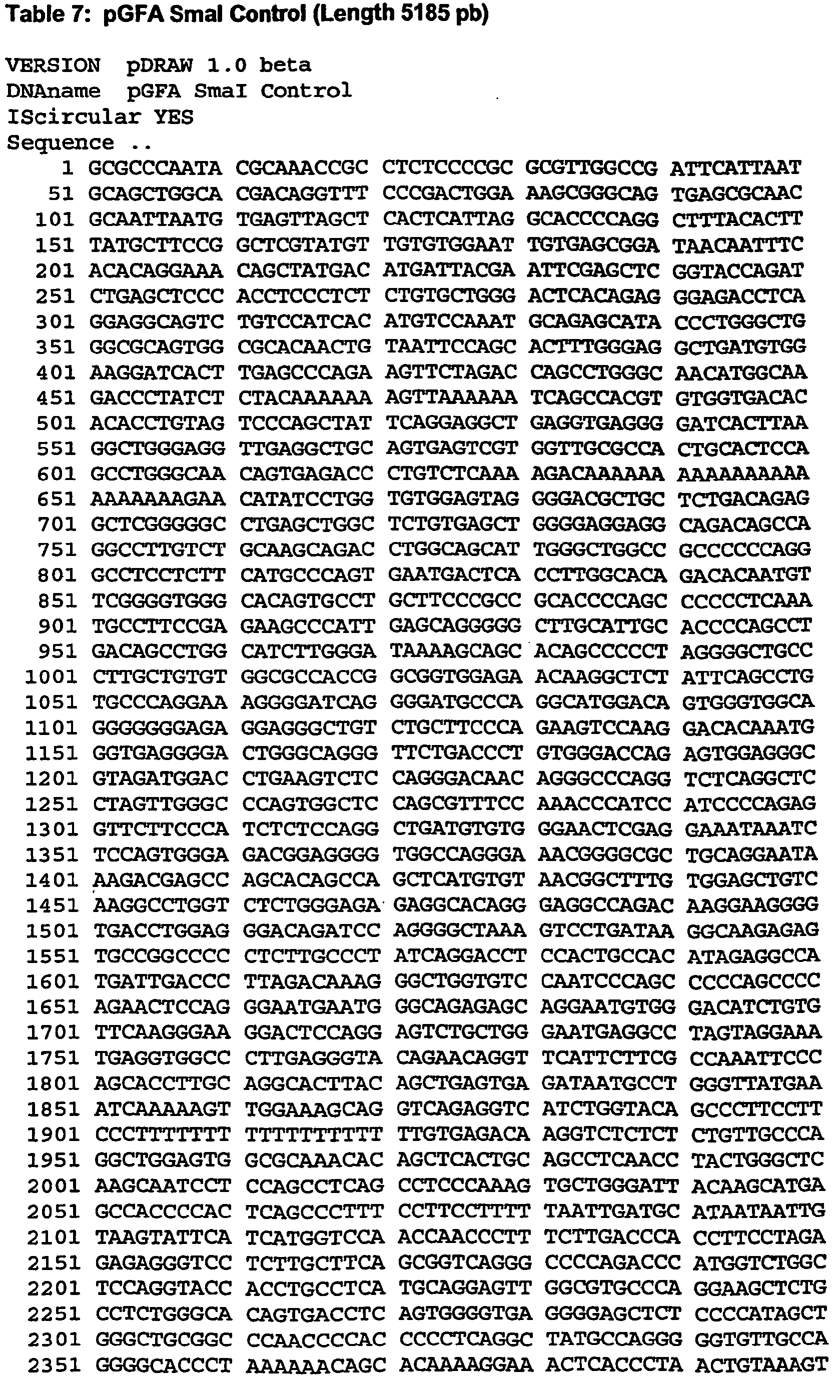

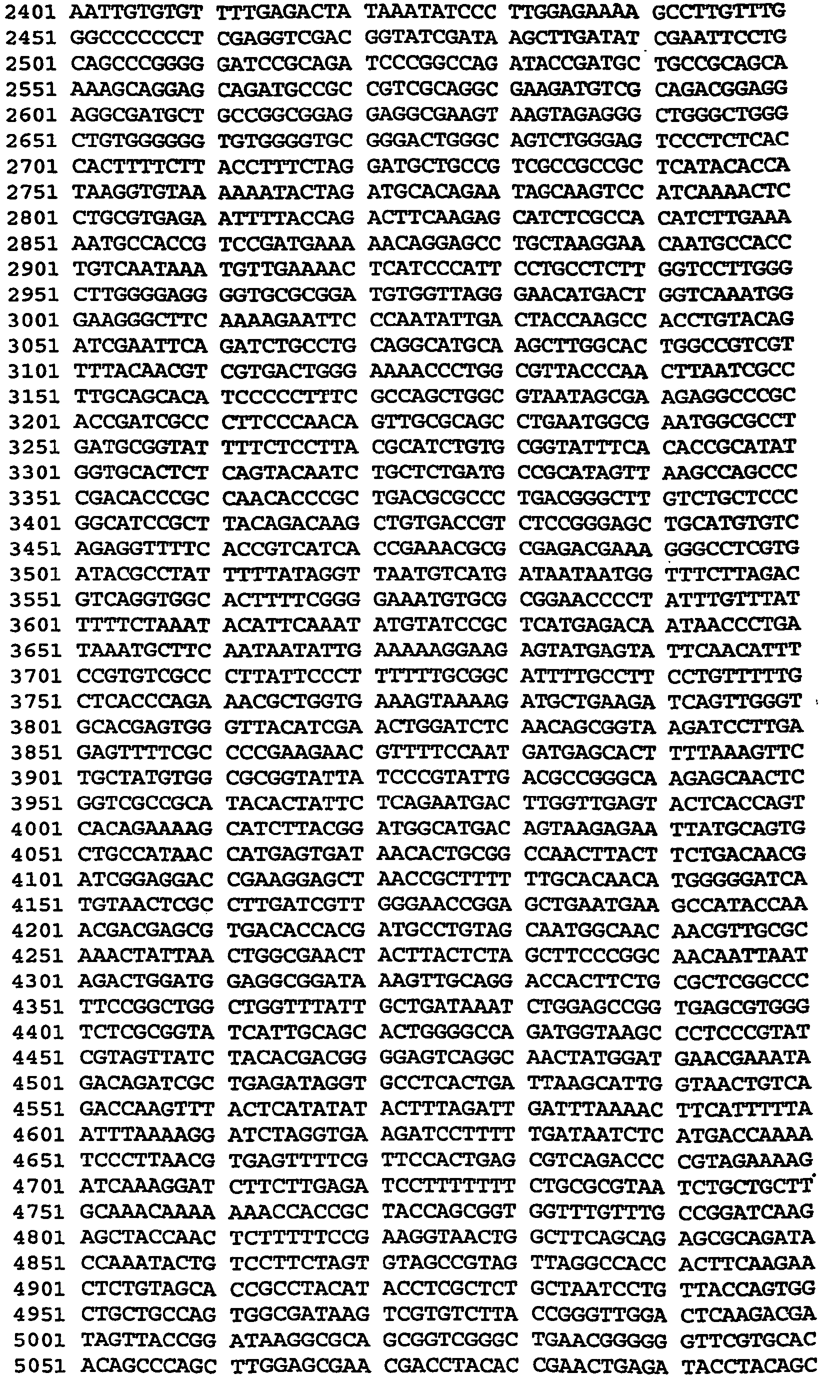

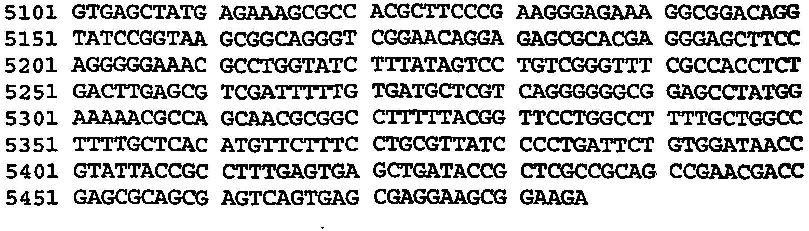

## Claims

1. A nucleic acid construct comprising a Pol III//Pol II fusion promoter comprising
a sequence that binds an RNA Polymerase III complex, wherein the sequence consists of a Pol III basal promoter region; and
cis-acting regulatory region from a Pol II promoter region, wherein said cis-acting regulatory region is operably linked with said basal promoter region, and provides specific regulation of expression from said construct.

2. The construct of claim 1, wherein said construct further comprises a sequence encoding an RNAi agent operably linked with said fusion promoter.

3. The construct of claim 1, wherein said basal promoter region is from a Pol III promoter; is a U6 basal promoter; is an H1 basal promoter; is a tRNA basal promoter; or is a mutated Pol II basal promoter, wherein said mutated Pol II basal promoter preferentially binds Pol III instead of Pol II.

4. The construct of claim 1, wherein said Pol II cis-acting regulatory region comprises a CMV early intermediate regulatory region or a complete Pol II regulatory region less the basal promoter.

5. The construct of claim 1, further comprising a sequence encoding an RNAi agent targeting an mRNA of a disease-associated gene.

6. A vector comprising the nucleic acid construct of any of claims 1-5.

7. A cell comprising the nucleic acid construct or vector of any of claims 1-6.

8. A non-human transgenic organism, comprising
a plurality of cells comprising the nucleic acid construct of any of claims 1-5.

9. A pharmaceutical composition comprising the nucleic acid construct or vector of any of claims 1-6, and
a pharmaceutically acceptable carrier or excipient.

10. A method for making a genetic construct, comprising operably linking a nucleic acid sequence encoding an RNAi agent with a Pol III/ Pol II fusion promoter, wherein said promoter comprises a sequence that binds an RNA polymerase III complex, wherein the sequence consists of a Pol III basal promoter, and a cis-acting regulatory region from a Pol II promoter region, wherein said cis-acting region is operably linked with said basal promoter region, and allows specific regulation of expression from said fusion promoter.

11. An in vitro method for expressing an RNAi agent in a cell, comprising
maintaining said a cell under expression conditions, wherein said cell comprises a genetic construct comprising a Pol III/ Pol II fusion promoter operably linked with an RNAi agent encoding sequence, wherein said Pol III/Pol II fusion promoter comprises
a sequence that binds an DNA polymerase III complex, wherein the sequence consists of a Pol III basal promoter region; and
a cis-acting regulatory region from a Pol II promoter region, wherein said cis-acting region is operably linked with said basal promoter region and allows specific regulation of expression from said fusion promoter.

12. An in vitro method for inhibiting expression of a target gene in a cell, comprising:
transfecting said cell with a vector comprising a genetic construct, wherein said construct comprises a Pol III/Pol II fusion promoter operably linked with a nucleic acid sequence encoding an RNAi agent targeted to said target gene,
wherein said Pol III/Pol II fusion promoter comprises a sequence that binds an DNA polymerase III complex, wherein the sequence consists of a Pol III basal promoter region; and a cis-acting regulatory region from a Pol II promoter region, wherein said cis-acting region is operably linked with said basal promoter region, and allows specific regulation of expression from said fusion promoter; and
maintaining said cell under expression conditions.

13. An in vitro method for analyzing gene function in a cell, comprising
inhibiting expression of a gene in said cell, wherein said inhibiting is due to expression of an RNAi agent from a genetic construct comprising a Pol III/Pol II fusion promoter operably linked with a nucleic acid sequence encoding said RNAi agent,
wherein said fusion promoter comprises a sequence that binds an RNA Polymerase III complex, wherein the sequence consists ofa Pol III basal promoter region; and a cis-acting regulatory region from a Pol II promoter region, wherein said cis-acting region is operably linked with said basal promoter region and allows specific regulation of expression from said fusion promoter; and
determining a biological change in said cell following said inhibiting, wherein said biological change is indicative of the function of said gene.

14. An in vitro method for validating a target as a therapeutic target in a cell, comprising:
inhibiting expression of a putative therapeutic target gene in a cell, wherein said inhibiting is due to expression of an RNAi agent from a genetic construct comprising a Pol III/Pol II fusion promoter operably linked with a nucleic acid sequence encoding said RNAi agent,
wherein said fusion promoter comprises a sequence that binds an RNA polymerase III complex, wherein the sequence consists of a Pol III basal promoter region; and a cis-acting regulatory region from a Pol II promoter region, wherein said cis-acting region is operably linked with said basal promoter region and allows specific regulation of expression from said fusion promoter; and
determining whether a biological change in said cell following said inhibiting corresponds with a therapeutic effect, wherein correspondence of said biological change with said therapeutic effect is indicative that said gene is a therapeutic target gene.

15. An in vitro method for positive control of a biological effect of a small molecule test compound, comprising
contacting a first cell with a test compound;
inhibiting a target gene in a comparison cell in vitro using expression of an RNAi agent from a genetic construct comprising a Pol III/Pol II fusion promoter providing specific regulation of expression, operably linked with a sequence encoding said RNAi agent, wherein said fusion promoter comprises a sequence that binds an RNA polymerase III complex, wherein the sequence consists of a Pol III basal promoter region; and a cis-acting regulator region from a Pol II promoter region, wherein said cis-acting region is operably linked with said basal promoter region, and allows specific regulation of expression from said fusion promoter; and
comparing the effect of said test compound in said first cell with the effect of inhibition of said target gene in said comparison cell.

16. Use of a vector comprising a genetic construct comprising a Pol III/Pol II fusion promoter providing specific regulation of expression, operably linked with a sequence encoding an RNAi agent, wherein said fusion promoter comprises a sequence that binds an RNA polymerase III complex, wherein the sequence consists of a Pol III basal promoter region and a cis-acting regulatory region from a Pol II promoter region, wherein said cis-acting region is operably linked with said basal promoter region, and provides specific regulation of expression from said fusion promoter in preparation of a medicament for treatment of a disease or condition wherein inhibition of a target gene provides a beneficial effect.

## Patentansprüche

1. Nucleinsäurekonstrukt, umfassend einen Pol-III/Pol-II-Fusionspromotor, umfassend
eine Sequenz, die einen RNA-Polymerase-III-Komplex bindet, wobei die Sequenz aus einer basalen Pol-III-Promotor-Region besteht; und
cis-wirkende regulatorische Region aus einer Pol-II-Promotor-Region, wobei die cis-wirkende regulatorische Region operabel mit der basalen Promotor-Region verknüpft ist und spezifische Regulation von Expression aus dem Konstrukt bereitstellt.

2. Konstrukt nach Anspruch 1, wobei das Konstrukt weiterhin eine Sequenz, codierend ein RNAi-Mittel, operabel verknüpft mit dem Fusionspromotor, umfasst.

3. Konstrukt nach Anspruch 1, wobei die basale Promotor-Region von einem Pol-III-Promotor ist; ein basaler U6-Promotor ist; ein basaler H1-Promotor ist; ein basaler tRNA-Promotor ist; oder ein mutierter basaler Pol-II-Promotor ist, wobei der mutierte basale Pol-II-Promotor vorzugsweise Pol III anstatt Pol II bindet.

4. Konstrukt nach Anspruch 1, wobei die cis-wirkende regulatorische Pol-II-Region eine frühe intermediäre regulatorische CMV-Region oder eine vollständige regulatorische Pol-II-Region ohne den basalen Promotor umfasst.

5. Konstrukt nach Anspruch 1, weiterhin umfassend eine Sequenz, codierend ein RNAi-Mittel, zielend auf eine mRNA eines Krankheits-assoziierten Gens.

6. Vektor, umfassend das Nucleinsäurekonstrukt nach einem der Ansprüche 1-5.

7. Zelle, umfassend das Nucleinsäurekonstrukt oder den Vektor nach einem der Ansprüche 1-6.

8. Nicht-humaner transgener Organismus, umfassend
eine Mehrzahl von Zellen, umfassend das Nucleinsäurekonstrukt nach einem der Ansprüche 1-5.

9. Pharmazeutische Zusammensetzung, umfassend das Nucleinsäurekonstrukt oder den Vektor nach einem der Ansprüche 1-6; und
einen pharmazeutisch verträglichen Träger oder Excipienten.

10. Verfahren zum Herstellen eines genetischen Konstrukts, umfassend, operabel eine Nucleinsäuresequenz, codierend ein RNAi-Mittel, mit einem Pol-III/Pol-II-Fusionspromotor zu verknüpfen, wobei der Promotor umfasst eine Sequenz, die einen RNA-Polymerase-III-Komplex bindet, wobei die Sequenz aus einem basalen Pol-III-Promotor besteht, und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt.

11. In-vitro-Verfahren zum Exprimieren eines RNAi-Mittels in einer Zelle, umfassend
Halten der Zelle unter Expressionsbedingungen, wobei die Zelle ein genetisches Konstrukt, umfassend einen Pol-III/Pol-II-Fusionspromotor, operabel verknüpft mit einer RNAi-Mittel codierenden Sequenz, umfasst, wobei der Pol-III/Pol-II-Fusionspromotor umfasst
eine Sequenz, die einen RNA-Polymerase-III-Komplex bindet, wobei die Sequenz aus einer basalen Pol-III-Promotor-Region besteht; und
eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt.

12. In-vitro-Verfahren zum Hemmen der Expression eines Ziel-Gens in einer Zelle, umfassend:
Transfizieren der Zelle mit einem Vektor, umfassend ein genetisches Konstrukt, wobei das Konstrukt einen Pol-III/Pol-II-Fusionspromotor, operabel verknüpft mit einer Nucleinsäuresequenz, codierend ein RNAi-Mittel, gezielt auf das Ziel-Gen, umfasst,
wobei der Pol-III/Pol-II-Fusionspromotor umfasst eine Sequenz, die einen RNA-Polymerase-III-Komplex bindet, wobei die Sequenz aus einer basalen Pol-III-Promotor-Region besteht; und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt; und
Halten der Zelle unter Expressionsbedingungen.

13. In-vitro-Verfahren zum Analysieren der Gen-Funktion in einer Zelle, umfassend
Hemmen der Expression eines Gens in der Zelle, wobei das Hemmen auf Expression eines RNAi-Mittels aus einem genetischen Konstrukt, umfassend einen Pol-III/Pol-II-Fusionspromotor, operabel verknüpft mit einer Nucleinsäuresequenz, codierend das RNAi-Mittel, zurückzuführen ist,
wobei der Fusionspromotor umfasst eine Sequenz, die einen RNA-Polymerase-III-Komplex bindet, wobei die Sequenz aus einer basalen Pol-III-Promotor-Region besteht; und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt; und
Bestimmen einer biologischen Veränderung in der Zelle nach dem Hemmen, wobei die biologische Veränderung ein Anzeichen der Funktion des Gens ist.

14. In-vitro-Verfahren zum Validieren eines Ziels als ein therapeutisches Ziel in einer Zelle, umfassend:
Hemmen der Expression eines mutmaßlichen therapeutischen Ziel-Gens in einer Zelle, wobei das Hemmen auf Expression eines RNAi-Mittels aus einem genetischen Konstrukt, umfassend einen Pol-III/Pol-II-Fusionspromotor, operabel verknüpft mit einer Nucleinsäuresequenz, codierend das RNAi Mittel, zurückzuführen ist,
wobei der Fusionspromotor umfasst eine Sequenz, die einen RNA-Polymerase-III-Komplex bindet, wobei die Sequenz aus einer basalen Pol-III-Promotor-Region besteht; und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt; und
Bestimmen, ob eine biologische Veränderung in der Zelle nach dem Hemmen mit einer therapeutischen Wirkung korrespondiert, wobei Korrespondenz der biologischen Veränderung mit der therapeutischen Wirkung ein Anzeichen ist, dass das Gen ein therapeutisches Ziel-Gen ist.

15. In-vitro-Verfahren für positive Kontrolle einer biologischen Wirkung einer Testverbindung in Form eines kleinen Moleküls, umfassend
Inkontaktbringen einer ersten Zelle mit einer Testverbindung;
Hemmen eines Ziel-Gens in einer Vergleichszelle in vitro unter Verwendung der Expression eines RNAi-Mittels aus einem genetischen Konstrukt, umfassend einen Pol-III/Pol-II-Fusionspromotor, bereitstellend spezifische Regulation von Expression, operabel verknüpft mit einer Sequenz, codierend das RNAi-Mittel, wobei der Fusionspromotor umfasst eine Sequenz, die einen RNA-Polymerase-III-Komplex bindet, wobei die Sequenz aus einer basalen Pol-III-Promotor-Region besteht; und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor erlaubt; und
Vergleichen der Wirkung der Testverbindung in der ersten Zelle mit der Wirkung der Hemmung des Ziel-Gens in der Vergleichszelle.

16. Verwendung eines Vektors, umfassend ein genetisches Konstrukt, umfassend einen Pol-III/Pol-II-Fusionspromotor, bereitstellend spezifische Regulation von Expression, operabel verknüpft mit einer Sequenz, codierend ein RNAi-Mittel, wobei der Fusionspromotor umfasst eine Sequenz, die einen RNA-Polymerase-III-Komplex bindet, wobei die Sequenz aus einer basalen Pol-III-Promotor-Region besteht, und eine cis-wirkende regulatorische Region aus einer Pol-II-Promotor-Region, wobei die cis-wirkende Region operabel mit der basalen Promotor-Region verknüpft ist und spezifische Regulation von Expression aus dem Fusionspromotor bereitstellt, in der Herstellung eines Medikaments zur Behandlung einer Krankheit oder eines Leidens, wobei Hemmung eines Ziel-Gens eine vorteilhafte Wirkung bereitstellt.

## Revendications

1. Construction d'acide nucléique comprenant un promoteur de fusion Pol III/Pol II comprenant:
une séquence qui se lie avec un complexe d'ARN polymérase III, dans laquelle la séquence est constituée d'une région de promoteur basal Pol III; et
une région régulatrice agissant en cis issue d'une région de promoteur Pol II, dans laquelle ladite région régulatrice agissant en cis est liée de manière fonctionnelle avec ladite région de promoteur basal et apporte une régulation spécifique d'expression provenant de ladite construction.

2. Construction selon la revendication 1, où ladite construction comprend en outre une séquence codant pour un agent ARNi liée de manière fonctionnelle avec ledit promoteur de fusion.

3. Construction selon la revendication 1, dans laquelle ladite région de promoteur basal est issue d'un promoteur Pol III; est un promoteur basal U6; est un promoteur basal H1; est un promoteur basal ARNt; ou est un promoteur basal Pol II muté, dans laquelle ledit promoteur basal Pol II muté se lie préférentiellement avec Pol III au lieu de Pol II.

4. Construction selon la revendication 1, dans laquelle ladite région régulatrice agissant en cis Pol II comprend une région régulatrice intermédiaire précoce de CMV ou une région régulatrice Pol II complète moins le promoteur basal.

5. Construction selon la revendication 1, comprenant en outre une séquence codant pour un agent ARNi ciblant un ARNm d'un gène associé à une maladie.

6. Vecteur comprenant la construction d'acide nucléique selon l'une quelconque des revendications 1-5.

7. Cellule comprenant la construction d'acide nucléique ou le vecteur selon l'une quelconque des revendications 1-6.

8. Organisme transgénique non humain, comprenant:
une pluralité de cellules comprenant la construction d'acide nucléique selon l'une quelconque des revendications 1-5.

9. Composition pharmaceutique comprenant la construction d'acide nucléique ou le vecteur selon l'une quelconque des revendications 1-6, et
un support ou un excipient pharmaceutiquement acceptable.

10. Procédé pour la fabrication d'une construction génétique, comprenant la liaison de manière fonctionnelle d'une séquence d'acide nucléique codant pour un agent ARNi avec un promoteur de fusion Pol III/Pol II, dans lequel ledit promoteur comprend une séquence qui se lie avec un complexe d'ARN polymérase III, dans lequel la séquence est constituée d'un promoteur basal Pol III, et une région régulatrice agissant en cis issue d'une région de promoteur Pol II, dans lequel ladite région agissant en cis est liée de manière fonctionnelle avec ladite région de promoteur basal et permet une régulation spécifique d'expression provenant dudit promoteur de fusion.

11. Méthode in vitro pour l'expression d'un agent ARNi dans une cellule, comprenant:
le maintien de ladite une cellule dans des conditions d'expression, dans laquelle ladite cellule comprend une construction génétique comprenant un promoteur de fusion Pol III/Pol II lié de manière fonctionnelle avec un agent ARNi codant pour une séquence, dans laquelle ledit promoteur de fusion Pol III/Pol II comprend:
une séquence qui se lie avec un complexe d'ARN polymérase III, dans laquelle la séquence est constituée d'une région de promoteur basal Pol III; et
une région régulatrice agissant en cis issue d'une région de promoteur Pol II, dans laquelle ladite région agissant en cis est liée de manière fonctionnelle avec ladite région de promoteur basal et permet une régulation spécifique d'expression provenant dudit promoteur de fusion.

12. Méthode in vitro pour l'inhibition de l'expression d'un gène cible dans une cellule, comprenant:
la transfection de ladite cellule avec un vecteur comprenant une construction génétique, dans laquelle ladite construction comprend un promoteur de fusion Pol III/Pol II lié de manière fonctionnelle avec une séquence d'acide nucléique codant pour un agent ARNi ciblé vers ledit gène cible,
dans laquelle ledit promoteur de fusion Pol III/Pol II comprend une séquence qui se lie avec un complexe d'ARN polymérase III, dans laquelle la séquence est constituée d'une région de promoteur basal Pol III; et une région régulatrice agissant en cis issue d'une région de promoteur Pol II, dans laquelle ladite région agissant en cis est liée de manière fonctionnelle avec ladite région de promoteur basal et permet une régulation spécifique d'expression provenant dudit promoteur de fusion; et
le maintien de ladite cellule dans des conditions d'expression.

13. Méthode in vitro pour l'analyse d'une fonction de gène dans une cellule, comprenant:
l'inhibition de l'expression d'un gène dans ladite cellule, dans laquelle ladite inhibition est due à l'expression d'un agent ARNi provenant d'une construction génétique comprenant un promoteur de fusion Pol III/Pol II lié de manière fonctionnelle avec une séquence d'acide nucléique codant pour ledit agent ARNi,
dans laquelle ledit promoteur de fusion comprend une séquence qui se lie avec un complexe d'ARN polymérase III, dans laquelle la séquence est constituée d'une région de promoteur basal Pol III; et une région régulatrice agissant en cis issue d'une région de promoteur Pol II, dans laquelle ladite région agissant en cis est liée de manière fonctionnelle avec ladite région de promoteur basal et permet une régulation spécifique d'expression provenant dudit promoteur de fusion; et
la détermination d'un changement biologique dans ladite cellule à la suite de ladite inhibition, dans laquelle ledit changement biologique est indicateur de la fonction dudit gène.

14. Méthode in vitro pour la validation d'une cible en tant que cible thérapeutique dans une cellule, comprenant:
l'inhibition de l'expression d'un gène cible thérapeutique putatif dans une cellule, dans laquelle ladite inhibition est due à l'expression d'un agent ARNi provenant d'une construction génétique comprenant un promoteur de fusion Pol III/Pol II lié de manière fonctionnelle avec une séquence d'acide nucléique codant pour ledit agent ARNi,
dans laquelle ledit promoteur de fusion comprend une séquence qui se lie avec un complexe d'ARN polymérase III, dans laquelle la séquence est constituée d'une région de promoteur basal Pol III; et une région régulatrice agissant en cis issue d'une région de promoteur Pol II, dans laquelle ladite région agissant en cis est liée de manière fonctionnelle avec ladite région de promoteur basal et permet une régulation spécifique d'expression provenant dudit promoteur de fusion; et
la détermination si un changement biologique dans ladite cellule à la suite de ladite inhibition correspond à un effet thérapeutique, dans laquelle la correspondance dudit changement biologique avec ledit effet thérapeutique est indicatrice du fait que ledit gène est un gène cible thérapeutique.

15. Méthode in vitro pour le contrôle positif d'un effet biologique d'un composé d'essai à petite molécule, comprenant:
la mise en contact d'une première cellule avec un composé d'essai;
l'inhibition d'un gène cible dans une cellule de comparaison in vitro en utilisant l'expression d'un agent ARNi provenant d'une construction génétique comprenant un promoteur de fusion Pol III/Pol II apportant une régulation spécifique d'expression, lié de manière fonctionnelle avec une séquence codant pour ledit agent ARNi, dans laquelle ledit promoteur de fusion comprend une séquence qui se lie avec un complexe d'ARN polymérase III, dans laquelle la séquence est constituée d'une région de promoteur basal Pol III; et une région régulatrice agissant en cis issue d'une région de promoteur Pol II, dans laquelle ladite région agissant en cis est liée de manière fonctionnelle avec ladite région de promoteur basal et permet une régulation spécifique d'expression provenant dudit promoteur de fusion; et
la comparaison de l'effet dudit composé d'essai dans ladite première cellule avec l'effet d'inhibition dudit gène cible dans ladite cellule de comparaison.

16. Utilisation d'un vecteur, comprenant une construction génétique comprenant un promoteur de fusion Pol III/Pol II apportant une régulation spécifique d'expression, lié de manière fonctionnelle avec une séquence codant pour un agent ARNi, dans laquelle ledit promoteur de fusion comprend une séquence qui se lie avec un complexe d'ARN polymérase III, dans laquelle la séquence est constituée d'une région de promoteur basal Pol III, et une région régulatrice agissant en cis issue d'une région de promoteur Pol II, dans laquelle ladite région agissant en cis est liée de manière fonctionnelle avec ladite région de promoteur basal et apporte une régulation spécifique d'expression provenant dudit promoteur de fusion, dans la préparation d'un médicament pour le traitement d'une maladie ou d'un état, dans laquelle l'inhibition d'un gène cible apporte un effet bénéfique.


### SEQUENCE LISTING

<110> Charles Stout
<120> REGULATABLE FUSION PROMOTERS
<130> 21683-002WO1
<150> US 60/722,568
   <151> 2005-10-01
<160> 7
<170> FastSEQ for Windows Version 4.0
<210> 1
   <211> 3292
   <212> DNA
   <213> Artificial Sequence
<220>
   <223> pSilencer 1.0
<220>
   <221> misc_feature
   <222> 2225, 2226
   <223> n = A,T,C or G
<400> 1
   ctaaattgta agcgttaata ttttgttaaa attcgcgtta aatttttgtt aaatcagctc 60
   attttttaac caataggccg aaatcggcaa aatcccttat aaatcaaaag aatagaccga 120
   gatagggttg agtgttgttc cagtttggaa caagagtcca ctattaaaga acgtggactc 180
   caacgtcaaa gggcgaaaaa ccgtctatca gggcgatggc ccactacgtg aaccatcacc 240
   ctaatcaagt tttttggggt cgaggtgccg taaagcacta aatcggaacc ctaaagggag 300
   cccccgattt agagcttgac ggggaaagcc ggcgaacgtg gcgagaaagg aagggaagaa 360
   agcgaaagga gcgggcgcta gggcgctggc aagtgtagcg gtcacgctgc gcgtaaccac 420
   cacacccgcc gcgcttaatg cgccgctaca gggcgcgtcc cattcgccat tcaggctgcg 480
   caactgttgg gaagggcgat cggtgcgggc ctcttcgcta ttacgccagc tggcgaaagg 540
   gggatgtgct gcaaggcgat taagttgggt aacgccaggg ttttcccagt cacgacgttg 600
   taaaacgacg gccagtgagc gcgcgtaata cgactcacta tagggcgaat tgggtacccg 660
   ctctagaact agtggatccg acgccgccat ctctaggccc gcgccggccc cctcgcacag 720
   acttgtggga gaagctcggc tactcccctg ccccggttaa tttgcatata atatttccta 780
   gtaactatag aggcttaatg tgcgataaaa gacagataat ctgttctttt taatactagc 840
   tacattttac atgataggct tggatttcta taagagatac aaatactaaa ttattatttt 900
   aaaaaacagc acaaaaggaa actcacccta actgtaaagt aattgtgtgt tttgagacta 960
   taaatatccc ttggagaaaa gccttgtttg ggccccccct cgaggtcgac ggtatcgata 1020
   agcttgatat cgaattcctg cagcccgggg gatccactag ttctagagcg gccgccaccg 1080
   cggtggagct ccagcttttg ttccctttag tgagggttaa ttgcgcgctt ggcgtaatca 1140
   tggtcatagc tgtttcctgt gtgaaattgt tatccgctca caattccaca caacatacga 1200
   gccggaagca taaagtgtaa agcctggggt gcctaatgag tgagctaact cacattaatt 1260
   gcgttgcgct cactgcccgc tttccagtcg ggaaacctgt cgtgccagct gcattaatga 1320
   atcggccaac gcgcggggag aggcggtttg cgtattgggc gctcttccgc ttcctcgctc 1380
   actgactcgc tgcgctcggt cgttcggctg cggcgagcgg tatcagctca ctcaaaggcg 1440
   gtaatacggt tatccacaga atcaggggat aacgcaggaa agaacatgtg agcaaaaggc 1500
   cagcaaaagg ccaggaaccg taaaaaggcc gcgttgctgg cgtttttcca taggctccgc 1560
   ccccctgacg agcatcacaa aaatcgacgc tcaagtcaga ggtggcgaaa cccgacagga 1620
   ctataaagat accaggcgtt tccccctgga agctccctcg tgcgctctcc tgttccgacc 1680
   ctgccgctta ccggatacct gtccgccttt ctcccttcgg gaagcgtggc gctttctcat 1740
   agctcacgct gtaggtatct cagttcggtg taggtcgttc gctccaagct gggctgtgtg 1800
   cacgaacccc ccgttcagcc cgaccgctgc gccttatccg gtaactatcg tcttgagtcc 1860
   aacccggtaa gacacgactt atcgccactg gcagcagcca ctggtaacag gattagcaga 1920
   gcgaggtatg taggcggtgc tacagagttc ttgaagtggt ggcctaacta cggctacact 1980
   agaagaacag tatttggtat ctgcgctctg ctgaagccag ttaccttcgg aaaaagagtt 2040
   ggtagctctt gatccggcaa acaaaccacc gctggtagcg gtggtttttt tgtttgcaag 2100
   cagcagatta cgcgcagaaa aaaaggatct caagaagatc ctttgatctt ttctacgggg 2160
   tctgacgctc agtggaacga aaactcacgt taagggattt tggtcatgag attatcaaaa 2220
   aggannttca cctagatcct tttaaattaa aaatgaagtt ttaaatcaat ctaaagtata 2280
   tatgagtaaa cttggtctga cagttaccaa tgcttaatca gtgaggcacc tatctcagcg 2340
   atctgtctat ttcgttcatc catagttgcc tgactccccg tcgtgtagat aactacgata 2400
   cgggagggct taccatctgg ccccagtgct gcaatgatac cgcgagaccc acgctcaccg 2460
   gctccagatt tatcagcaat aaaccagcca gccggaaggg ccgagcgcag aagtggtcct 2520
   gcaactttat ccgcctccat ccagtctatt aattgttgcc gggaagctag agtaagtagt 2580
   tcgccagtta atagtttgcg caacgttgtt gccattgcta caggcatcgt ggtgtcacgc 2640
   tcgtcgtttg gtatggcttc attcagctcc ggttcccaac gatcaaggcg agttacatga 2700
   tcccccatgt tgtgcaaaaa agcggttagc tccttcggtc ctccgatcgt tgtcagaagt 2760
   aagttggccg cagtgttatc actcatggtt atggcagcac tgcataattc tcttactgtc 2820
   atgccatccg taagatgctt ttctgtgact ggtgagtact caaccaagtc attctgagaa 2880
   tagtgtatgc ggcgaccgag ttgctcttgc ccggcgtcaa tacgggataa taccgcgcca 2940
   catagcagaa ctttaaaagt gctcatcatt ggaaaacgtt cttcggggcg aaaactctca 3000
   aggatcttac cgctgttgag atccagttcg atgtaaccca ctcgtgcacc caactgatct 3060
   tcagcatctt ttactttcac cagcgtttct gggtgagcaa aaacaggaag gcaaaatgcc 3120
   gcaaaaaagg gaataagggc gacacggaaa tgttgaatac tcatactctt cctttttcaa 3180
   tattattgaa gcatttatca gggttattgt ctcatgagcg gatacatatt tgaatgtatt 3240
   tagaaaaata aacaaatagg ggttccgcgc acatttcccc gaaaagtgcc ac         3292
<210> 2
   <211> 2129
   <212> DNA
   <213> Artificial Sequence
<220>
   <223> Synthetic construct
<400> 2
   agatctgagc tcccacctcc ctctctgtgc tgggactcac agagggagac ctcaggaggc 60
   agtctgtcca tcacatgtcc aaatgcagag cataccctgg gctgggcgca gtggcgcaca 120
   actgtaattc cagcactttg ggaggctgat gtggaaggat cacttgagcc cagaagttct 180
   agaccagcct gggcaacatg gcaagaccct atctctacaa aaaaagttaa aaaatcagcc 240
   acgtgtggtg acacacacct gtagtcccag ctattcagga ggctgaggtg aggggatcac 300
   ttaaggctgg gaggttgagg ctgcagtgag tcgtggttgc gccactgcac tccagcctgg 360
   gcaacagtga gaccctgtct caaaagacaa aaaaaaaaaa aaaaaaaaaa agaacatatc 420
   ctggtgtgga gtaggggacg ctgctctgac agaggctcgg gggcctgagc tggctctgtg 480
   agctggggag gaggcagaca gccaggcctt gtctgcaagc agacctggca gcattgggct 540
   ggccgccccc cagggcctcc tcttcatgcc cagtgaatga ctcaccttgg cacagacaca 600
   atgttcgggg tgggcacagt gcctgcttcc cgccgcaccc cagcccccct caaatgcctt 660
   ccgagaagcc cattgagcag ggggcttgca ttgcacccca gcctgacagc ctggcatctt 720
   gggataaaag cagcacagcc ccctaggggc tgcccttgct gtgtggcgcc accggcggtg 780
   gagaacaagg ctctattcag cctgtgccca ggaaagggga tcaggggatg cccaggcatg 840
   gacagtgggt ggcagggggg gagaggaggg ctgtctgctt cccagaagtc caaggacaca 900
   aatgggtgag gggactgggc agggttctga ccctgtggga ccagagtgga gggcgtagat 960
   ggacctgaag tctccaggga caacagggcc caggtctcag gctcctagtt gggcccagtg 1020
   gctccagcgt ttccaaaccc atccatcccc agaggttctt cccatctctc caggctgatg 1080
   tgtgggaact cgaggaaata aatctccagt gggagacgga ggggtggcca gggaaacggg 1140
   gcgctgcagg aataaagacg agccagcaca gccagctcat gtgtaacggc tttgtggagc 1200
   tgtcaaggcc tggtctctgg gagagaggca cagggaggcc agacaaggaa ggggtgacct 1260
   ggagggacag atccaggggc taaagtcctg ataaggcaag agagtgccgg ccccctcttg 1320
   ccctatcagg acctccactg ccacatagag gccatgattg acccttagac aaagggctgg 1380
   tgtccaatcc cagcccccag ccccagaact ccagggaatg aatgggcaga gagcaggaat 1440
   gtgggacatc tgtgttcaag ggaaggactc caggagtctg ctgggaatga ggcctagtag 1500
   gaaatgaggt ggcccttgag ggtacagaac aggttcattc ttcgccaaat tcccagcacc 1560
   ttgcaggcac ttacagctga gtgagataat gcctgggtta tgaaatcaaa aagttggaaa 1620
   gcaggtcaga ggtcatctgg tacagccctt ccttcccttt tttttttttt ttttttgtga 1680
   gacaaggtct ctctctgttg cccaggctgg agtggcgcaa acacagctca ctgcagcctc 1740
   aacctactgg gctcaagcaa tcctccagcc tcagcctccc aaagtgctgg gattacaagc 1800
   atgagccacc ccactcagcc ctttccttcc tttttaattg atgcataata attgtaagta 1860
   ttcatcatgg tccaaccaac cctttcttga cccaccttcc tagagagagg gtcctcttgc 1920
   ttcagcggtc agggccccag acccatggtc tggctccagg taccacctgc ctctaaaaaa 1980
   cagcacaaaa ggaaactcac cctaactgta aagtaattgt gtgttttgag actataaata 2040
   tcccttggag aaaagccttg tttggccccc ccctcgaggt cgacggtatc gataagcttg 2100
   atatcgaatt cctgcagccc gggggatcc                                   2129
<210> 3
   <211> 2269
   <212> DNA
   <213> Artificial Sequence
<220>
   <223> Synthetic construct
<400> 3
   agatctgagc tcccacctcc ctctctgtgc tgggactcac agagggagac ctcaggaggc 60
   agtctgtcca tcacatgtcc aaatgcagag cataccctgg gctgggcgca gtggcgcaca 120
   actgtaattc cagcactttg ggaggctgat gtggaaggat cacttgagcc cagaagttct 180
   agaccagcct gggcaacatg gcaagaccct atctctacaa aaaaagttaa aaaatcagcc 240
   acgtgtggtg acacacacct gtagtcccag ctattcagga ggctgaggtg aggggatcac 300
   ttaaggctgg gaggttgagg ctgcagtgag tcgtggttgc gccactgcac tccagcctgg 360
   gcaacagtga gaccctgtct caaaagacaa aaaaaaaaaa aaaaaaaaaa agaacatatc 420
   ctggtgtgga gtaggggacg ctgctctgac agaggctcgg gggcctgagc tggctctgtg 480
   agctggggag gaggcagaca gccaggcctt gtctgcaagc agacctggca gcattgggct 540
   ggccgccccc cagggcctcc tcttcatgcc cagtgaatga ctcaccttgg cacagacaca 600
   atgttcgggg tgggcacagt gcctgcttcc cgccgcaccc cagcccccct caaatgcctt 660
   ccgagaagcc cattgagcag gggcttgcag ttgcacccca gcctgacagc ctggcatctt 720
   gggataaaag cagcacagcc ccctaggggc tgcccttgct gtgtggcgcc accggcggtg 780
   gagaacaagg ctctattcag cctgtgccca ggaaagggga tcaggggatg cccaggcatg 840
   gacagtgggt ggcagggggg gagaggaggg ctgtctgctt cccagaagtc caaggacaca 900
   aatgggtgag gggactgggc agggttctga ccctgtggga ccagagtgga gggcgtagat 960
   ggacctgaag tctccaggga caacagggcc caggtctcag gctcctagtt gggcccagtg 1020
   gctccagcgt ttccaaaccc atccatcccc agaggttctt cccatctctc caggctgatg 1080
   tgtgggaact cgaggaaata aatctccagt gggagacgga ggggtggcca gggaaacggg 1140
   gcgctgcagg aataaagacg agccagcaca gccagctcat gtgtaacggc tttgtggagc 1200
   tgtcaaggcc tggtctctgg gagagaggca cagggaggcc agacaaggaa ggggtgacct 1260
   ggagggacag atccaggggc taaagtcctg ataaggcaag agagtgccgg ccccctcttg 1320
   ccctatcagg acctccactg ccacatagag gccatgattg acccttagac aaagggctgg 1380
   tgtccaatcc cagcccccag ccccagaact ccagggaatg aatgggcaga gagcaggaat 1440
   gtgggacatc tgtgttcaag ggaaggactc caggagtctg ctgggaatga ggcctagtag 1500
   gaaatgaggt ggcccttgag ggtacagaac aggttcattc ttcgccaaat tcccagcacc 1560
   ttgcaggcac ttacagctga gtgagataat gcctgggtta tgaaatcaaa aagttggaaa 1620
   gcaggtcaga ggtcatctgg tacagccctt ccttcccttt tttttttttt ttttttgtga 1680
   gacaaggtct ctctctgttg cccaggctgg agtggcgcaa acacagctca ctgcagcctc 1740
   aacctactgg gctcaagcaa tcctccagcc tcagcctccc aaagtgctgg gattacaagc 1800
   atgagccacc ccactcagcc ctttccttcc tttttaattg atgcataata attgtaagta 1860
   ttcatcatgg tccaaccaac cctttcttga cccaccttcc tagagagagg gtcctcttgc 1920
   ttcagcggtc agggccccag acccatggtc tggctccagg taccacctgc ctcatgcagg 1980
   agttggcgtg cccaggaagc tctgcctctg ggcacagtga cctcagtggg gtgaggggag 2040
   ctctccccat agctgggctg cggcccaacc ccaccccctc aggctatgcc agggggtgtt 2100
   gccaggggca ccctaaaaaa cagcacaaaa ggaaactcac cctaactgta aagtaattgt 2160
   gtgttttgag actataaata tcccttggag aaaagccttg tttgggcccc ccctcgaggt 2220
   cgacggtatc gataagcttg atatcgaatt cctgcagccc gggggatcc 2269
<210> 4
   <211> 6710
   <212> DNA
   <213> Artificial Sequence
<220>
   <223> Expression vector peGFP-HcRed1
<400> 4
   tcaatattgg ccattagcca tattattcat tggttatata gcataaatca atattggcta 60
   ttggccattg catacgttgt atctatatca taatatgtac atttatattg gctcatgtcc 120
   aatatgaccg ccatgttggc attgattatt gactagttat taatagtaat caattacggg 180
   gtcattagtt catagcccat atatggagtt ccgcgttaca taacttacgg taaatggccc 240
   gcctggctga ccgcccaacg acccccgccc attgacgtca ataatgacgt atgttcccat 300
   agtaacgcca atagggactt tccattgacg tcaatgggtg gagtatttac ggtaaactgc 360
   ccacttggca gtacatcaag tgtatcatat gccaagtccg ccccctattg acgtcaatga 420
   cggtaaatgg cccgcctggc attatgccca gtacatgacc ttacgggact ttcctacttg 480
   gcagtacatc tacgtattag tcatcgctat taccatggtg atgcggtttt ggcagtacac 540
   caatgggcgt ggatagcggt ttgactcacg gggatttcca agtctccacc ccattgacgt 600
   caatgggagt ttgttttggc accaaaatca acgggacttt ccaaaatgtc gtaataaccc 660
   cgccccgttg acgcaaatgg gcggtaggcg tgtacggtgg gaggtctata taagcagagc 720
   tcgtttagtg aaccgtcaga tcactagaag ctttattgcg gtagtttatc acagttaaat 780
   tgctaacgca gtcagtgctt ctgacacaac agtctcgaac ttaagctgca gaagttggtc 840
   gtgaggcact gggcaggtaa gtatcaaggt tacaagacag gtttaaggag accaatagaa 900
   actgggcttg tcgagacaga gaagactctt gcgtttctga taggcaccta ttggtcttac 960
   tgacatccac tttgcctttc tctccacagg tgtccactcc cagttcaatt acagctctta 1020
   aggctagagt acttaatacg actcactata ggctaggggt accggtcgcc accatggtga 1080
   gcaagggcga ggagctgttc accggggtgg tgcccatcct ggtcgagctg gacggcgacg 1140
   taaacggcca caagttcagc gtgtccggcg agggcgaggg cgatgccacc tacggcaagc 1200
   tgaccctgaa gttcatctgc accaccggca agctgcccgt gccctggccc accctcgtga 1260
   ccaccctgac ctacggcgtg cagtgcttca gccgctaccc cgaccacatg aagcagcacg 1320
   acttcttcaa gtccgccatg cccgaaggct acgtccagga gcgcaccatc ttcttcaagg 1380
   acgacggcaa ctacaagacc cgcgccgagg tgaagttcga gggcgacacc ctggtgaacc 1440
   gcatcgagct gaagggcatc gacttcaagg aggacggcaa catcctgggg cacaagctgg 1500
   agtacaacta caacagccac aacgtctata tcatggccga caagcagaag aacggcatca 1560
   aggtgaactt caagatccgc cacaacatcg aggacggcag cgtgcagctc gccgaccact 1620
   accagcagaa cacccccatc ggcgacggcc ccgtgctgct gcccgacaac cactacctga 1680
   gcacccagtc cgccctgagc aaagacccca acgagaagcg cgatcacatg gtcctgctgg 1740
   agttcgtgac cgccgccggg atcactctcg gcatggacga gctgtacaag taaagcggcc 1800
   gcttcgagca gacatgataa gatacattga tgagtttgga caaaccacaa ctagaatgca 1860
   gtgaaaaaaa tgctttattt gtgaaatttg tgatgctatt gctttatttg taaccattat 1920
   aagctgcaat aaacaagtta acaacaacaa ttgcattcat tttatgtttc aggttcaggg 1980
   ggagatgtgg gaggtttttt aaagcaagta aaacctctac aaatgtggta aaatcgataa 2040
   ggatcttcaa tattggccat tagccatatt attcattggt tatatagcat aaatcaatat 2100
   tggctattgg ccattgcata cgttgtatct atatcataat atgtacattt atattggctc 2160
   atgtccaata tgaccgccat gttggcattg attattgact agttattaat agtaatcaat 2220
   tacggggtca ttagttcata gcccatatat ggagttccgc gttacataac ttacggtaaa 2280
   tggcccgcct ggctgaccgc ccaacgaccc ccgcccattg acgtcaataa tgacgtatgt 2340
   tcccatagta acgccaatag ggactttcca ttgacgtcaa tgggtggagt atttacggta 2400
   aactgcccac ttggcagtac atcaagtgta tcatatgcca agtccgcccc ctattgacgt 2460
   caatgacggt aaatggcccg cctggcatta tgcccagtac atgaccttac gggactttcc 2520
   tacttggcag tacatctacg tattagtcat cgctattacc atggtgatgc ggttttggca 2580
   gtacaccaat gggcgtggat agcggtttga ctcacgggga tttccaagtc tccaccccat 2640
   tgacgtcaat gggagtttgt tttggcacca aaatcaacgg gactttccaa aatgtcgtaa 2700
   taaccccgcc ccgttgacgc aaatgggcgg taggcgtgta cggtgggagg tctatataag 2760
   cagagctcgt ttagtgaacc gtcagatcac tagaagcttt attgcggtag tttatcacag 2820
   ttaaattgct aacgcagtca gtgcttctga cacaacagtc tcgaacttaa gctgcagaag 2880
   ttggtcgtga ggcactgggc aggtaagtat caaggttaca agacaggttt aaggagacca 2940
   atagaaactg ggcttgtcga gacagagaag actcttgcgt ttctgatagg cacctattgg 3000
   tcttactgac atccactttg cctttctctc cacaggtgtc cactcccagt tcaattacag 3060
   ctcttaaggc tagagtactt aatacgactc actataggct agtcgccacc atggtgagcg 3120
   gcctgctgaa ggagagtatg cgcatcaaga tgtacatgga gggcaccgtg aacggccact 3180
   acttcaagtg cgagggcgag ggcgacggca accccttcgc cggcacccag agcatgagaa 3240
   tccacgtgac cgagggcgcc cccctgccct tcgccttcga catcctggcc ccctgctgcg 3300
   agtacggcag caggaccttc gtgcaccaca ccgccgagat ccccgacttc ttcaagcaga 3360
   gcttccccga gggcttcacc tgggagagaa ccaccaccta cgaggacggc ggcatcctga 3420
   ccgcccacca ggacaccagc ctggagggca actgcctgat ctacaaggtg aaggtgcacg 3480
   gcaccaactt ccccgccgac ggccccgtga tgaagaacaa gagcggcggc tgggagccca 3540
   gcaccgaggt ggtgtacccc gagaacggcg tgctgtgcgg ccggaacgtg atggccctga 3600
   aggtgggcga ccggcacctg atctgccacc actacaccag ctaccggagc aagaaggccg 3660
   tgcgcgccct gaccatgccc ggcttccact tcaccgacat ccggctccag atgctgcgga 3720
   agaagaagga cgagtacttc gagctgtacg aggccagcgt ggcccggtac agcgacctgc 3780
   ccgagaaggc caactgaagc ggccgcttcg agcagacatg ataagataca ttgatgagtt 3840
   tggacaaacc acaactagaa tgcagtgaaa aaaatgcttt atttgtgaaa tttgtgatgc 3900
   tattgcttta tttgtaacca ttataagctg caataaacaa gttaacaaca acaattgcat 3960
   tcattttatg tttcaggttc agggggagat gtgggaggtt ttttaaagca agtaaaacct 4020
   ctacaaatgt ggtaaaatcg ataaggatcc gggctggcgt aatagcgaag aggcccgcac 4080
   cgatcgccct tcccaacagt tgcgcagcct gaatggcgaa tggacgcgcc ctgtagcggc 4140
   gcattaagcg cggcgggtgt ggtggttacg cgcagcgtga ccgctacact tgccagcgcc 4200
   ctagcgcccg ctcctttcgc tttcttccct tcctttctcg ccacgttcgc cggctttccc 4260
   cgtcaagctc taaatcgggg gctcccttta gggttccgat ttagagcttt acggcacctc 4320
   gaccgcaaaa aacttgattt gggtgatggt tcacgtagtg ggccatcgcc ctgatagacg 4380
   gtttttcgcc ctttgacgtt ggagtccacg ttctttaata gtggactctt gttccaaact 4440
   ggaacaacac tcaaccctat ctcggtctat tcttttgatt tataagggat tttgccgatt 4500
   tcggcctatt ggttaaaaaa tgagctgatt taacaaatat ttaacgcgaa ttttaacaaa 4560
   atattaacgt ttacaatttc gcctgatgcg gtattttctc cttacgcatc tgtgcggtat 4620
   ttcacaccgc atatggtgca ctctcagtac aatctgctct gatgccgcat agttaagcca 4680
   gccccgacac ccgccaacac ccgctgacgc gccctgacgg gcttgtctgc tcccggcatc 4740
   cgcttacaga caagctgtga ccgtctccgg gagctgcatg tgtcagaggt tttcaccgtc 4800
   atcaccgaaa cgcgcgagac gaaagggcct cgtgatacgc ctatttttat aggttaatgt 4860
   catgataata atggtttctt agacgtcagg tggcactttt cggggaaatg tgcgcggaac 4920
   ccctatttgt ttatttttct aaatacattc aaatatgtat ccgctcatga gacaataacc 4980
   ctgataaatg cttcaataat attgaaaaag gaagagtatg agtattcaac atttccgtgt 5040
   cgcccttatt cccttttttg cggcattttg ccttcctgtt tttgctcacc cagaaacgct 5100
   ggtgaaagta aaagatgctg aagatcagtt gggtgcacga gtgggttaca tcgaactgga 5160
   tctcaacagc ggtaagatcc ttgagagttt tcgccccgaa gaacgttttc caatgatgag 5220
   cacttttaaa gttctgctat gtggcgcggt attatcccgt attgacgccg ggcaagagca 5280
   actcggtcgc cgcatacact attctcagaa tgacttggtt gagtactcac cagtcacaga 5340
   aaagcatctt acggatggca tgacagtaag agaattatgc agtgctgcca taaccatgag 5400
   tgataacact gcggccaact tacttctgac aacgatcgga ggaccgaagg agctaaccgc 5460
   ttttttgcac aacatggggg atcatgtaac tcgccttgat cgttgggaac cggagctgaa 5520
   tgaagccata ccaaacgacg agcgtgacac cacgatgcct gtagcaatgg caacaacgtt 5580
   gcgcaaacta ttaactggcg aactacttac tctagcttcc cggcaacaat taatagactg 5640
   gatggaggcg gataaagttg caggaccact tctgcgctcg gcccttccgg ctggctggtt 5700
   tattgctgat aaatctggag ccggtgagcg tgggtctcgc ggtatcattg cagcactggg 5760
   gccagatggt aagccctccc gtatcgtagt tatctacacg acggggagtc aggcaactat 5820
   ggatgaacga aatagacaga tcgctgagat aggtgcctca ctgattaagc attggtaact 5880
   gtcagaccaa gtttactcat atatacttta gattgattta aaacttcatt tttaatttaa 5940
   aaggatctag gtgaagatcc tttttgataa tctcatgacc aaaatccctt aacgtgagtt 6000
   ttcgttccac tgagcgtcag accccgtaga aaagatcaaa ggatcttctt gagatccttt 6060
   ttttctgcgc gtaatctgct gcttgcaaac aaaaaaacca ccgctaccag cggtggtttg 6120
   tttgccggat caagagctac caactctttt tccgaaggta actggcttca gcagagcgca 6180
   gataccaaat actgtccttc tagtgtagcc gtagttaggc caccacttca agaactctgt 6240
   agcaccgcct acatacctcg ctctgctaat cctgttacca gtggctgctg ccagtggcga 6300
   taagtcgtgt cttaccgggt tggactcaag acgatagtta ccggataagg cgcagcggtc 6360
   gggctgaacg gggggttcgt gcacacagcc cagcttggag cgaacgacct acaccgaact 6420
   gagataccta cagcgtgagc tatgagaaag cgccacgctt cccgaaggga gaaaggcgga 6480
   caggtatccg gtaagcggca gggtcggaac aggagagcgc acgagggagc ttccaggggg 6540
   aaacgcctgg tatctttata gtcctgtcgg gtttcgccac ctctgacttg agcgtcgatt 6600
   tttgtgatgc tcgtcagggg ggcggagcct atggaaaaac gccagcaacg cggccttttt 6660
   acggttcctg gccttttgct ggccttttgc tcacatggct cgacagatct 6710
<210> 5
   <211> 8569
   <212> DNA
   <213> Artificial Sequence
<220>
   <223> Synthetic construct
<400> 5
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac atgattacga attcgagctc 240
   ggtaccagat ctgagctccc acctccctct ctgtgctggg actcacagag ggagacctca 300
   ggaggcagtc tgtccatcac atgtccaaat gcagagcata ccctgggctg ggcgcagtgg 360
   cgcacaactg taattccagc actttgggag gctgatgtgg aaggatcact tgagcccaga 420
   agttctagac cagcctgggc aacatggcaa gaccctatct ctacaaaaaa agttaaaaaa 480
   tcagccacgt gtggtgacac acacctgtag tcccagctat tcaggaggct gaggtgaggg 540
   gatcacttaa ggctgggagg ttgaggctgc agtgagtcgt ggttgcgcca ctgcactcca 600
   gcctgggcaa cagtgagacc ctgtctcaaa agacaaaaaa aaaaaaaaaa aaaaaaagaa 660
   catatcctgg tgtggagtag gggacgctgc tctgacagag gctcgggggc ctgagctggc 720
   tctgtgagct ggggaggagg cagacagcca ggccttgtct gcaagcagac ctggcagcat 780
   tgggctggcc gccccccagg gcctcctctt catgcccagt gaatgactca ccttggcaca 840
   gacacaatgt tcggggtggg cacagtgcct gcttcccgcc gcaccccagc ccccctcaaa 900
   tgccttccga gaagcccatt gagcaggggg cttgcattgc accccagcct gacagcctgg 960
   catcttggga taaaagcagc acagccccct aggggctgcc cttgctgtgt ggcgccaccg 1020
   gcggtggaga acaaggctct attcagcctg tgcccaggaa aggggatcag gggatgccca 1080
   ggcatggaca gtgggtggca gggggggaga ggagggctgt ctgcttccca gaagtccaag 1140
   gacacaaatg ggtgagggga ctgggcaggg ttctgaccct gtgggaccag agtggagggc 1200
   gtagatggac ctgaagtctc cagggacaac agggcccagg tctcaggctc ctagttgggc 1260
   ccagtggctc cagcgtttcc aaacccatcc atccccagag gttcttccca tctctccagg 1320
   ctgatgtgtg ggaactcgag gaaataaatc tccagtggga gacggagggg tggccaggga 1380
   aacggggcgc tgcaggaata aagacgagcc agcacagcca gctcatgtgt aacggctttg 1440
   tggagctgtc aaggcctggt ctctgggaga gaggcacagg gaggccagac aaggaagggg 1500
   tgacctggag ggacagatcc aggggctaaa gtcctgataa ggcaagagag tgccggcccc 1560
   ctcttgccct atcaggacct ccactgccac atagaggcca tgattgaccc ttagacaaag 1620
   ggctggtgtc caatcccagc ccccagcccc agaactccag ggaatgaatg ggcagagagc 1680
   aggaatgtgg gacatctgtg ttcaagggaa ggactccagg agtctgctgg gaatgaggcc 1740
   tagtaggaaa tgaggtggcc cttgagggta cagaacaggt tcattcttcg ccaaattccc 1800
   agcaccttgc aggcacttac agctgagtga gataatgcct gggttatgaa atcaaaaagt 1860
   tggaaagcag gtcagaggtc atctggtaca gcccttcctt cccttttttt tttttttttt 1920
   ttgtgagaca aggtctctct ctgttgccca ggctggagtg gcgcaaacac agctcactgc 1980
   agcctcaacc tactgggctc aagcaatcct ccagcctcag cctcccaaag tgctgggatt 2040
   acaagcatga gccaccccac tcagcccttt ccttcctttt taattgatgc ataataattg 2100
   taagtattca tcatggtcca accaaccctt tcttgaccca ccttcctaga gagagggtcc 2160
   tcttgcttca gcggtcaggg ccccagaccc atggtctggc tccaggtacc acctgcctca 2220
   tgcaggagtt ggcgtgccca ggaagctctg cctctgggca cagtgacctc agtggggtga 2280
   ggggagctct ccccatagct gggctgcggc ccaaccccac cccctcaggc tatgccaggg 2340
   ggtgttgcca ggggcacccg ggcatcgcca gtctagccca ctccttcata aagccctcgc 2400
   atcccaggag cgagcagagc cagagcaggt tggagaggag acgcatcacc tccgctgctc 2460
   gcggggatcc tctagagtcg acggatccgg ggaattcccc agtctcagga tccaccatgg 2520
   gggatcccgt cgttttacaa cgtcgtgact gggaaaaccc tggcgttacc caacttaatc 2580
   gccttgcagc acatccccct ttcgccagct ggcgtaatag cgaagaggcc cgcaccgatc 2640
   gcccttccca acagttgcgc agcctgaatg gcgaatggcg ctttgcctgg tttccggcac 2700
   cagaagcggt gccggaaagc tggctggagt gcgatcttcc tgaggccgat actgtcgtcg 2760
   tcccctcaaa ctggcagatg cacggttacg atgcgcccat ctacaccaac gtaacctatc 2820
   ccattacggt caatccgccg tttgttccca cggagaatcc gacgggttgt tactcgctca 2880
   catttaatgt tgatgaaagc tggctacagg aaggccagac gcgaattatt tttgatggcg 2940
   ttaactcggc gtttcatctg tggtgcaacg ggcgctgggt cggttacggc caggacagtc 3000
   gtttgccgtc tgaatttgac ctgagcgcat ttttacgcgc cggagaaaac cgcctcgcgg 3060
   tgatggtgct gcgttggagt gacggcagtt atctggaaga tcaggatatg tggcggatga 3120
   gcggcatttt ccgtgacgtc tcgttgctgc ataaaccgac tacacaaatc agcgatttcc 3180
   atgttgccac tcgctttaat gatgatttca gccgcgctgt actggaggct gaagttcaga 3240
   tgtgcggcga gttgcgtgac tacctacggg taacagtttc tttatggcag ggtgaaacgc 3300
   aggtcgccag cggcaccgcg cctttcggcg gtgaaattat cgatgagcgt ggtggttatg 3360
   ccgatcgcgt cacactacgt ctgaacgtcg aaaacccgaa actgtggagc gccgaaatcc 3420
   cgaatctcta tcgtgcggtg gttgaactgc acaccgccga cggcacgctg attgaagcag 3480
   aagcctgcga tgtcggtttc cgcgaggtgc ggattgaaaa tggtctgctg ctgctgaacg 3540
   gcaagccgtt gctgattcga ggcgttaacc gtcacgagca tcatcctctg catggtcagg 3600
   tcatggatga gcagacgatg gtgcaggata tcctgctgat gaagcagaac aactttaacg 3660
   ccgtgcgctg ttcgcattat ccgaaccatc cgctgtggta cacgctgtgc gaccgctacg 3720
   gcctgtatgt ggtggatgaa gccaatattg aaacccacgg catggtgcca atgaatcgtc 3780
   tgaccgatga tccgcgctgg ctaccggcga tgagcgaacg cgtaacgcga atggtgcagc 3840
   gcgatcgtaa tcacccgagt gtgatcatct ggtcgctggg gaatgaatca ggccacggcg 3900
   ctaatcacga cgcgctgtat cgctggatca aatctgtcga tccttcccgc ccggtgcagt 3960
   atgaaggcgg cggagccgac accacggcca ccgatattat ttgcccgatg tacgcgcgcg 4020
   tggatgaaga ccagcccttc ccggctgtgc cgaaatggtc catcaaaaaa tggctttcgc 4080
   tacctggaga gacgcgcccg ctgatccttt gcgaatacgc ccacgcgatg ggtaacagtc 4140
   ttggcggttt cgctaaatac tggcaggcgt ttcgtcagta tccccgttta cagggcggct 4200
   tcgtctggga ctgggtggat cagtcgctga ttaaatatga tgaaaacggc aacccgtggt 4260
   cggcttacgg cggtgatttt ggcgatacgc cgaacgatcg ccagttctgt atgaacggtc 4320
   tggtctttgc cgaccgcacg ccgcatccag cgctgacgga agcaaaacac cagcagcagt 4380
   ttttccagtt ccgtttatcc gggcaaacca tcgaagtgac cagcgaatac ctgttccgtc 4440
   atagcgataa cgagctcctg cactggatgg tggcgctgga tggtaagccg ctggcaagcg 4500
   gtgaagtgcc tctggatgtc gctccacaag gtaaacagtt gattgaactg cctgaactac 4560
   cgcagccgga gagcgccggg caactctggc tcacagtacg cgtagtgcaa ccgaacgcga 4620
   ccgcatggtc agaagccggg cacatcagcg cctggcagca gtggcgtctg gcggaaaacc 4680
   tcagtgtgac gctccccgcc gcgtcccacg ccatcccgca tctgaccacc agcgaaatgg 4740
   atttttgcat cgagctgggt aataagcgtt ggcaatttaa ccgccagtca ggctttcttt 4800
   cacagatgtg gattggcgat aaaaaacaac tgctgacgcc gctgcgcgat cagttcaccc 4860
   gtgcaccgct ggataacgac attggcgtaa gtgaagcgac ccgcattgac cctaacgcct 4920
   gggtcgaacg ctggaaggcg gcgggccatt accaggccga agcagcgttg ttgcagtgca 4980
   cggcagatac acttgctgat gcggtgctga ttacgaccgc tcacgcgtgg cagcatcagg 5040
   ggaaaacctt atttatcagc cggaaaacct accggattga tggtagtggt caaatggcga 5100
   ttaccgttga tgttgaagtg gcgagcgata caccgcatcc ggcgcggatt ggcctgaact 5160
   gccagctggc gcaggtagca gagcgggtaa actggctcgg attagggccg caagaaaact 5220
   atcccgaccg ccttactgcc gcctgttttg accgctggga tctgccattg tcagacatgt 5280
   ataccccgta cgtcttcccg agcgaaaacg gtctgcgctg cgggacgcgc gaattgaatt 5340
   atggcccaca ccagtggcgc ggcgacttcc agttcaacat cagccgctac agtcaacagc 5400
   aactgatgga aaccagccat cgccatctgc tgcacgcgga agaaggcaca tggctgaata 5460
   tcgacggttt ccatatgggg attggtggcg acgactcctg gagcccgtca gtatcggcgg 5520
   aattccagct gagcgccggt cgctaccatt accagttggt ctggtgtcaa aaataataat 5580
   aaccgggcag gggggatccg cagatcccgg ccagataccg atgctgccgc agcaaaagca 5640
   ggagcagatg ccgccgtcgc aggcgaagat gtcgcagacg gaggaggcga tgctgccggc 5700
   ggaggaggcg aagtaagtag agggctgggc tgggctgtgg ggggtgtggg gtgcgggact 5760
   gggcagtctg ggagtccctc tcaccacttt tcttaccttt ctaggatgct gccgtcgccg 5820
   ccgctcatac accataaggt gtaaaaaata ctagatgcac agaatagcaa gtccatcaaa 5880
   actcctgcgt gagaatttta ccagacttca agagcatctc gccacatctt gaaaaatgcc 5940
   accgtccgat gaaaaacagg agcctgctaa ggaacaatgc cacctgtcaa taaatgttga 6000
   aaactcatcc cattcctgcc tcttggtcct tgggcttggg gaggggtgcg cggatgtggt 6060
   tagggaacat gactggtcaa atgggaaggg cttcaaaaga attcccaata ttgactacca 6120
   agccacctgt acagatcgaa ttcagatctg cctgcaggca tgcaagcttg gcactggccg 6180
   tcgttttaca acgtcgtgac tgggaaaacc ctggcgttac ccaacttaat cgccttgcag 6240
   cacatccccc tttcgccagc tggcgtaata gcgaagaggc ccgcaccgat cgcccttccc 6300
   aacagttgcg cagcctgaat ggcgaatggc gcctgatgcg gtattttctc cttacgcatc 6360
   tgtgcggtat ttcacaccgc atatggtgca ctctcagtac aatctgctct gatgccgcat 6420
   agttaagcca gccccgacac ccgccaacac ccgctgacgc gccctgacgg gcttgtctgc 6480
   tcccggcatc cgcttacaga caagctgtga ccgtctccgg gagctgcatg tgtcagaggt 6540
   tttcaccgtc atcaccgaaa cgcgcgagac gaaagggcct cgtgatacgc ctatttttat 6600
   aggttaatgt catgataata atggtttctt agacgtcagg tggcactttt cggggaaatg 6660
   tgcgcggaac ccctatttgt ttatttttct aaatacattc aaatatgtat ccgctcatga 6720
   gacaataacc ctgataaatg cttcaataat attgaaaaag gaagagtatg agtattcaac 6780
   atttccgtgt cgcccttatt cccttttttg cggcattttg ccttcctgtt tttgctcacc 6840
   cagaaacgct ggtgaaagta aaagatgctg aagatcagtt gggtgcacga gtgggttaca 6900
   tcgaactgga tctcaacagc ggtaagatcc ttgagagttt tcgccccgaa gaacgttttc 6960
   caatgatgag cacttttaaa gttctgctat gtggcgcggt attatcccgt attgacgccg 7020
   ggcaagagca actcggtcgc cgcatacact attctcagaa tgacttggtt gagtactcac 7080
   cagtcacaga aaagcatctt acggatggca tgacagtaag agaattatgc agtgctgcca 7140
   taaccatgag tgataacact gcggccaact tacttctgac aacgatcgga ggaccgaagg 7200
   agctaaccgc ttttttgcac aacatggggg atcatgtaac tcgccttgat cgttgggaac 7260
   cggagctgaa tgaagccata ccaaacgacg agcgtgacac cacgatgcct gtagcaatgg 7320
   caacaacgtt gcgcaaacta ttaactggcg aactacttac tctagcttcc cggcaacaat 7380
   taatagactg gatggaggcg gataaagttg caggaccact tctgcgctcg gcccttccgg 7440
   ctggctggtt tattgctgat aaatctggag ccggtgagcg tgggtctcgc ggtatcattg 7500
   cagcactggg gccagatggt aagccctccc gtatcgtagt tatctacacg acggggagtc 7560
   aggcaactat ggatgaacga aatagacaga tcgctgagat aggtgcctca ctgattaagc 7620
   attggtaact gtcagaccaa gtttactcat atatacttta gattgattta aaacttcatt 7680
   tttaatttaa aaggatctag gtgaagatcc tttttgataa tctcatgacc aaaatccctt 7740
   aacgtgagtt ttcgttccac tgagcgtcag accccgtaga aaagatcaaa ggatcttctt 7800
   gagatccttt ttttctgcgc gtaatctgct gcttgcaaac aaaaaaacca ccgctaccag 7860
   cggtggtttg tttgccggat caagagctac caactctttt tccgaaggta actggcttca 7920
   gcagagcgca gataccaaat actgtccttc tagtgtagcc gtagttaggc caccacttca 7980
   agaactctgt agcaccgcct acatacctcg ctctgctaat cctgttacca gtggctgctg 8040
   ccagtggcga taagtcgtgt cttaccgggt tggactcaag acgatagtta ccggataagg 8100
   cgcagcggtc gggctgaacg gggggttcgt gcacacagcc cagcttggag cgaacgacct 8160
   acaccgaact gagataccta cagcgtgagc tatgagaaag cgccacgctt cccgaaggga 8220
   gaaaggcgga caggtatccg gtaagcggca gggtcggaac aggagagcgc acgagggagc 8280
   ttccaggggg aaacgcctgg tatctttata gtcctgtcgg gtttcgccac ctctgacttg 8340
   agcgtcgatt tttgtgatgc tcgtcagggg ggcggagcct atggaaaaac gccagcaacg 8400
   cggccttttt acggttcctg gccttttgct ggccttttgc tcacatgttc tttcctgcgt 8460
   tatcccctga ttctgtggat aaccgtatta ccgcctttga gtgagctgat accgctcgcc 8520
   gcagccgaac gaccgagcgc agcgagtcag tgagcgagga agcggaaga             8569
<210> 6
   <211> 5346
   <212> DNA
   <213> Artificial Sequence
<220>
   <223> Synthetic construct
<400> 6
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac atgattacga attcgagctc 240
   ggtaccagat ctgagctccc acctccctct ctgtgctggg actcacagag ggagacctca 300
   ggaggcagtc tgtccatcac atgtccaaat gcagagcata ccctgggctg ggcgcagtgg 360
   cgcacaactg taattccagc actttgggag gctgatgtgg aaggatcact tgagcccaga 420
   agttctagac cagcctgggc aacatggcaa gaccctatct ctacaaaaaa agttaaaaaa 480
   tcagccacgt gtggtgacac acacctgtag tcccagctat tcaggaggct gaggtgaggg 540
   gatcacttaa ggctgggagg ttgaggctgc agtgagtcgt ggttgcgcca ctgcactcca 600
   gcctgggcaa cagtgagacc ctgtctcaaa agacaaaaaa aaaaaaaaaa aaaaaaagaa 660
   catatcctgg tgtggagtag gggacgctgc tctgacagag gctcgggggc ctgagctggc 720
   tctgtgagct ggggaggagg cagacagcca ggccttgtct gcaagcagac ctggcagcat 780
   tgggctggcc gccccccagg gcctcctctt catgcccagt gaatgactca ccttggcaca 840
   gacacaatgt tcggggtggg cacagtgcct gcttcccgcc gcaccccagc ccccctcaaa 900
   tgccttccga gaagcccatt gagcaggggg cttgcattgc accccagcct gacagcctgg 960
   catcttggga taaaagcagc acagccccct aggggctgcc cttgctgtgt ggcgccaccg 1020
   gcggtggaga acaaggctct attcagcctg tgcccaggaa aggggatcag gggatgccca 1080
   ggcatggaca gtgggtggca gggggggaga ggagggctgt ctgcttccca gaagtccaag 1140
   gacacaaatg ggtgagggga ctgggcaggg ttctgaccct gtgggaccag agtggagggc 1200
   gtagatggac ctgaagtctc cagggacaac agggcccagg tctcaggctc ctagttgggc 1260
   ccagtggctc cagcgtttcc aaacccatcc atccccagag gttcttccca tctctccagg 1320
   ctgatgtgtg ggaactcgag gaaataaatc tccagtggga gacggagggg tggccaggga 1380
   aacggggcgc tgcaggaata aagacgagcc agcacagcca gctcatgtgt aacggctttg 1440
   tggagctgtc aaggcctggt ctctgggaga gaggcacagg gaggccagac aaggaagggg 1500
   tgacctggag ggacagatcc aggggctaaa gtcctgataa ggcaagagag tgccggcccc 1560
   ctcttgccct atcaggacct ccactgccac atagaggcca tgattgaccc ttagacaaag 1620
   ggctggtgtc caatcccagc cccagccccc agaactccag ggaatgaatg ggcagagagc 1680
   aggaatgtgg gacatctgtg ttcaagggaa ggactccagg agtctgctgg gaatgaggcc 1740
   tagtaggaaa tgaggtggcc cttgagggta cagaacaggt tcattcttcg ccaaattccc 1800
   agcaccttgc aggcacttac agctgagtga gataatgcct gggttatgaa atcaaaaagt 1860
   tggaaagcag gtcagaggtc atctggtaca gcccttcctt cccttttttt tttttttttt 1920
   ttgtgagaca aggtctctct ctgttgccca ggctggagtg gcgcaaacac agctcactgc 1980
   agcctcaacc tactgggctc aagcaatcct ccagcctcag cctcccaaag tgctgggatt 2040
   acaagcatga gccaccccac tcagcccttt ccttcctttt taattgatgc ataataattg 2100
   taagtattca tcatggtcca accaaccctt tcttgaccca ccttcctaga gagagggtcc 2160
   tcttgcttca gcggtcaggg ccccagaccc atggtctggc tccaggtacc acctgcctca 2220
   taaaaaacag cacaaaagga aactcaccct aactgtaaag taattgtgtg ttttgagact 2280
   ataaatatcc cttggagaaa agccttgttt gggccccccc tcgaggtcga cggtatcgat 2340
   aagcttgata tcgaattcct gcagcccggg ggatccgcag atcccggcca gataccgatg 2400
   ctgccgcagc aaaagcagga gcagatgccg ccgtcgcagg cgaagatgtc gcagacggag 2460
   gaggcgatgc tgccggcgga ggaggcgaag taagtagagg gctgggctgg gctgtggggg 2520
   gtgtggggtg cgggactggg cagtctggga gtccctctca ccacttttct tacctttcta 2580
   ggatgctgcc gtcgccgccg ctcatacacc ataaggtgta aaaaatacta gatgcacaga 2640
   atagcaagtc catcaaaact cctgcgtgag aattttacca gacttcaaga gcatctcgcc 2700
   acatcttgaa aaatgccacc gtccgatgaa aaacaggagc ctgctaagga acaatgccac 2760
   ctgtcaataa atgttgaaaa ctcatcccat tcctgcctct tggtccttgg gcttggggag 2820
   gggtgcgcgg atgtggttag ggaacatgac tggtcaaatg ggaagggctt caaaagaatt 2880
   cccaatattg actaccaagc cacctgtaca gatcgaattc agatctgcct gcaggcatgc 2940
   aagcttggca ctggccgtcg ttttacaacg tcgtgactgg gaaaaccctg gcgttaccca 3000
   acttaatcgc cttgcagcac atcccccttt cgccagctgg cgtaatagcg aagaggcccg 3060
   caccgatcgc ccttcccaac agttgcgcag cctgaatggc gaatggcgcc tgatgcggta 3120
   ttttctcctt acgcatctgt gcggtatttc acaccgcata tggtgcactc tcagtacaat 3180
   ctgctctgat gccgcatagt taagccagcc ccgacacccg ccaacacccg ctgacgcgcc 3240
   ctgacgggct tgtctgctcc cggcatccgc ttacagacaa gctgtgaccg tctccgggag 3300
   ctgcatgtgt cagaggtttt caccgtcatc accgaaacgc gcgagacgaa agggcctcgt 3360
   gatacgccta tttttatagg ttaatgtcat gataataatg gtttcttaga cgtcaggtgg 3420
   cacttttcgg ggaaatgtgc gcggaacccc tatttgttta tttttctaaa tacattcaaa 3480
   tatgtatccg ctcatgagac aataaccctg ataaatgctt caataatatt gaaaaaggaa 3540
   gagtatgagt attcaacatt tccgtgtcgc ccttattccc ttttttgcgg cattttgcct 3600
   tcctgttttt gctcacccag aaacgctggt gaaagtaaaa gatgctgaag atcagttggg 3660
   tgcacgagtg ggttacatcg aactggatct caacagcggt aagatccttg agagttttcg 3720
   ccccgaagaa cgttttccaa tgatgagcac ttttaaagtt ctgctatgtg gcgcggtatt 3780
   atcccgtatt gacgccgggc aagagcaact cggtcgccgc atacactatt ctcagaatga 3840
   cttggttgag tactcaccag tcacagaaaa gcatcttacg gatggcatga cagtaagaga 3900
   attatgcagt gctgccataa ccatgagtga taacactgcg gccaacttac ttctgacaac 3960
   gatcggagga ccgaaggagc taaccgcttt tttgcacaac atgggggatc atgtaactcg 4020
   ccttgatcgt tgggaaccgg agctgaatga agccatacca aacgacgagc gtgacaccac 4080
   gatgcctgta gcaatggcaa caacgttgcg caaactatta actggcgaac tacttactct 4140
   agcttcccgg caacaattaa tagactggat ggaggcggat aaagttgcag gaccacttct 4200
   gcgctcggcc cttccggctg gctggtttat tgctgataaa tctggagccg gtgagcgtgg 4260
   gtctcgcggt atcattgcag cactggggcc agatggtaag ccctcccgta tcgtagttat 4320
   ctacacgacg gggagtcagg caactatgga tgaacgaaat agacagatcg ctgagatagg 4380
   tgcctcactg attaagcatt ggtaactgtc agaccaagtt tactcatata tactttagat 4440
   tgatttaaaa cttcattttt aatttaaaag gatctaggtg aagatccttt ttgataatct 4500
   catgaccaaa atcccttaac gtgagttttc gttccactga gcgtcagacc ccgtagaaaa 4560
   gatcaaagga tcttcttgag atcctttttt tctgcgcgta atctgctgct tgcaaacaaa 4620
   aaaaccaccg ctaccagcgg tggtttgttt gccggatcaa gagctaccaa ctctttttcc 4680
   gaaggtaact ggcttcagca gagcgcagat accaaatact gtccttctag tgtagccgta 4740
   gttaggccac cacttcaaga actctgtagc accgcctaca tacctcgctc tgctaatcct 4800
   gttaccagtg gctgctgcca gtggcgataa gtcgtgtctt accgggttgg actcaagacg 4860
   atagttaccg gataaggcgc agcggtcggg ctgaacgggg ggttcgtgca cacagcccag 4920
   cttggagcga acgacctaca ccgaactgag atacctacag cgtgagctat gagaaagcgc 4980
   cacgcttccc gaagggagaa aggcggacag gtatccggta agcggcaggg tcggaacagg 5040
   agagcgcacg agggagcttc cagggggaaa cgcctggtat ctttatagtc ctgtcgggtt 5100
   tcgccacctc tgacttgagc gtcgattttt gtgatgctcg tcaggggggc ggagcctatg 5160
   gaaaaacgcc agcaacgcgg cctttttacg gttcctggcc ttttgctggc cttttgctca 5220
   catgttcttt cctgcgttat cccctgattc tgtggataac cgtattaccg cctttgagtg 5280
   agctgatacc gctcgccgca gccgaacgac cgagcgcagc gagtcagtga gcgaggaagc 5340
   ggaaga 5346
<210> 7
   <211> 5485
   <212> DNA
   <213> Artificial Sequence
<220>
   <223> Synthetic construct
<400> 7
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac atgattacga attcgagctc 240
   ggtaccagat ctgagctccc acctccctct ctgtgctggg actcacagag ggagacctca 300
   ggaggcagtc tgtccatcac atgtccaaat gcagagcata ccctgggctg ggcgcagtgg 360
   cgcacaactg taattccagc actttgggag gctgatgtgg aaggatcact tgagcccaga 420
   agttctagac cagcctgggc aacatggcaa gaccctatct ctacaaaaaa agttaaaaaa 480
   tcagccacgt gtggtgacac acacctgtag tcccagctat tcaggaggct gaggtgaggg 540
   gatcacttaa ggctgggagg ttgaggctgc agtgagtcgt ggttgcgcca ctgcactcca 600
   gcctgggcaa cagtgagacc ctgtctcaaa agacaaaaaa aaaaaaaaaa aaaaaaagaa 660
   catatcctgg tgtggagtag gggacgctgc tctgacagag gctcgggggc ctgagctggc 720
   tctgtgagct ggggaggagg cagacagcca ggccttgtct gcaagcagac ctggcagcat 780
   tgggctggcc gccccccagg gcctcctctt catgcccagt gaatgactca ccttggcaca 840
   gacacaatgt tcggggtggg cacagtgcct gcttcccgcc gcaccccagc ccccctcaaa 900
   tgccttccga gaagcccatt gagcaggggg cttgcattgc accccagcct gacagcctgg 960
   catcttggga taaaagcagc acagccccct aggggctgcc cttgctgtgt ggcgccaccg 1020
   gcggtggaga acaaggctct attcagcctg tgcccaggaa aggggatcag gggatgccca 1080
   ggcatggaca gtgggtggca gggggggaga ggagggctgt ctgcttccca gaagtccaag 1140
   gacacaaatg ggtgagggga ctgggcaggg ttctgaccct gtgggaccag agtggagggc 1200
   gtagatggac ctgaagtctc cagggacaac agggcccagg tctcaggctc ctagttgggc 1260
   ccagtggctc cagcgtttcc aaacccatcc atccccagag gttcttccca tctctccagg 1320
   ctgatgtgtg ggaactcgag gaaataaatc tccagtggga gacggagggg tggccaggga 1380
   aacggggcgc tgcaggaata aagacgagcc agcacagcca gctcatgtgt aacggctttg 1440
   tggagctgtc aaggcctggt ctctgggaga gaggcacagg gaggccagac aaggaagggg 1500
   tgacctggag ggacagatcc aggggctaaa gtcctgataa ggcaagagag tgccggcccc 1560
   ctcttgccct atcaggacct ccactgccac atagaggcca tgattgaccc ttagacaaag 1620
   ggctggtgtc caatcccagc ccccagcccc agaactccag ggaatgaatg ggcagagagc 1680
   aggaatgtgg gacatctgtg ttcaagggaa ggactccagg agtctgctgg gaatgaggcc 1740
   tagtaggaaa tgaggtggcc cttgagggta cagaacaggt tcattcttcg ccaaattccc 1800
   agcaccttgc aggcacttac agctgagtga gataatgcct gggttatgaa atcaaaaagt 1860
   tggaaagcag gtcagaggtc atctggtaca gcccttcctt cccttttttt tttttttttt 1920
   ttgtgagaca aggtctctct ctgttgccca ggctggagtg gcgcaaacac agctcactgc 1980
   agcctcaacc tactgggctc aagcaatcct ccagcctcag cctcccaaag tgctgggatt 2040
   acaagcatga gccaccccac tcagcccttt ccttcctttt taattgatgc ataataattg 2100
   taagtattca tcatggtcca accaaccctt tcttgaccca ccttcctaga gagagggtcc 2160
   tcttgcttca gcggtcaggg ccccagaccc atggtctggc tccaggtacc acctgcctca 2220
   tgcaggagtt ggcgtgccca ggaagctctg cctctgggca cagtgacctc agtggggtga 2280
   ggggagctct ccccatagct gggctgcggc ccaaccccac cccctcaggc tatgccaggg 2340
   ggtgttgcca ggggcaccct aaaaaacagc acaaaaggaa actcacccta actgtaaagt 2400
   aattgtgtgt tttgagacta taaatatccc ttggagaaaa gccttgtttg ggccccccct 2460
   cgaggtcgac ggtatcgata agcttgatat cgaattcctg cagcccgggg gatccgcaga 2520
   tcccggccag ataccgatgc tgccgcagca aaagcaggag cagatgccgc cgtcgcaggc 2580
   gaagatgtcg cagacggagg aggcgatgct gccggcggag gaggcgaagt aagtagaggg 2640
   ctgggctggg ctgtgggggg tgtggggtgc gggactgggc agtctgggag tccctctcac 2700
   cacttttctt acctttctag gatgctgccg tcgccgccgc tcatacacca taaggtgtaa 2760
   aaaatactag atgcacagaa tagcaagtcc atcaaaactc ctgcgtgaga attttaccag 2820
   acttcaagag catctcgcca catcttgaaa aatgccaccg tccgatgaaa aacaggagcc 2880
   tgctaaggaa caatgccacc tgtcaataaa tgttgaaaac tcatcccatt cctgcctctt 2940
   ggtccttggg cttggggagg ggtgcgcgga tgtggttagg gaacatgact ggtcaaatgg 3000
   gaagggcttc aaaagaattc ccaatattga ctaccaagcc acctgtacag atcgaattca 3060
   gatctgcctg caggcatgca agcttggcac tggccgtcgt tttacaacgt cgtgactggg 3120
   aaaaccctgg cgttacccaa cttaatcgcc ttgcagcaca tccccctttc gccagctggc 3180
   gtaatagcga agaggcccgc accgatcgcc cttcccaaca gttgcgcagc ctgaatggcg 3240
   aatggcgcct gatgcggtat tttctcctta cgcatctgtg cggtatttca caccgcatat 3300
   ggtgcactct cagtacaatc tgctctgatg ccgcatagtt aagccagccc cgacacccgc 3360
   caacacccgc tgacgcgccc tgacgggctt gtctgctccc ggcatccgct tacagacaag 3420
   ctgtgaccgt ctccgggagc tgcatgtgtc agaggttttc accgtcatca ccgaaacgcg 3480
   cgagacgaaa gggcctcgtg atacgcctat ttttataggt taatgtcatg ataataatgg 3540
   tttcttagac gtcaggtggc acttttcggg gaaatgtgcg cggaacccct atttgtttat 3600
   ttttctaaat acattcaaat atgtatccgc tcatgagaca ataaccctga taaatgcttc 3660
   aataatattg aaaaaggaag agtatgagta ttcaacattt ccgtgtcgcc cttattccct 3720
   tttttgcggc attttgcctt cctgtttttg ctcacccaga aacgctggtg aaagtaaaag 3780
   atgctgaaga tcagttgggt gcacgagtgg gttacatcga actggatctc aacagcggta 3840
   agatccttga gagttttcgc cccgaagaac gttttccaat gatgagcact tttaaagttc 3900
   tgctatgtgg cgcggtatta tcccgtattg acgccgggca agagcaactc ggtcgccgca 3960
   tacactattc tcagaatgac ttggttgagt actcaccagt cacagaaaag catcttacgg 4020
   atggcatgac agtaagagaa ttatgcagtg ctgccataac catgagtgat aacactgcgg 4080
   ccaacttact tctgacaacg atcggaggac cgaaggagct aaccgctttt ttgcacaaca 4140
   tgggggatca tgtaactcgc cttgatcgtt gggaaccgga gctgaatgaa gccataccaa 4200
   acgacgagcg tgacaccacg atgcctgtag caatggcaac aacgttgcgc aaactattaa 4260
   ctggcgaact acttactcta gcttcccggc aacaattaat agactggatg gaggcggata 4320
   aagttgcagg accacttctg cgctcggccc ttccggctgg ctggtttatt gctgataaat 4380
   ctggagccgg tgagcgtggg tctcgcggta tcattgcagc actggggcca gatggtaagc 4440
   cctcccgtat cgtagttatc tacacgacgg ggagtcaggc aactatggat gaacgaaata 4500
   gacagatcgc tgagataggt gcctcactga ttaagcattg gtaactgtca gaccaagttt 4560
   actcatatat actttagatt gatttaaaac ttcattttta atttaaaagg atctaggtga 4620
   agatcctttt tgataatctc atgaccaaaa tcccttaacg tgagttttcg ttccactgag 4680
   cgtcagaccc cgtagaaaag atcaaaggat cttcttgaga tccttttttt ctgcgcgtaa 4740
   tctgctgctt gcaaacaaaa aaaccaccgc taccagcggt ggtttgtttg ccggatcaag 4800
   agctaccaac tctttttccg aaggtaactg gcttcagcag agcgcagata ccaaatactg 4860
   tccttctagt gtagccgtag ttaggccacc acttcaagaa ctctgtagca ccgcctacat 4920
   acctcgctct gctaatcctg ttaccagtgg ctgctgccag tggcgataag tcgtgtctta 4980
   ccgggttgga ctcaagacga tagttaccgg ataaggcgca gcggtcgggc tgaacggggg 5040
   gttcgtgcac acagcccagc ttggagcgaa cgacctacac cgaactgaga tacctacagc 5100
   gtgagctatg agaaagcgcc acgcttcccg aagggagaaa ggcggacagg tatccggtaa 5160
   gcggcagggt cggaacagga gagcgcacga gggagcttcc agggggaaac gcctggtatc 5220
   tttatagtcc tgtcgggttt cgccacctct gacttgagcg tcgatttttg tgatgctcgt 5280
   caggggggcg gagcctatgg aaaaacgcca gcaacgcggc ctttttacgg ttcctggcct 5340
   tttgctggcc ttttgctcac atgttctttc ctgcgttatc ccctgattct gtggataacc 5400
   gtattaccgc ctttgagtga gctgataccg ctcgccgcag ccgaacgacc gagcgcagcg 5460
   agtcagtgag cgaggaagcg gaaga                                       5485